(19) Europäisches Patentamt
European Patent Office
Office européen des brevets

(11) Veröffentlichungsnummer: **0 080 976**
**B1**

(12) # EUROPÄISCHE PATENTSCHRIFT

(45) Veröffentlichungstag der Patentschrift: **24.09.86**

(51) Int. Cl.⁴: **A 61 K 7/06**

(21) Anmeldenummer: **82810503.1**

(22) Anmeldetag: **24.11.82**

(54) **Gemische aus quaternären, polymeren Ammoniumsalzen auf Acrylbasis, aus quaternären, mono- bis oligomeren Ammoniumsalzen und aus Tensiden, deren Herstellung und Verwendung in kosmetischen Mitteln.**

(30) Priorität: **30.11.81 CH 7655/81**

(43) Veröffentlichungstag der Anmeldung: **08.06.83 Patentblatt 83/23**

(45) Bekanntmachung des Hinweises auf die Patenterteilung: **24.09.86 Patentblatt 86/39**

(84) Benannte Vertragsstaaten: **CH DE FR GB IT LI**

(56) Entgegenhaltungen:
**EP-A-0 045 720**
**EP-A-0 047 714**
**GB-A-1 073 947**
**GB-A-1 342 176**
**GB-A-2 027 045**

**CHEMICAL ABSTRACTS, Band 89, Nr. 8, 21. August 1978, Seite 332, Spalte 2, Nr. 65141d, Columbus, Ohio, USA**
**PATENTS ABSTRACTS OF JAPAN, Band 3, Nr. 68(C-48), 13. Juni 1979, Seite 128C48, 2. Zusammenfassung**
**CHEMICAL ABSTRACTS, Band 92, Nr. 14, April 1980, Seite 343, Spalte 2, Zusammenfassung Nr. 116256s, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Moldovanyi, Laszlo, Dr., Austrasse 78, CH- 4051 Basel (CH)**
Erfinder: **Fearnley, Charles, Dr., Wettsteinanlage 50, CH- 4125 Riehen (CH)**
Erfinder: **Hungerbühler, Walter, Höhenstrasse 8, CH- 4125 Riehen (CH)**

**EP 0 080 976 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

In der Haarpflege stellt die Reinigung der Haare einen der wichtigsten Vorgänge dar. Neuerdings werden als Reinigungsmittel zweckmässig synthetische Waschrohstoffe eingesetzt, die den Vorteil aufweisen, auch bei hoher Wasserhärte ihre volle Reinigungskraft zu behalten. Bei alleiniger Verwendung von Waschrohstoffen zeigt das behandelte Haar in der Regel keine Konditioniereffekte.

Der vorliegenden Erfindung liegt nun die Aufgabe zugrunde, ein für kosmetische Zwecke, insbesondere für die Haarpflege geeignetes, kostengünstiges Reinigungsmittel zur Verfügung zu stellen, das neben einer guten Waschwirkung die Erzielung ausgezeichneter Konditioniereffekte auf dem Haar ermöglicht. Diese sog. Konditioniereffekte stellen einen Sammelbegriff für u.a. folgende erwünschte Eigenschaften des behandelten Haares dar:

- leichte Nass- und Trockenkämmbarkeit
- Verhinderung der statischen Aufladung
- keine negative Beeinträchtigung des ursprünglichen Glanzes
- Aspekt, insbesondere Volumen und Fülle
- Griff.

Gute Konditioniereffekte sind mit monomeren bis oligomeren, quaternären Ammoniumsalzen insbesondere in Nachspülmitteln erreichbar. Die Affinität dieser Ammoniumsalze zum Haarkeratin ist jedoch nur in Abwesenheit von synthetischen Waschrohstoffen, insbesondere von anionischen Tensiden, voll gewährleistet so dass der Einsatz solcher Ammoniumsalze in Shampoos kaum in Frage kommt. Um an sich gute Konditioniereffekte zu erzielen, ist es deshalb in der Regel nötig, Haar zuerst zu shampoonieren und in einer getrennten, weiteren Stufe mit einem tensidarmen Nachspülmittel zu behandeln, das das angegebene quaternäre Ammoniumsalz enthält. Obwohl die monomeren bis oligomeren, quaternären Ammoniumsalze an sich im allgemeinen besonders preisgünstig sind, ist ihre begrenzte Formulierungsmöglichkeit ein wesentlicher Nachteil, indem immer ein zweistufiges und damit recht aufwendiges Haarbehandlungsverfahren erforderlich ist.

Auch mit hochmolekularen, polymeren quaternären Ammoniumsalzen sind gute Konditioniereffekte erreichbar, die jedoch im allgemeinen schwächer sind als beim gleichzeitigen Einsatz der monomeren bis oligomeren quaternären Ammoniumsalze. Dies trifft vor allem für die Verhinderung der statischen Aufladung der behandelten Haare zu. Obschon die polymeren, quaternären Ammoniumsalze in Gegenwart von vielen, in der Kosmetikindustrie üblicherweise verwendeten Tensiden ihre Affinität zum Haarkeratin behalten und somit in den meisten Shampooformulierungen einsetzbar sind, weisen sie neben ihrem relativ hohen Preis den wesentlichen Nachteil auf, bei der Haarbehandlung zu unerwünschten Akkumuliereffekten führen zu können.

Durch die Kombination von monomeren bis oligomeren, quaternären Ammoniumsalzen mit ausgewählten, polymeren, quaternären Ammoniumsalzen auf Acrylbasis in Gegenwart von Tensiden in neuartiger Zusammensetzung ist es erfindungsgemäss nun auf unerwartete Weise gelungen, die vorstehend erwähnten Nachteile, wie sie bei der separaten Verwendung der Ammoniumsalze der beiden angegebenen Arten auftraten, weitgehend zu eliminieren.

Erfindungsgemäss werden hochmolekulare, polymere, quaternäre Ammoniumsalze auf Acrylbasis eingesetzt, die eine Molekulargewichtsverteilung von $10^4$ bis $10^9$ aufweisen, wobei das Molekulargewicht von mindestens 5 % des Polymers $10^7$ bis $10^9$ beträgt.

Aus GB-A-1 073 947 sind Tensidzusammensetzungen bekannt, die jedoch zum Reinigen von z.B. Leder, Glas, Anstrichen und Holz verwendet werden und keine oligomere, quaternäre Ammoniumsalze enthalten. Zudem enthalten die bekannten Zusammensetzungen Polymere auf Acrylbasis, die jedoch nicht quaterniert sind und Molekulargewichte von nur 3 bis $15.10^6$ aufweisen.

Aus GB-A-1 342 176 sind Zusammensetzungen bekannt, die anionische oder nicht-ionogene Tenside, monomere, quaternäre Ammoniumsalze und polymere, quaternäre Ammoniumsalze auf Acrylbasis enthalten, die jedoch als Waschflotte zum Antistatischmachen von synthetischen Polyamidtextilien verwendet werden. Zudem enthält GB-A-1 342 176 über das Molekulargewicht der eingesetzten polymeren, quaternären Ammoniumsalze keinerlei Angaben.

Aus GB-A-2 027 045 sind Haarshampoos bekannt, die anionische Tenside und polymere, quaternäre Ammoniumsalze auf Acrylbasis, jedoch keine monomere bis oligomere Ammoniumsalze enthalten. Zudem beträgt das Molekulargewicht der eingesetzten, polymeren Ammoniumsalze nur $10^3$ bis $10^6$.

Aus JP-A-78 24036 sind Haarnachspülmittel bekannt, die monomere, quaternäre Ammoniumsalze und polymere, quaternäre Ammoniumsalze auf Acrylbasis, jedoch keine weiteren Tenside, wie z.B. anionische oder nicht-ionogene Tenside enthalten. Zudem ist das Molekulargewicht der eingesetzten, polymeren Ammoniumsalze nur $1.10^4$ bis $5.10^5$.

Aus JP-A-54 43211 und JP-A-79 135 234 sind Haarnachspülmittel bekannt, die ein anionisches oder ein nichtionogenes Tensid, ein monomeres bis oligomeres Ammoniumsalz und ein polymeres, quaternäres Ammoniumsalz enthalten, wobei das polymere Ammoniumsalz jedoch nicht auf Acrylbasis, sondern eine kationische Cellulose ist, deren Molekulargewicht nur $10^3$ bis $10^6$ beträgt.

Aus EP-A-0 045 720 und EP-A-0 047 714 sind Haarshampoos bekannt, die Tenside und polymere, quaternäre Ammoniumsalze enthalten, die mit den erfindungsgemäss eingesetzten Tensiden und polymeren, quaternären Ammoniumsalzen identisch sind. Die bekannten Shampoos enthalten jedoch keine monomere bis oligomere Ammoniumsalze und ergeben daher Konditionseffekte auf dem Haar, die schlechter anfallen, als bei

Verwendung der erfindungsgemässen Zusammensetzungen.

Gegenstand der vorliegenden Erfindung ist somit eine wässrige Zusammensetzung aus polymeren, quaternären Ammoniumsalzen, aus monomeren bis oligomeren, quaternären Ammoniumsalzen und aus nichtionischen oder anionischen Tensiden, die dadurch gekennzeichnet ist, dass sie einen pH-Wert von 3 bis 9 aufweist und mindestens (A) ein in wässrigen Tensidsystemen lösliches oder mikroemulgierbares er Ammoniumsalz, das eine Molekulargewichtsverteilung von $10^4$ bis $10^9$ aufweist, wobei das Molekulargewicht von mindestens 5 Gewichtsprozent des polymeren Salzes $10^7$ bis $10^9$ beträgt und das Salz wiederkehrende Strukturelemente der Formel

(1) 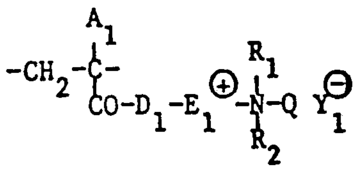

und gegebenenfalls in beliebiger Reihemfolge mindestens eines der wiederkehrenden Strukturelemente der Formeln

(2) 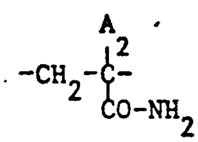 ,

(3) 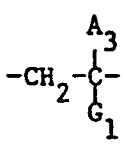 und

(4) 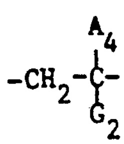

aufweist, worin $A_1$, $A_2$, $A_3$ und $A_4$ je Wasserstoff oder Methyl, $G_1$ und $G_2$ voneinander verschieden sind und je

-CN, -COOH oder 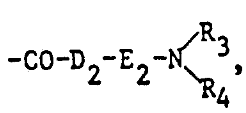,

$D_1$ und $D_2$ je Sauerstoff oder -NH-, $E_1$ und $E_2$ je Alkylen mit 1 bis 4 Kohlenstoffatomen, das gegebenenfalls durch Hydroxyl substituiert ist,

$R_1$, $R_2$, $R_3$ und $R_4$ je Methyl oder Aethyl, Q Alkyl, Hydroxyalkyl mit 1 bis 4 Kohlenstoffatomen oder Benzyl und $Y_1^{\ominus}$ ein Halogenid-, Alkylsulfat- oder Alkylphospbonatanion mit 1 bis 4 Kohlenstoffatomen im Alkylrest

bedeuten,

(B) ein Ammoniumsalz, das 1 bis 3 quaternäre Stickstoffatome und ein Molekulargewicht von höchstens 9'000 aufweist und

(C) ein nicht-ionisches Tensid, ein Tensid mit intramolekular je einer positiven und negativen Ladung oder ein anionisches, gegebenenfalls zwitterionisches Tensid oder deren Gemische

enthält,

wobei bei Einsatz eines anionischen Tensides als Komponente (C) das polymere Ammoniumsalz als Komponente (A) ein Copolymerisat ist, das neben Strukturelementen der Formel (1) auch Strukturelemente der Formel (2) aufweist, und das anionische Tensid mindestens teilweise mit den Komponenten (A) und (B) unter Ionenaustausch umgesetzt ist.

Weitere Gegenstände der Erfindung sind das Herstellungsverfahren für die vorstehemd angegebene Zusammensetzung, deren Verwendung in kosmetischen Mitteln, die kosmetischen Mittel (Haarkosmetika), welche die erfindungsgemässen Gemische enthalten und deren Applikationsverfahren, insbesondere Verfahren zum Behandeln von Haar sowie nach den Applikationsverfahren behandeltes Haar, z.B. in Form von Perücken.

Der pH-Wert von 3 bis 9 der erfindungsgemässen Zusammensetzung wird, sofern notwendig, durch Zusatz einer Säure oder Base eingestellt, wobei die Auswahl der Säure bzw. Base nicht kritisch ist.

Die in den erfindungsgemässen Gemischen als Komponente (A) eingesetzten, polymeren Ammoniumsalze zeichnen sich dadurch aus, dass sie durch Wasser-in-Oel Emulsionspolymerisation oder Lösungspolymerisation eines quaternären Ammoniumsalzes der Acrylsäurereihe und gegebenenfalls mindestens eines weiteren Comonomers auf Acrylbasis erhältlich sind.

Durch Wasser-in-Oel Emulsionspolymerisation, auch inverse Emulsionspolymerisation genannt, oder durch Lösungspolymerisation, erreicht man den hohen Molekulargewichtsbereich der erfindungsgemäss eingesetzten Polymerisate von $10^7$ bis $10^9$, deren Anteil vorzugsweise 5 bis 60, vor allem 10 bis 60 und insbesondere 20 bis 50 Gewichtsprozent der Copolymerisate innerhalb der breiten Molekulargewichtsvertei-i lung von $10^4$ bis $10^9$ ausmacht. Eine Anreicherung an Anteile eines Molekulargewichtes von $10^7$ bis $10^9$ innerhalb der breiten Molekulargewichtsverteilung von $10^4$ bis $10^9$ kann nötigenfalls auch durch Behandlung des Polymers in einem vorzugsweise mit Oel und Wasser mischbaren Lösungsmittel, z.B. Methanol, Isopropanol oder Aceton, erzielt werden. Eine solche Behandlung wird vor allem nach durchgeführter Lösungspolymerisation durchgeführt. Besonders bevorzugte Polymere weisen 15-45 oder sogar 30-45 Gewichtsprozent Anteile im Molgewichtsbereich $10^7$ bis $10^9$ und weniger als 15 Gewichtsprozent Anteile im Molgewichtsbereich kleiner als $10^5$ auf.

Der Anteil an Strukturelementen der Formel (1) in den Polymerisaten, auch Quatgehalt genannt, bildet neben der Molekulargewichtsverteilung ein weiteres, wesentliches Kennzeichen der eingesetzten Ammoniumsalze, welche durchschnittlich etwa 5 bis 100, vorzugsweise 5 bis 80 und insbesondere 6 bis 40, bzw. 10 bis 30 Mol% Strukturelemente der Formel (I), 0 bis durchschnittlich etwa 95, vorzugsweise 10 bis 95 oder 10 bis 75 und insbesondere 50 bis 90 Mol% Strukturelemente der Formel (2) und 0 bis durchschnittlich etwa 10, vorzugsweise insgesamt 1 bis 8 Mol% Strukturelemente der Formel (3) und gegebenenfalls (4), d.h. (3) und/oder (4) und insbesondere je 1 bis 4 Mol% Strukturelemente der Formeln (3) und (4) enthalten. Ammoniumsalze mit einem Quatgehalt, d.h. einem Gehalt an Strukturelementen der Formel (I), von 100%, stellen Homopolymerisate dar. Der Quatgehalt von 100% ist aber als Idealwert anzusehen, da durch Hydrolyse der Strukturelemente der Formel (I) die Homopolymerisate stets Spuren (z.B. 0,01 bis 0,5 Gewichtsprozent) an Strukturelementen der Formel (4), worin $G_2$ -COOH bedeutet, enthalten. Copolymerisate, die mindestens eines der Strukturelemente (2), (3) und/oder (4) aufweisen, sind den Homopolymerisaten gegenüber als Komponente (A) in den erfindungsgemässen Gemischen bevorzugt, da sie auch in Gegenwart von anionischen Tensiden für sich allein als Komponente (C) zu vollständig klar gelösten Zusammensetzungen aus (A), (B) und (C) führen. Insbesondere enthält bei Einsatz eines anionischen Tensides als Komponente (C) das polymere Ammoniumsalz als Komponente (A) höchstens 80 Mol%, z.B. 5 bis 80 Mol%, Strukturelemente der Formel (1) und mindestens 10 Mol%, z.B. 10 bis 75 Mol%, Strukturelemente der Formel (2).

Als bevorzugte Bedeutungen für die Symbole der Formel (1) gelten für $A_1$ Methyl, für $D_1$ Sauerstoff, für $E_1$ unsubstituiertes n-Propylen oder insbesondere unsubstituiertes Aethylen, für $R_1$ und $R_2$ Methyl und für Q unsubstituiertes Propyl, vorzugsweise Aethyl oder insbesondere Methyl. $A_2$ in Formel (2) steht vorzugsweise für Wasserstoff. Falls Strukturelemente der Formel (3) für sich allein, d.h. in Abwesenheit von Strukturelementen der Formel (4) vorhanden sind, gelten als bevorzugte Bedeutungen für die Symbole in Formel (3):

Methyl für $A_3$ und $-CO-D_2-E_2-$ 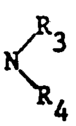

für $G_1$, wobei $D_2$, $E_2$, $R_3$ und $R_4$ die gleichen bevorzugten Bedeutungen haben wie für $D_1$, $E_1$, $R_1$ und $R_2$ vorstehend angegeben. Falls Strukturelemente der Formel (4) zusätzlich zu den Strukturelementen der Formel (3) vorhanden sind, bedeuten in Formel (4) $A_4$ vorzugsweise Wasserstoff und $G_2$ vorzugsweise -CN oder $A_4$ insbesondere Methyl und $G_2$ insbesondere -COOH.

Als bevorzugte Komponenten (B) enthalten die erfindungsgemässen Zusammensetzungen Ammoniumsalze der Formel

(5) 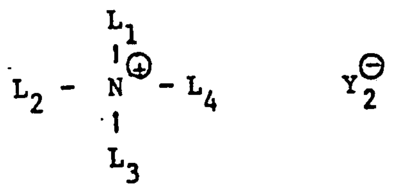 ,

worin $L_1$ Wasserstoff, Alkyl oder Alkenyl mit höchstens 4 Kohlenstoffatomen, das gegebenenfalls durch Hydroxyl substituiert ist, $L_2$, $L_3$ und $L_4$ je gegebenenfalls verzweigtes Alkyl oder Alkenyl mit höchstens 22 Kohlenstoffatomen, das gegebenenfalls durch Sauerstoffatome oder Säureamidreste unterbrochen ist und gegebenenfalls endständig durch Hydroxyl, Carbamoyl, Dialkylamino, unsubstituiertes Phenyl oder Phenoxy, halogensubstituiertes oder halogenmethylsubstituiertes Phenyl oder Phenoxy substituiert ist, wobei durch -0- oder -CONH- unterbrochene $L_2$-, $L_3$- und $L_4$-Reste insgesamt je höchstens 120 Kohlenstoffatome aufweisen, oder bei Ammioniumsalzen mit zwei quaternären Stickstoffatomen, einer der $L_2$-, $L_3$- oder $L_4$-Reste der angegebenen Art endständig mit einem Di- oder Trialkylammonium-Kation substituiert ist, wobei die Alkylreste in Alkylamino- bzw. Alkylammonium-Substituenten 1 bis 4 Kohlenstoffatome aufweisen und gegebenenfalls durch Hydroxyl substituiert sind, oder $L_3$ und $L_4$ zusammen mit dem quaternären Stickstoffatom einen 4,5-Dihydroimidazolring oder einen 3,4,5,6-Tetrahydropyrimidinring bilden, wobei diese Ringe in 2-Stellung mit Alkyl mit 1 bis 22 Kohlenstoffatomen und am quaternären Stickstoffatom mit $L_1$ und $L_2$ mit je den angegebenen Bedeutungen substituiert sind, oder bei Ammoniumsalzen mit 3 quaternären Stickstoffatomen $L_1$ und $L_2$ die für $L_1$ angegebenen Bedeutungen haben, $L_3$ Carbalkoxyalkyl oder Carbalkenyloxyalkyl mit 8 bis 22 Kohlenstoffatomen im Carbalkoxy- oder Carbalkenyloxyteil und 1 oder 2 Kohlenstoffatome im Alkylteil, und $L_4$ einen geradkettigen Alkylrest mit 5 bis 8 Kohlenstoffatomen, der durch 2 quaternäre Stickstoffatome unterbrochen ist, wobei die Stickstoffatome durch Aethylen oder Propylen verbunden sind und jeweils mit $L_1$- und $L_3$-Reste der zuletzt angegebenen Bedeutungen substituiert sind und

$Y_2^{\ominus}$ in Halogenid-, Alkylsulfat- oder Alkylphosphonatanion mit 1 bis 4 Kohlenstoffatomen im Alkylrest oder das Anion einer Alkylcarbonsäure oder einer Oxycarbonsäure mit höchstens 6 Kohlenstoffatomen oder einer Phosphorsäure bedeuten.

Bevorzugte Bedeutungen für $L_1$ in Formel (5) sind Wasserstoff, Alkenyl mit 3 oder 4 Kohlenstoffatomen, z.B. Allyl, l- und 2-Butenyl oder Isopropenyl, Hydroxyalkyl mit 2 bis 4 Kohlenstoffatomen, z.B. Hydroxypropyl oder Hydroxyäthyl oder vor allem Alkyl mit 1 bis 4 Kohlenstoffatomen, z.B. Butyl, Isopropyl, Propyl und insbesondere Aethyl und Methyl. Infolge des pH-Wertes von 3 bis 8 der erfindungsgemässen Zusammensetzungen liegen die Verbindungen der Formel (5), insbesondere solche worin $L_1$ für Wasserstoff steht, stets als quaternäre Ammoniumsalze vor.

Sind $L_2$, $L_3$ und $L_4$ in Formel (5) Alkyl- oder Alkenylreste, so weisen sie in der Regel 1 bis 22 Kohlenstoffatome auf. Als bevorzuge Alkyl- oder Alkenylreste dieser Art kommen neben der für $L_1$ angegebenen Bedeutungen z.B. Diallyl, Isoamyl, Cetyl oder vor allem Alkyloder Alkenylreste, die sich von den entsprechenden gesättigten oder ungesättigten Fettsäuren mit 8 bis 22, vorzugsweise 12 bis 18 Kohlenstoffatomen ableiten, in Frage.

Als Beispiele der entsprechenden Fettsäuren seien Capryl-, Caprin-, Arachin- und Behensäure, insbesondere Laurin-, Myristin-, Palmitin- und Stearinsäure oder Myristolein-, Palmitolein-, Elaeostearin-, Clupanodonsäure, insbesondere Oel-, Elaidin-, Eruka-, Linolund Linolensäure genannt. Alkyl- und Alkenylreste für $L_2$, $L_3$ und $L_4$, die sich von technischen Gemischem der genannten gesättigten und/oder ungesättigten Fettsäuren ableiten, sind besonders bevorzugt.

Die durch Säureamidreste unterbrochenen Alkyl- oder Alkenylketten weisen in der Regel nur ein -CONH- oder -NHCO-Brückenglied auf, wobei 2 Alkyl- oder Alkenylketten und ein Säureamidbrückenglied insgesamt vorzugsweise höchstens 40 Kohlenstoffatome aufweisen.

Bedeuten $L_2$, $L_3$ und $L_4$ in Formel (5) durch Sauerstoffatome unterbrochene Alkylreste, so handelt es sich hierbei z.B. um Polyalkylen-, vorzugsweise Polypropylen- und insbesondere Polyäthylenketten, die etwa 1 bis 40 Polyalkyleneinheiten aufweisen.

Dialkylamino als endständiger Substituent der Alkyl- oder alkylenketten $L_2$, $L_3$ und $L_4$ weist die Di- oder Trialkylammonium-Substituenten je 1 bis 4 Kohlenstoffatome im Alkylteil, wobei Methyl und Aethyl im Vordergrund des Interesses stehen, auf.

Bevorzugte Ammoniumsalze mit 1 oder 2 quaternären Stickstoffatomen entsprechen demgemäss der Formel

(6) $$L_6-\overset{L_5}{\underset{L_7}{N^{\oplus}}}-L_8 \qquad Y_2^{\ominus}$$ ,

worin $L_5$ Wasserstoff, Alkyl mit 1 bis 4 Kohlenstoffatomen oder Hydroxyalkyl mit 2 bis 4 Kohlenstoffatomen oder Alkenyl mit 3 oder 4 Kohlenstoffatomen, $L_6$, $L_7$ und $L_8$ je Alkyl mit 1 bis 22 Kohlenstoffatomen, Hydroxyalkyl mit 2 bis 4 Kohlenstoffatomen, Alkenyl mit 3 bis 22 Kohlenstoffatomen oder Reste der Formeln

(7) $$-(CH_2)_{a-1}-(CH_2-\overset{(H)_{n-1}}{\underset{(CH_3)_{2-n}}{CHO}})_b-H$$ ,

(8) $-(CH_2)_{n-1}-CH_2-(O)_{n'-1}-$ 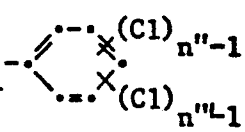 ,

(9) $-(CH_2)_a-CONH_2$ , (10) $-(CH_2)_a-CONH-L_9$ oder (11) $-(CH_2)_a-NHCO-L_9$,

worin $L_9$ Hydroxyäthyl oder Alkyl mit 1 bis 22 Kohlenstoffatomen oder $L_9$ eimen Rest der Formel

(12) $$-(CH_2)_a-O-(CH_2-\overset{(H)_{n-1}}{\underset{(CH_3)_{2-n}}{CHO}})_b-H$$ oder

(13) $$-(CH_2)_c-\overset{L_{10}}{\underset{(L_{12})_{n-1}}{N^{\oplus}_{n-1}}}-L_{11} \qquad Y_{2\,n-1}^{\ominus}$$ ,

worin $L_{10}$ und $L_{11}$ je Methyl, Aethyl oder Hydroxyäthyl und $L_{12}$ Wasser stoff, Methyl, Aethyl oder Hydroxyäthyl, a 1 bis 22, b 1 bis 40 und c 1 bis 6 oder $L_7$ und $L_8$ zusammen mit dem quaternären Stickstoffatom einen Ring der Formel

(14) 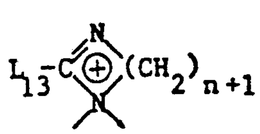 ,

worin $L_{13}$ Alkyl oder Alkenyl mit 8 bis 22 Kohlenstoffatomen und n, n', n'' und n''' je 1 oder 2 bedeuten und $Y_2^{\ominus}$ die angegebenen Bedeutungen hat.

Falls n in Formel (13) für 2 steht, weisen die Ammoniumsalze 2 quatermäre Stickstoffatome auf. In diesem Falle bedeutet nur einer der Reste $L_6$, $L_7$ und $L_8$ in Formel (6) ein durch Säureamidreste unterbrochenes Alkyl einer der Formeln (10) oder (11).

Die Ammoniumsalze, welche 3 quaternäre Stickstoffatome aufweisen, entsprechen der Formel (5), worin $L_4$ durch Stickstoffatome unterbrochenes Alkyl und $L_3$ z.B. Carbalkoxyalkyl bedeuten. Bevorzugte Ammomiumsalze dieser Art entsprechen der Formel

(15) 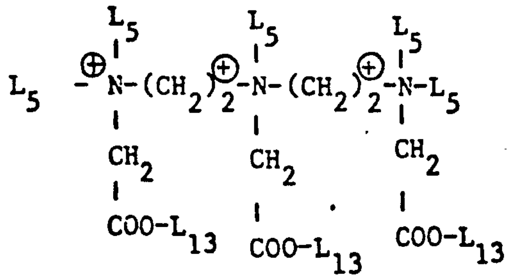 $3\ Y_2^{\ominus}$ ,

worin $L_5$, $L_{13}$ und $Y_2^{\ominus}$ die angegebenen Bedeutungen haben.

Die Ammoniumsalze mit nur einem quaternären Stickstoffatom sind gegenüber solchen mit 3 oder 2 quaternären Stickstoffatomen bevorzugt. Bevorzugte Ammoniumsalze mit einem quaternären Stickstoffatom entsprechen der Formel

(16) 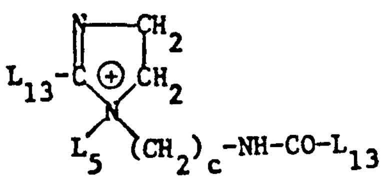 $Y_3^{\ominus}$ ,

worin $L_5$, $L_{13}$ und c die angegebenen Bedeutungen haben und $Y_3^{\ominus}$ ein Chlorid-, Methylsulfat- oder Acetatanion bedeutet.

Andere bevorzugte Ammoniumsalze mit einem quaternären Stickstoffatom entsprechen der Formel

(17) 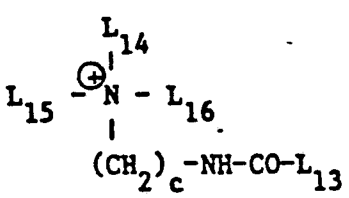 $Y_4^{\ominus}$ ,

worin $L_{14}$ und $L_{15}$ je Alkyl mit 1 bis 4 Kohlenstoffatomen, $L_{16}$ Wasserstoff, Alkyl mit 1 bis 4 Kohlenstoffatomen oder Hydroxyalkyl mit 2 bis 4 Kohlenstoffatomen oder $-CH_2-CO-NH_2$ und $Y_4^{\ominus}$ ein Chlorid-, Methylsulfat- oder Acetatanion oder sofern $L_{16}$ Wasserstoff ist, ein Citrat-, Lactat- oder Phosphatanion bedeuten und $L_{13}$ und c die angegebenen Bedeutungen haben, oder der Formel

(18) 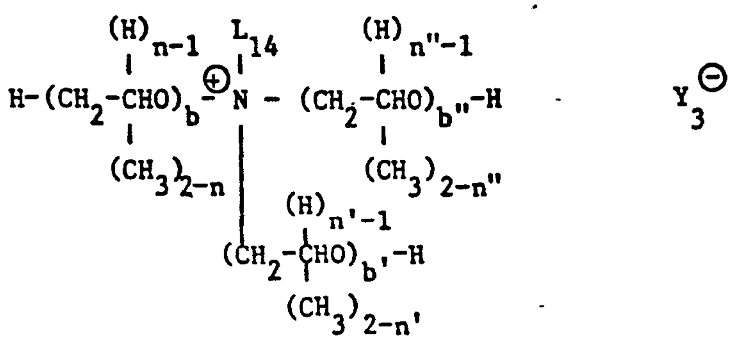

worin b, b' und b'' je 1 bis 40, n, n' und n'' je 1 oder 2 bedeuten und $L_{14}$ und $Y_3^{\ominus}$ die angegebenen Bedeutung haben.

Ein einziges, quaternares Stickstoffatom aufweisende Ammoniumsalze, die im Vordergrund des Interesses stehen, entsprechen indessem der Formel

(19) 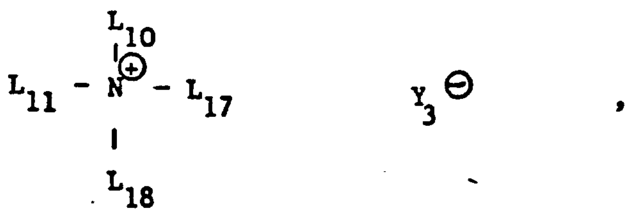

worin $L_{17}$ Alkyl mit 10 bis 22, vorzugsweise 10 bis 18 Kohlenstoffatomen umd $L_{18}$ Alkyl mit 1 bis 22, vorzugsweise 10 bis 22 Kohlenstoffatomen, Benzyl, Phenoxymethylen oder Phenoxyäthylen bedeuten und $L_{10}$, $L_{11}$ und $Y_3^{\ominus}$ die angegebenen Bedeutungen haben.

Im Vordergrund des Interesses stehen ebenfalls ein einziges quaternäres Stickstoffatom aufweisende Ammoniumsalze der Formel (17), worin $L_{14}$ umd $L_{15}$ je Aethyl oder vorzugsweise Methyl umd $L_{16}$ $-CH_2-CO-NH_2$ bedeuten und insbesondere Ammoniumsalze, die der Formel (16) entsprechen.

Als spezifische Beispiele der Komponente (B) der erfindungsgemässen Zubereitung seien die folgenden Ammoniumsalze genannt:

(20) 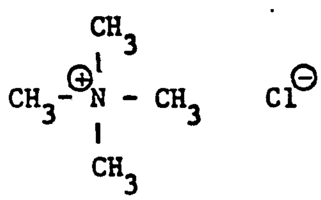

(21) 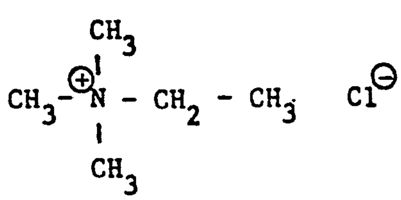

(22)

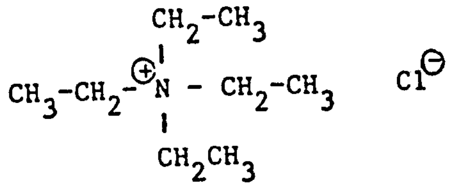

(23)

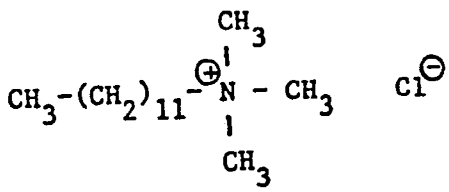

(24)

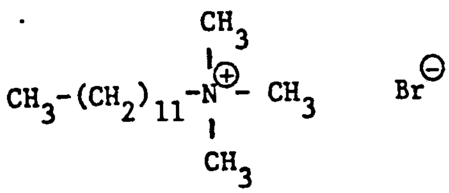

(25)

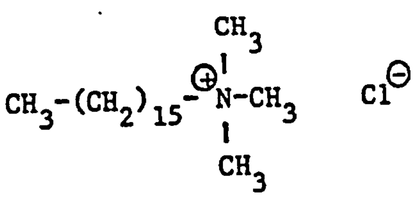

(26)

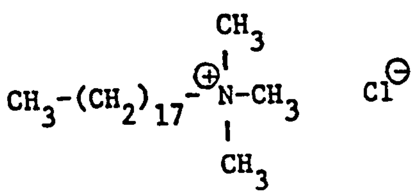

(27)

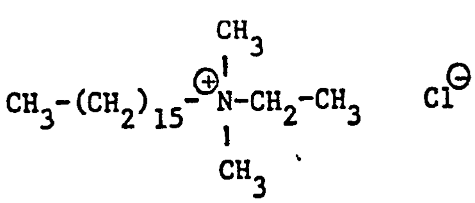

(28)

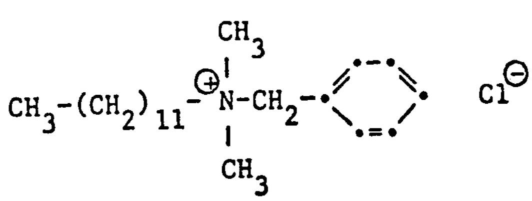

(29) 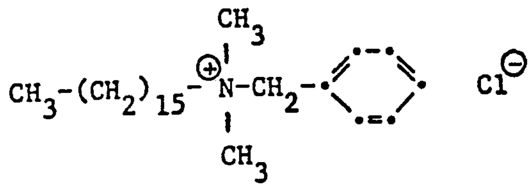

(30) 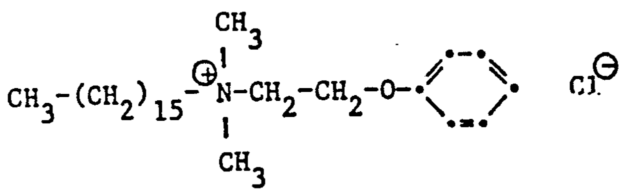

(31) 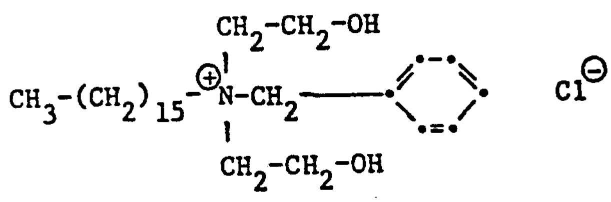

(32) 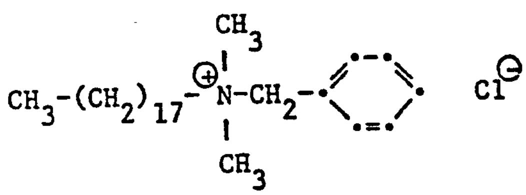

(33) 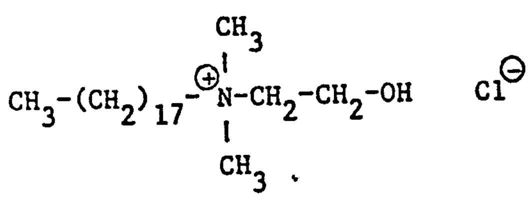

(34) 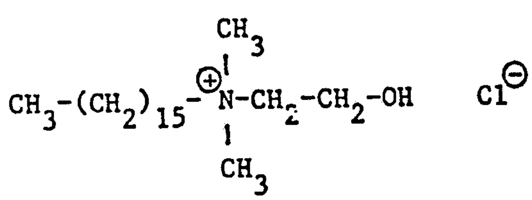

(35) 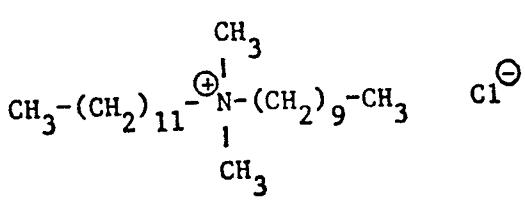

(36)

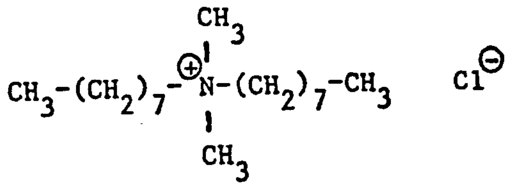

(37)

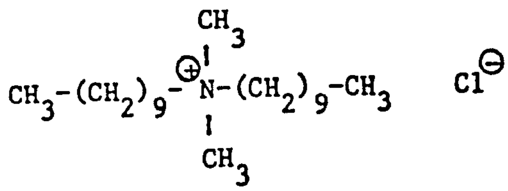

(38)

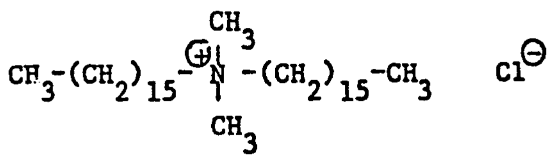

(39)

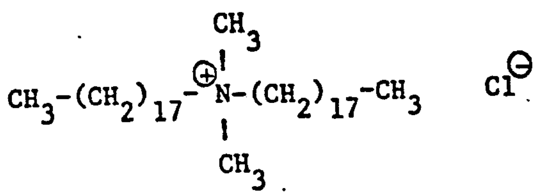

(40)

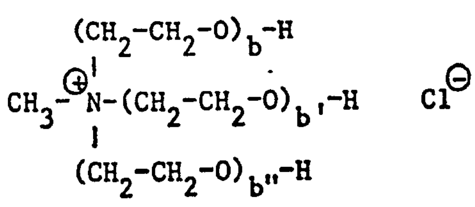

b, b' und b" = je 1 bis 40

(41)

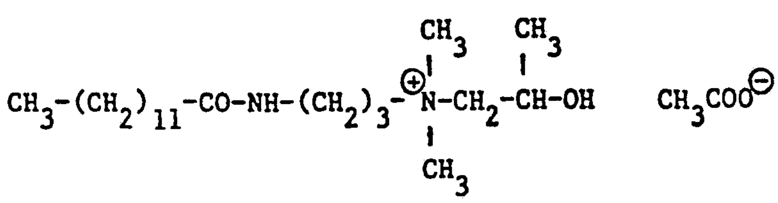

(42)

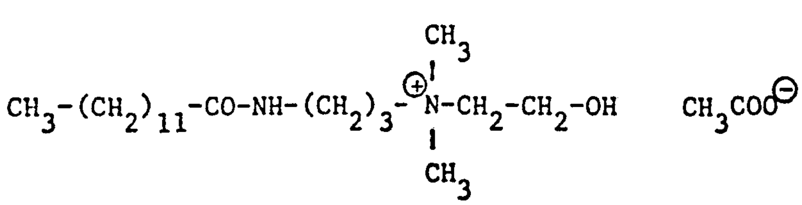

(43) 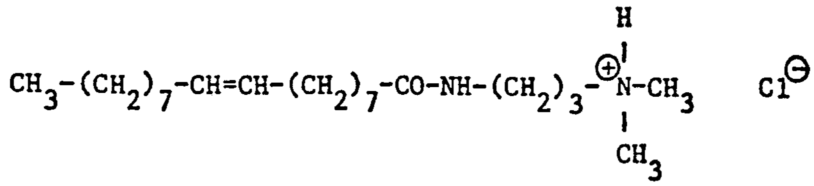

(44) 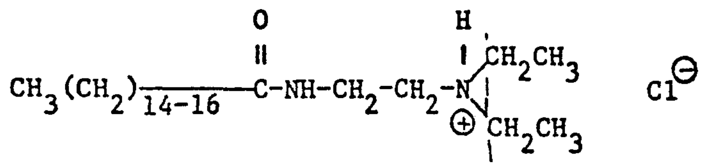

(45) 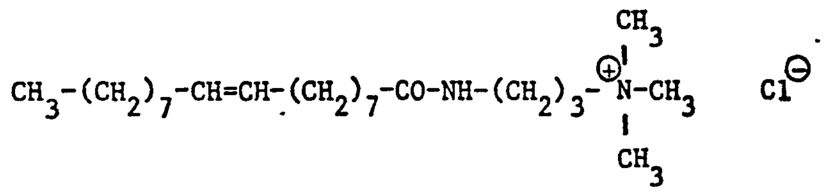

(46) 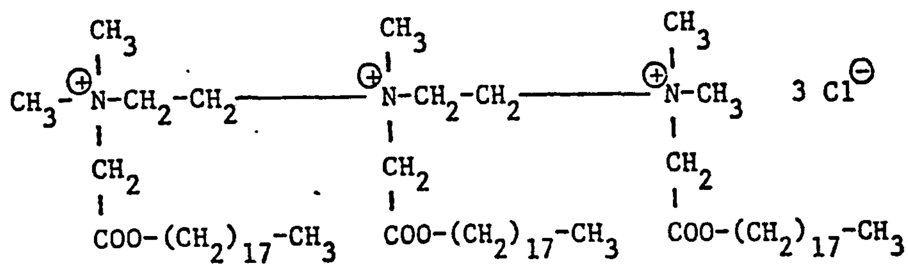

(47) 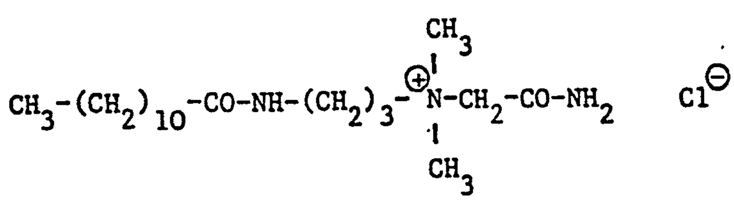

(48) 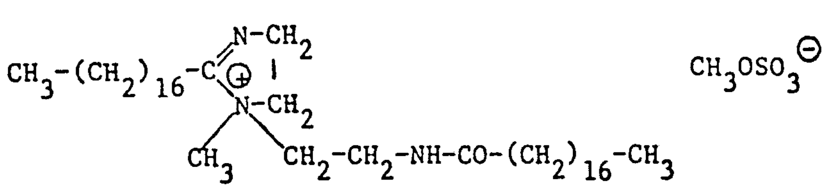

Hierbei stehen die Ammoniumsalze der Formeln (23) bis (32) vorzugsweise (23), (24), (25) und (28), und vor allem der Formeln bis (39), (47) und (48), insbesondere (37), (38), (39) und (48), im Vordergrund des Interesses.

Als nicht-ionische Tenside für die Komponente (C) in den erfindungsgemässen Zusammensetzungen kommen alkoxylierte, vorzugsweise propoxylierte und insbesondere äthoxylierte Fettamine, vor allem aber äthoxylierte Fettalkohole, Fettsäuren, Fettsäureamide, Alkylphenole oder Kohlenhydrate, worin die endständigen Hydroxylgruppen gegebenenfalls veräthert sind, und insbesondere als Alkyläther mit 1 bis 20 Kohlenstoffatomen im Aetherteil vorliegen, Addukte (Blockcopolymere) aus Aethylen- und Propylenoxyd, Phosphorsäurepolyglykolester oder Aminoxyde, die vorzugsweise einen Fettrest aufweisen, in Betracht.

Geeignet sind ferner auch Fettsäureester von 3- bis 6-wertigen Alkoholen (Glycerin, Pentaerythrit, Sorbit, Sorbitan) oder von Monound Disacchariden (Saccharose).

Die als nicht-ionische Tenside verwendeten, äthoxylierten Fettsäuren, Fettsäureamide, Fettalkohole, Alkylphenole oder Kohlenhydrate entsprechen vorzugsweise einer der Formeln

(49) $H-(O-CH_2-CH_2)_p-OOC-T_1$,

(50) $H-(O-CH_2-CH_2)_p-NH-CO-T_1$,

(51) $H-(O-CH_2-CH_2)_p-O-T_2$,

(52) 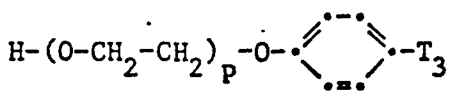 oder

(53) $H-(O-CH_2-CH_2)_p-O-CH_2-(CHOH)_s-CHO$,

worin $T_1$ Alkyl oder Alkenyl mit 7 bis 21, vorzugsweise II bis 17 Kohlenstoffatomen, $T_2$ Alkyl oder Alkenyl mit 8 bis 22, vorzugsweise 12 bis 18 Kohlenstoffatomen, $T_3$ Alkyl oder Alkenyl mit 6 bis 14, vorzugsweise 8 bis 12 Kohlenstoffatomen, p eine ganze Zahl von 1 bis 50, vorzugsweise 1 bis 20, und s 3 oder vorzugsweise 4 bedeuten.

In Formel (49) leiten sich die Reste $T_1COO$- von den entsprechenden gesättigten oder ungesättigten Fettsäuren mit 8 bis 22, vorzugsweise 12 bis 18 Kohlenstoffatomen ab. Hierbei handelt es sich im allgemeinen um die bei der Definition von $L_2$, $L_3$ und $L_4$ der Formel (5) vorstehend genanntem Fettsäurem bzw. Fettsäuregemische.

Die Fettsäureamid- und Fettalkoholreste $T_1$-CO-NH- und $T_2$-O-in den Formeln (50) und (51) leiten sich vorzugsweise ebenfalls von den entsprechenden erwähnten Fettsäuren ab.

Bevorzugte Alkylreste für $T_3$ in Formel (52) sind z.B. Alkylreste mit 6 bis 12 Kohlenstoffatomen wie n-Hexyl, i-Hexyl, n-Heptyl, n-Octyl, i-Octyl, n-Nonyl, i-Nonyl, n-Decyl, n-Dodecyl, ferner Alkylreste mit 12 bis 14 Kohlenstoffatomen, wie Lauryl und Myristyl, Alkenylreste wie Oleyl und Reste, die sich von dimerisierten Olefinen mit je 6 oder 7 Kohlenstoffatomen ableiten.

Als äthoxylierte Kohlenhydrate kommen je nach dem Wert von s in Formel (53) vor allem äthoxylierte Pentosen und insbesondere Hexosen, z.B. äthoxylierte Glukose, in Betracht.

Gegebenenfalls können die äthoxylierten Tenside der Formel (49) bis (53) auch in verätherter Form (Alkyläther mit 1 bis 20 Kohlenstoffatomen im Aetherteil) vorliegen.

Unter den äthoxylierten, nicht-ionischen Tensiden der Formeln (49) bis (53) sind die äthoxylierten Fettsäuren und Fettalkohole der Formeln (49) und (51) und insbesondere die äthoxylierten Alkylphenole der Formel (52) bevorzugt. Als spezifische Beispiele solcher äthoxylierter Alkylphenole sei u.a. das Tensid der Formel

(54) 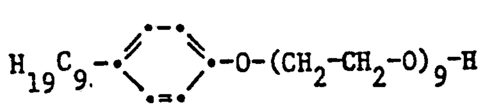

genannt.

Als nicht-ionisches Tensid für die Komponente (C) des erfindungsgemässen Gemisches sind ebenfalls handelsübliche Produkte aus Aethylenoxyd und Propylenoxyd bevorzugt, die durch Addition von Aethylenoxyd an Additionsprodukte aus Propylenoxyd und Propylenglykol erhältlich sind und Molekulargewichte von etwa 1000 bis etwa 15000 aufweisen. Solche Addukte stellen Blockcopolymerisate dar, die der wahrscheinlichen Formel

(55) $$HO-(CH_2-CH_2-O)_x-(CH_2-\underset{\displaystyle CH_3}{\underset{|}{CH}}-O)_y-(CH_2-CH_2-O)_z-H$$

entsprechen, worin x, y und z gleiche oder voneinander verschiedene, ganze Zahlen bedeuten. Die Werte für x, y und z hängen vom Molekulargewicht des Copolymerisates ab und stellen nur Durchschnittswerte dar. Besonders bevorzugt sind Copolymerisate mit durchschnittlichen Molekulargewichten von 2000 bis 8000, worin die Durchschnittswerte von x und z je zwischen 2 und 60 und von y zwischen 20 und 80 schwanken und somit der wahrscheinlichen Formel

(56) $$HO-(CH_2-CH_2-O)_{2-60}-(CH_2-\underset{\displaystyle CH_3}{\underset{|}{CH}}-O)_{20-80}-(CH_2-CH_2-O)_{2-60}-H$$

entsprechen.

Als nicht-ionisches Tensid für die Komponente (C) kommen auch Phosphorsäureester, Phosphorsäurepolyglykolester und Aminoxyde, vor allem solche mit Fettresten, in Frage, wobei die Phosphorsäureester und die Phosphorsäurepolyglykolester vorzugsweise der Formel

(57) $$\left.\begin{array}{l} T_3'-(O-CH_2-CH_2)_{q-1}-O \\ T_3''-(O-CH_2-CH_2)_{q'-1}-O \\ T_3'''-(O-CH_2-CH_2)_{q''-1}-O \end{array}\right\rangle P=O$$

entsprechen, worin $T_3'$, $T_3''$ und T'' voneinander verschieden oder vorzugsweise gleich sind und jeweils Alkyl mit 6 bis 14 Kohlenstoffatomen bedeuten, und q, q' und q'' voneinander verschieden oder vorzugsweise gleich sind und eine ganze Zahl von je 1 bis 13, insbesondere 7 bis 13, bedeuten, und Aminoxyde vorzugsweise einer der Formeln

(58) $$T_2-\overset{\displaystyle CH_3}{\overset{|}{\underset{\displaystyle CH_3}{\underset{|}{N}}}}=O$$ und insbesondere

(59) $$T_1-CO-NH-(CH_2)_3-\overset{\displaystyle CH_3}{\overset{|}{\underset{\displaystyle CH_3}{\underset{|}{N}}}}=O$$

entsprechen, worin $T_1$ und $T_2$ die angegebenen Bedeutungen haben.

Geeignet sind ferner auch Fettsäureester von 3- bis 6-wertigen Alkoholen mit 3 bis 6 Kohlenstoffatomen oder von Mono- oder Disacchariden (Saccharose). Die Fettsäurereste leiten sich z.B. von gesättigten oder ungesättigten Fettsäuren mit vorzugsweise 12 bis 18 Kohlenstoffatomen (Laurin-, Palmitim-, Stearim-, Oelsäure) ab. Genannt seien insbesondere die Sorbitan-Fettsäureester.

Die nicht-ionischen Temside der Formeln (49) bis (56), vor allem (49) (51), (52) und (55) und insbesondere (54) und (56), sind dem nicht-ionischen Tensiden der Formeln (57) bis (59) gegenüber bevorzugt.

Als Komponente (C) kommen auch Tenside mit intramolekular je einer positiven und negativen Ladung in Betracht, welchen im allgemeinen gegenüber dem nicht-ionischen Tensiden der angegebenen Art der Vorzug

gegeben wird. Bei diesen Tensiden handelt es sich vor allem um Betaine oder Sulfobetaine, die sich von Imidazolinderivaten oder offenkettigen, aliphatischen Aminen ableiten.

Die als Komponente (C) erfindungsgemäss eingesetzten, sich von Imidazolinderivaten ableitenden Betaine oder Sulfobetaine entsprechen vorzugsweise einer der Formeln

(60) 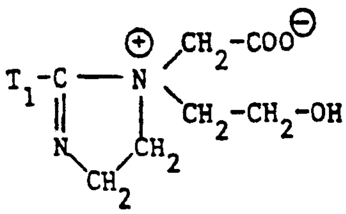

oder

(61) 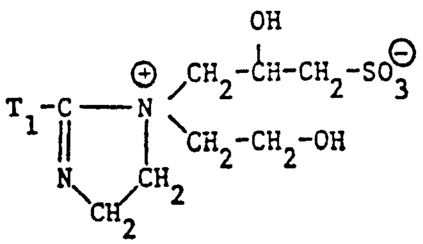 ,

worin $T_1$ die angegebenen Bedeutungen hat.

Als Beispiele spezifischer Vertreter solcher Imidazolimiumbetaine oder -sulfobetaine seien u.a. die Tenside, bzw. technischen Tensidgemische der Formeln

(62) 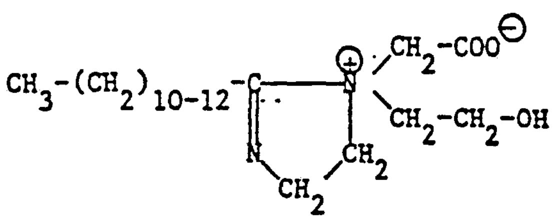

und

(63) 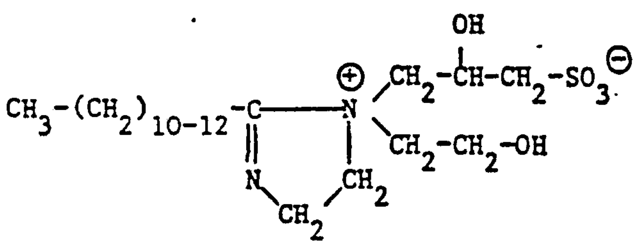

genannt.

Die erfindungsgemäss eingesetzten, sich von einem offenkettigen, aliphatischen Amin ableitenden Betainderivate entsprechen vorzugsweise einer der Formeln

(64) 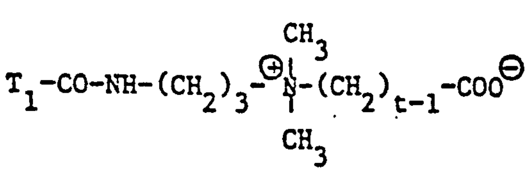 ,

(65) 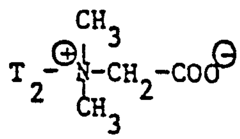 ,

(66) $T_2-\overset{\oplus}{N}H_2-CH_2-(CH_2)_{t-1}-COO^{\ominus}$ ,

(67) $T_2-(NH-CH_2-CH_2)_{t-1}-NH-CH_2-CH_2-\overset{\oplus}{N}H_2-CH_2-COO^{\ominus}$ ,

(68) 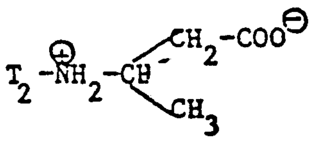 oder

(69) 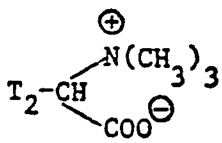 ,

worin $T_1$ und $T_2$ die angegebenen Bedeutungen haben, und t 1 oder 2 bedeutet.

Als Beispiele spezifischer Dertreter solcher Betainderivate seien u.a. die Tenside bzw. tecknischen Tensidgemische der Formeln

(70) 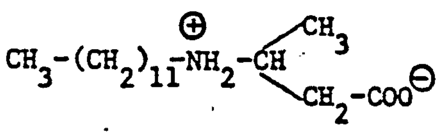 ,

(71) 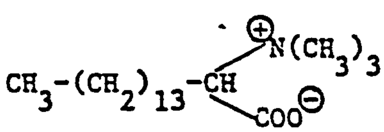 ,

insbesondere

(72) $CH_3-(CH_2)_{11-13}-\overset{\oplus}{N}H_2-(CH_2)_2-COO^{\ominus}$ ,

(73) 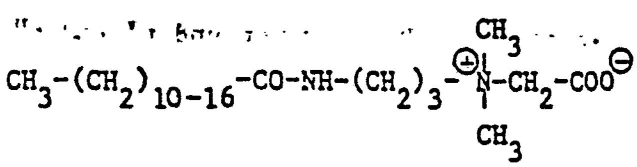 ,

(74) $CH_3-(CH_2)_{10-12}-CH_2-\overset{\oplus}{N}H_2-(CH_2)_2COO^{\ominus}$

und

(75) 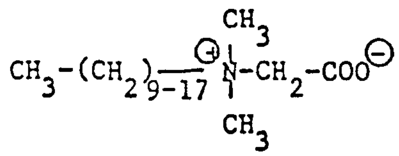

genannt.

Die erfindungsgemäss eingesetzten Sulfobetainderivate, die sich von einem aliphatischen, offenkettigen Amin ableiten, entsprechen vorzugsweise einer der Formeln

(76) 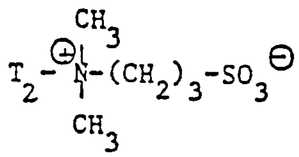 ,

(77) 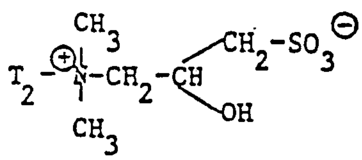 ,

(77a) 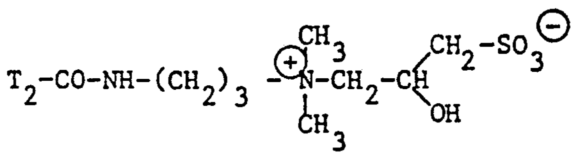

(78) 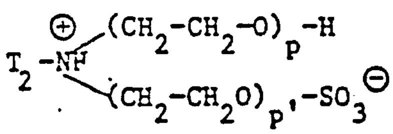 oder

(79) 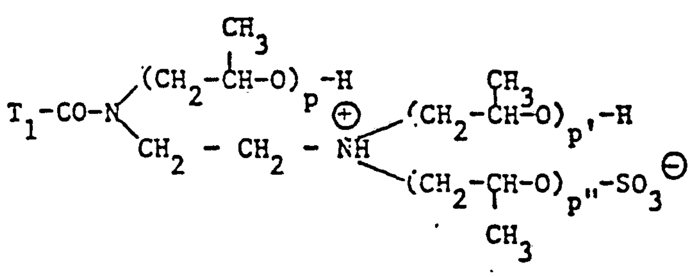 ,

worin $T_1$ und $T_2$ die angegebenen Bedeutungen und p' und p'' die für p angegebenen Bedeutungen haben, wobei p, p' und p'' gleich oder voneinander verschieden sind.

Als Beispiele spezifischer Vertreter solcher Sulfobetainderivate seien u.a. die Tenside der Formeln

(80) 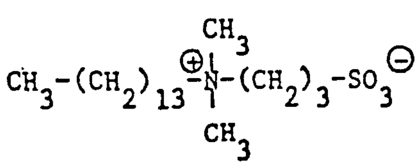 und

(81) 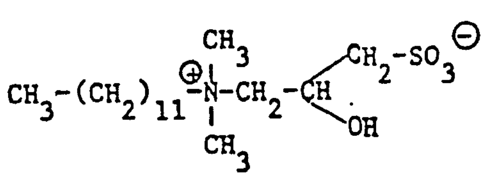

(81a) 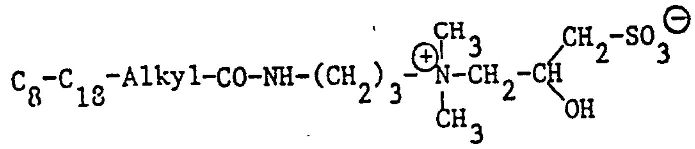

$C_8$-$C_{18}$ = technisches Gemisch aus Kokosfett aus
ca. 15% $C_8$- und $C_{10}$-Alkyl
ca. 40% $C_{12}$-Alkyl
ca. 30% $C_{14}$-Alkyl umd
ca. 15% $C_{16}$- und $C_{18}$-Alkyl.
genannt.

Als Komponente (C) des erfindungsgemässen Gemisches stehen nichtionische Tenside der Formeln (56), vor allem (54) und insbesondere Tenside mit intramolekular je einer positiven und negativen Ladung der Formeln (62), (63) und (72) bis (75) im Vordergrund des Interesses.

Anionische, gegebenenfalls zwitterionische Tenside, die als Ausführumgsform der Komponente (C) in den erfindungsgemässen Zusammensetzungen enthalten sind, entsprechen der Formel

(82) $X^{\ominus}\ Z^{\oplus}$ ,

worin $Z^{\oplus}$ ein Alkalimetall-, insbesondere Natrium- oder ein Ammoniumkation bedeutet, wobei das Ammoniumkation ($NH_4$+74) unsubstituiert oder vorzugsweise durch 1, 2 oder vor allen 3 Alkyl- oder Alkanolreste mit je 1 bis 4 Kohlenstoffatomen substituiert ist und $X^{\ominus}$ für den Rest eines anionischen, gegebenenfalls zwitterionischen Tensids steht.

Anionische Tenside der Formel (82) sind den Betainen oder Sulfobetainen und vor allem den nicht-ionischen Tensiden der vorstehend angegebenen Art gegenüber bevorzugt.

Falls $X^{\ominus}$ in Formel (82) für den Rest eines anionischen, jedoch nicht zwitterionischen Tensids steht, handelt es sich vorzugsweise um Reste oberflächenaktiver Sarkosinate, Sulfate, z.B. Alkylsulfate, Alkyläthersulfate, Alkylamidsulfate, Alkylamidäthersulfate, Alkylarylpolyäthersulfate oder Monoglyceridsulfate, Sulfonate, z.B. Alkylsulfonate, Alkylamidsulfonate, Alkylarylsulfonate, α-Oelfinsulfonate oder Sulfobernsteinsäurederivate, z.B. Alkylsulfosuccinate, Alkyläthersulfosuccinate, Alkylamidsulfosuccinate, Alkylamidpolyäthersulfosuccinate oder Alkylsulfosuccinamide, ferner auch um Reste von fluorierten Tensiden oder Phosphattensiden, wie z.B. Alkyl- oder Alkylätherphosphaten.

In Frage kommen z.B. für Sarkosinat-Tenside solche der Formel

(83) 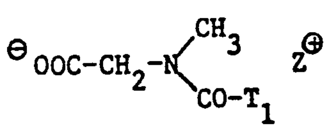 ,

worin $T_1$ und $2^{\ominus}$ die angegebenen Bedeutungen haben.

In Formel (83) leiten sich die Reste $T_1CO$- von dem entsprechenden gesättigten oder ungesättigten Fettsäuren mit 8 bis 22, vorzugsweise 12 bis 18 Kohlenstoffatomen ab. Hierbei handelt es sich im allgemeinen um die bei der Definition von $L_2$, $L_3$ und $L_4$ der Formel (5) vorstehend erwähnten Fettsäuren bzw. Fettsäure-Gemische. Als spezifische Vertreter von Sarkosinat-Tensiden seien vor allem solche der Formeln

(84) 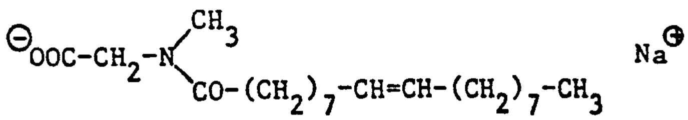

und insbesondere

(85) 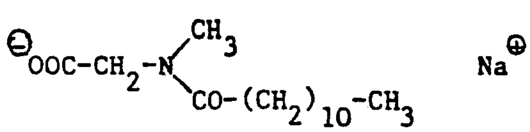 ,

genannt.

Falls $X^{\ominus}$ einen Sulfatrest (von Alkylsulfaten, sowie von Aether-, Ester-, Amid- oder Aminosulfaten) bedeutet, kommen z.B. insbesondere Tenside der Formeln

(86) $$T_1-O-(CH_2-CH_2-O)_{p-1}-SO_3^{\ominus}Z^{\oplus},$$

(87) 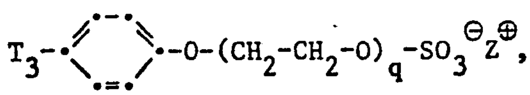

(88) $$T_1-CO-NH-(CH_2)_r-O-SO_3^{\ominus}Z^{\oplus}$$ oder

(89) $$T_1-CO-NH-(CH_2-CH_2-O)_p-SO_3^{\ominus}Z^{\oplus}$$

in Betracht, worin $T_1$, $T_3$ und $Z^{\oplus}$ die angegebenen Bedeutungen haben und p eine ganze Zahl von 1 bis 50, q eine ganze Zahl von 6 bis 12 und r eine ganze Zahl von 2 bis 6 bedeuten.

Als spezifische Vertreter von Sulfat-Tensiden seien vor allen solche der Formeln

(90) $$^{\ominus}O_3S-O-(CH_2)_{2-4}-NH-CO-(CH_2)_{11}-CH_3\ Na^{\oplus},$$

(91) $$^{\ominus}O_3S-(O-CH_2-CH_2)_{8-10}-NH-CO-(CH_2)_{11}-CH_3\ Na^{\oplus},$$

(92) 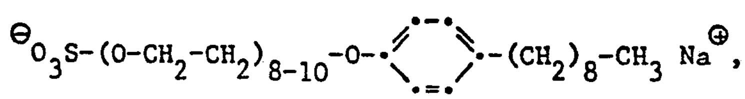

insbesondere

(93) $$^{\ominus}O_3S-(O-CH_2-CH_2)_2-O-(CH_2)_{11}-CH_3\ Na^{\oplus}$$ und

(94) 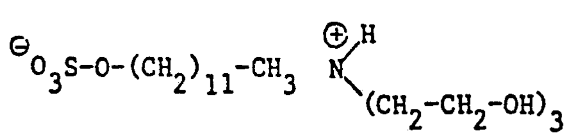

genannt.

Ist $X^{\ominus}$ ein Sulfonatrest, so kommen Tenside der Formeln

(95) 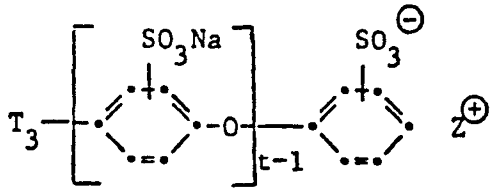 ,

(96) 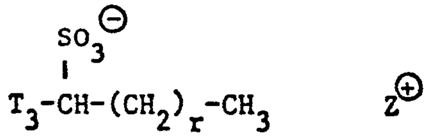 ,

(97) $T_3-COO-CH_2-CH_2-SO_3^{\ominus}$ $Z^{\oplus}$ ,

(98) 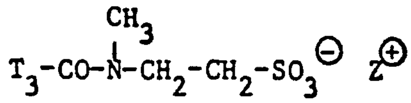 ,

(99) 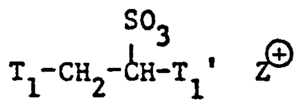 ,

(100) $T_1-CH=CH-SO_3^{\ominus}$ $Z^{\oplus}$ oder

(101) 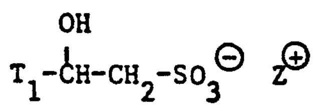

in Betracht, worin $T_1$, $T_3$, $Z^{\ominus}$ und r die angegebenen Bedeutungen haben, $T_1'$ die für $T_1$ angegebenen Bedeutungen hat, wobei $T_1$ und $T'_1$ gleich oder voneinander verschieden sind und t 1 oder 2 bedeutet.

Als spezifische Vertreter solcher Sulfonat-Tenside seien vor allem die Reste der Formeln

(102) 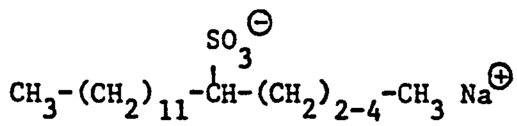 ,

(103) $^{\ominus}O_3S-(CH_2)_2-OOC-(CH_2)_{10}-CH_3$ $NH_4^{\oplus}$

(104) 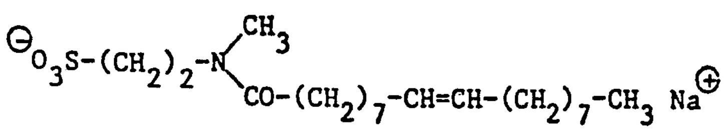 ,

insbesondere

(105) 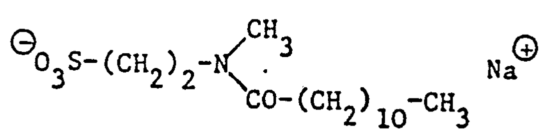 und

(106) 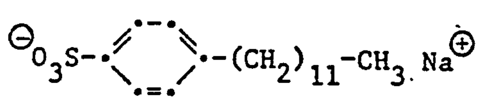

erwähnt.

Bedeutet X⊖ den Rest eines Sulfobernsteinsäurederivats, so entspricht das Tensid z.B. einer der Formeln

(107) 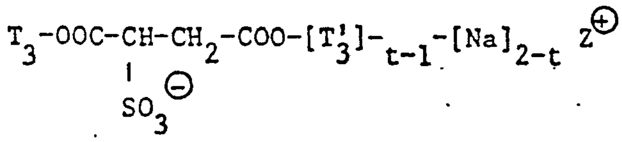 ,

(108) 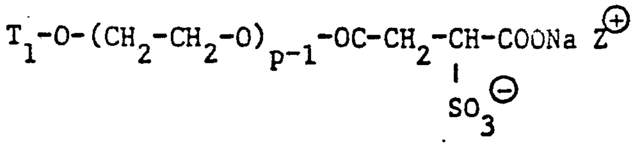 ,

(109) 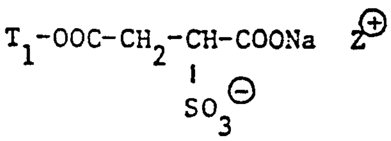 ,

(110) 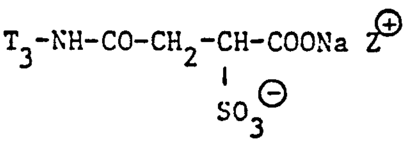 ,

(111) 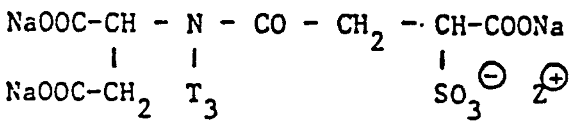 oder

(112) 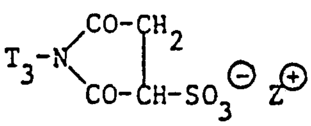

worin $T_1$, $T_3$, $Z^{\oplus}$, p und t die angegebenen Bedeutungen haben und $T_3'$ die für $T_3$ angegebenen Bedeutungen hat, wobei $T_3$ und $T'_3$ gleich oder voneinander verschieden sind.

Als spezifische Vertreter solcher Tenside seien die Sulfobernsteinsäurederivate der Formeln

(113) 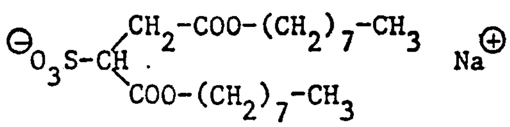 ,

(114) 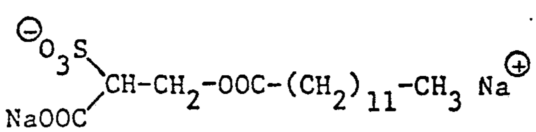 ,

(115) 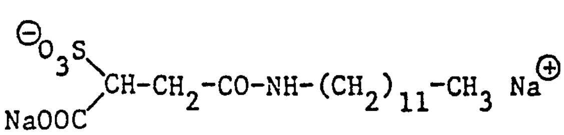 ,

(116) 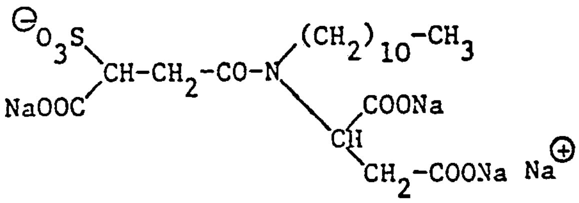 ,

(117) 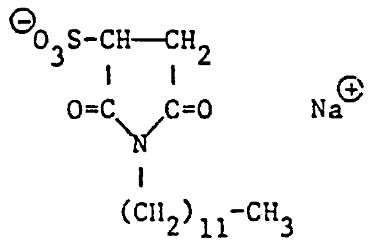 und insbesondere

(118) 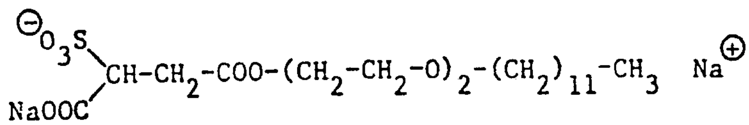

erwähnt.

Als mögliches fluoriertes Tensid kommen z.B. Tenside der Formel

(119) 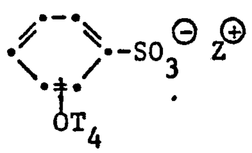

in Frage, worin $T_4$ perfluoriertes Alkyl oder Alkenyl mit 6 bis 14 Kohlenstoffatomen bedeutet und $Z^{\oplus}$ die angegebenen Bedeutungen hat.

Das perfluorierte Tensid der Formel

(120) $$^{\ominus}O_3S-C_6H_4OC_{10}F_{19}Na^{\oplus}$$

stellt ein spezifisches Beispiel solcher Tenside dar.

Als Phosphattenside kommen vor allem solche der Formeln

(121) 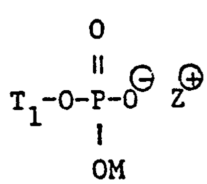 ,

(122) 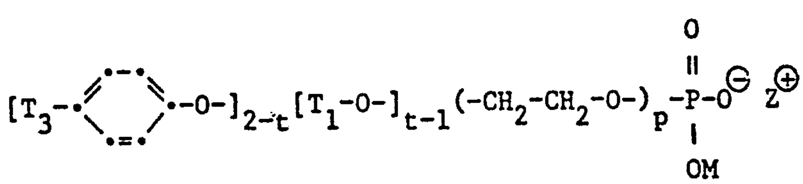

oder

(123) 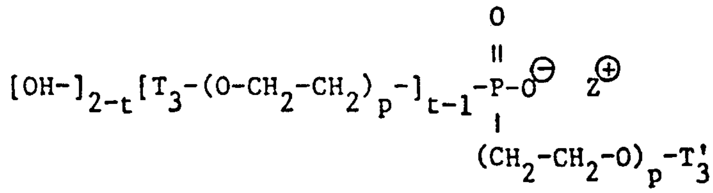

in Frage worin M Wasserstoff, Ammonium ein Alkalimetall oder Alkyl mit 1 bis 3 Kohlenstoffatomen ist T $T_3$ $T'_3$ $Z^{\ominus}$ p und t die angegebenen Bedeutungen haben, p' die für p angegebene Bedeutung hat und p und p' gleich oder voneinander verschieden sind.

Als Beispiele solcher Phosphattenside seien solche der Formeln

(124) 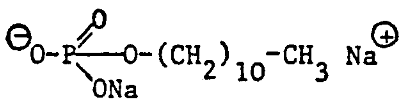 ,

(125) 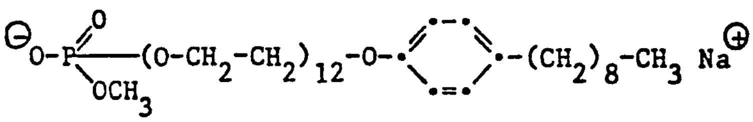 ,

(126) 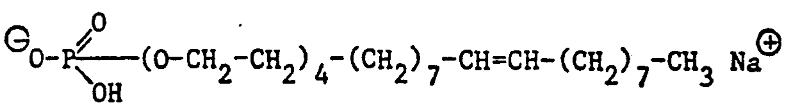 ,

(127) 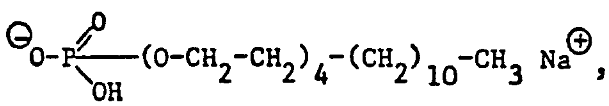 ,

(128) 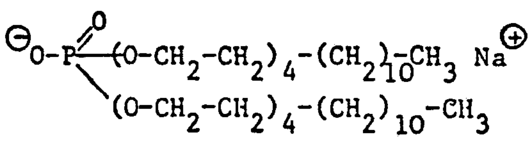 ,

(129) 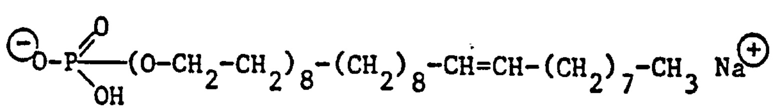 ,

(130) 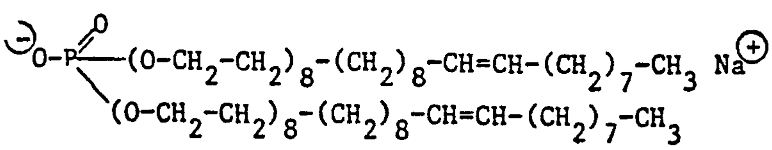 ,

(131) 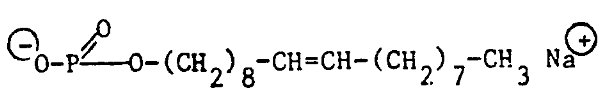 ,

(131a) 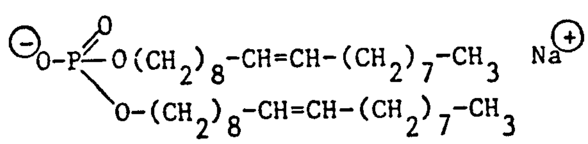 ,

und deren Gemische z.B. technische Gemische von Phosphattensidresten der Formeln (127) und (128), der Formeln (129) und (130) oder der Formeln (131) und (131a) genannt.

Bei den Resten $X^{\ominus}$ in der Bedeutung von Resten zwitterionisch anionischer Tenside in Formel (82) handelt es sich um Tensidreste, die in der Regel eine positive und zwei negative Ladungen aufweisen, d.h. um ampholytische Tensidreste, die stets einen Ueberschuss an negativen Ladungen aufweisen. Als Reste solcher zwitterionisch anionischer Tenside kommen vor allem Reste von oberflächenaktiven N-Alkyl-α-iminodipropionaten und insbesondere von in 2-Stellung alkylsubstituierten Imidazoliniumdicarbonsäurederivaten in Betracht.

Oberflächenaktive, alkylsubstituierte Iminodipropionate entsprechen vorzugsweise der Formel

(132) 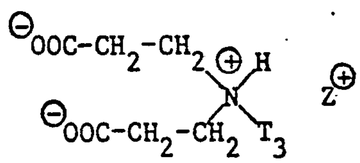 ,

worin $T_3$ und $Z^{\oplus}$ die angegebenen Bedeutungen haben.

Als spezifisches Beispiel seien die Tenside der Formeln

(133) 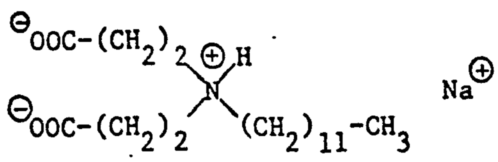

und

(133a) 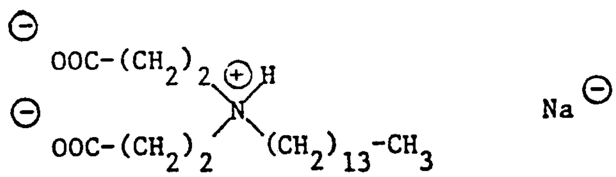

oder technische Gemische aus den Tensioen der Formeln (133) und (133a) genannt.

Bei den bevorzugten Imidazolinium-Tensiden der angegeben Art handelt es sich vor allem um solche der Formel

(134) 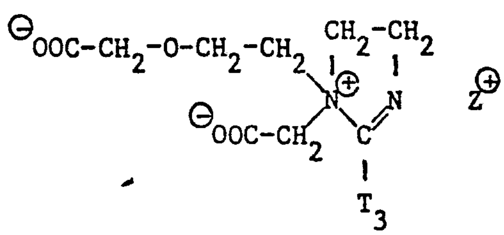 ,

worin $T_3$ und $Z^{\oplus}$ die angegebenen Bedeutungen haben. Als spezifische Beispiele seien die Tenside der Formeln

(135) 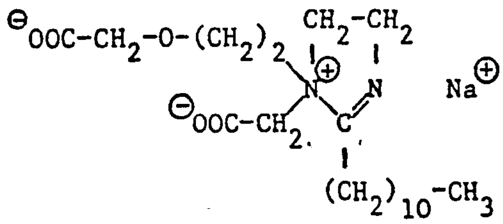 oder

(136) 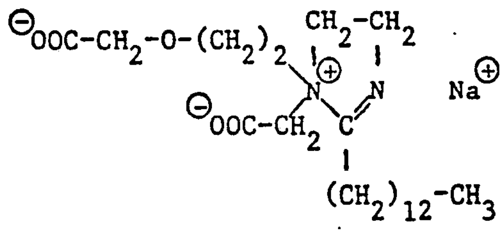

oder deren technische Gemische erwähnt.

Im Vordergrund des Interesses stehen als Tenside für die Komponente (C) der erfindungsgemässen Zusammensetzungen oberflächenaktive Sarkosinate, Sulfate, Sulfonate, Sulfobernsteinsäurederivate und Imidazolinium-dicarbonsäurederivate und insbesondere solche der Formeln (85), (92), (94), (96), (105), (106), (121), (135) und (136).

Bevorzugte erfindungsgemässe Zusammensetzungen enthalten die Komponenten (A), (B) und (C) in einem Gewichtsverhältnis (A):(B):(C) von 1: (1 bis 10): (2 bis 400), insbesondere 1: (2 bis 6): (20 bis 200).

Zur Herstellung der erfindungsgemässen Zusammensetzung wird z.B. so verfahren, dass man ein Monomer der Formel

(137) 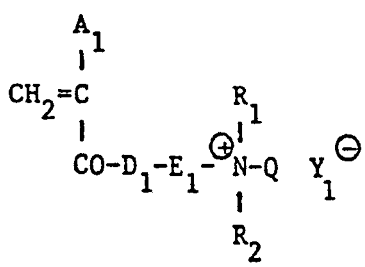

und gegebenenfalls mindestens ein Comonomer einer der Formeln

(138) 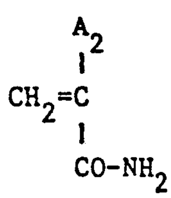 ,

(139) 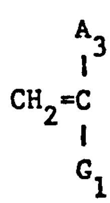 und

(140) 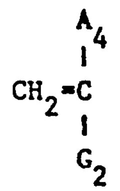 ,

worin $A_1$, $A_2$, $A_3$, $A_4$, $D_1$, $E_1$, $G_1$, $R_1$, $R_2$ Q und $Y_1\ominus$ die angegebenen Bedeutungen haben, durch Wasser-in-Oel Emulsionspolymerisation in Gegenwart eines Wasser-in-Oel Emulgators und gegebenenfalls eines Emulsionsstabilisators oder durch Lösungspolymerisation, jeweils in Gegenwart eines Polymerisationsinitiators zur Komponente (A) polymerisiert, das Polymerisat mit einem sowohl in Wasser als auch in Oel löslichen Lösungsmittel ausfällt und anschliessend trocknet, wobei das Verfahren dadurch gekennzeichnet ist, dass man das so erhaltene Polymerisat als Komponente (A) einsetzt und in wässrigem Medium mit mindestens einem 1 bis 3 quaternäre Stickstoffatome und ein Molekulargewicht von höchstens 9000 aufweisenden Ammoniumsalz als Komponente (B) sowie mit mindestens einem nicht-ionischen Tensid, einem Tensid mit intramolekular je einer positiven und negativen Ladung oder einem anionischen, gegebenenfalls zwitterionischen Tensid als Komponente (C) bei 10 bis 90° vorzugsweise 10 bis 60°C und einem pH-Wert von 3 bis 9 vermischt, wobei man gleichzeitig, sofern ein anionisches, gegebenenfalls zwitterionisches Tensid als Komponente (C) der Formel (82) eingesetzt wird, das anionische Tensid mit dem unter Einsatz des Comonomeren der Formel (138) hergestellte Copolymerisat als Komponente (A) und mit dem 1 bis 3 quaternäre Stickstoffatome aufweisenden Ammoniumsalz als Komponente (B) unter Ionenaustausch mindestens teilweise umsetzt.

Arbeitet man nach der Methode der Lösungspolymerisation, so ist der Einsatz von Emulgatoren und Emulsionsstabilisatoren nicht notwendig. Als Lösungsmittel dient in der Regel Wasser.

Bei der Wasser-in-Oel Emulsionspolymerisation werden für die Oelphase hydrophobe, organische Flüssigkeiten benötigt. Zu diesem Zweck eignen sich z.B. aliphatische oder aromatische Kohlenwasserstoffe, Oele tierischer oder pflanzlicher Herkunft und die entsprechenden denaturierten Oele (.z.B. hydrierte Oele, polymerisierte Oele). Zu den bevorzugten hydrophoben organischen Flüssigkeiten gehören aliphatische Kohlenwasserstoffe wie Kerosin, Paraffin, Isoparaffin und aromatische Kohlenwasserstoffe wie Benzol, Toluol und Xylol. Handelsübliche technische Gemische von vorzugsweise verzweigten Paraffinölen mit einem Siede-Bereich von 160-260°C, vorzugsweise 180 bis 210°C, stchen im Vordergrund des Interesses.

Als bei der inversen Emulsionspolymerisation eingesetzte Wasser-in-Oel Emulgatoren kommen Polyoxyalkylen-, vorzugsweise Polyoxyäthylenaddukte von aliphatischen Alkoholen mit 8 bis 24 Kohlenstoffatomen wie Lauryl-, Cetyl-, Stearyl- und Oleylalkohole, von Fettsäuren mit 8 bid 24 Kohlenstoffatomen der vorstehend angegebenen Art, vorzugsweise Laurin-, Palmitin-, Stearin- umd Oelsäure, von Alkylphenolen mit 8 bis 24 Kohlenstoffatomen im Alkylrest, z.B. Octyl-, Nonyl-, Dodecyl- und Dinonylphenol und von Estern von Fettsäuren der angegebenen Art mit mehrwertigen Alkoholen, z.B. Glycerin, Pentaerythrit, Sorbit und Sorbitan in Betracht. Auch handelsübliche Gemische, d.h. Polyoxyalkylenaddukte von technischen Alkoholgemischen, Fettsäuregemischen, Alkylphenolgenischen und Estergemischen sind als Wasser-in-Oel Emulgatoren besonders geeignet. Besonders bevorzugt sind indessen Ester der Fettsäuren der angegebenen Art bzw. Fettsäuregemische mit mehrwertigen Alkoholen der angegebenen Art, wobei Sorbitmonooleat im Vordergrund des Interesses steht.

In besonderen Fällen hat sich die Verwendung eines Emulsionsstabilisators in der Oelphase als zweckmässig erwiesen. Zu diesem Zweck eignen sich vor allem in der Oelphase lösliche Kautschuke, und zwar sowohl solche natürlicher Herkunft, z.B. Kristallgummi, als auch vorzugsweise solche synthetischer Herkunft, z.B. Polybutadien, Copolymerisate aus Styrol und Butadien und insbesondere Polyisopren, das im Vordergrund des Interesses steht.

In der Regel enthält die Oelphase etwa 2 bis 15 Gewichtsprozent Emulgator und 0 bis etwa 1, vorzugsweise 0,4 bis 0,8 Gewichtsprozent Stabilisator.

Nach dem Vermischen der Oelphase mit der wassrigen Phase, welche die Monomeren der Formel (137) und gegebenenfalls die Comonomeren der Formeln (138), (139) und (140) enthält, wird in der Regel die Polymerisation durch Zugabe eines Polymerisationsinitiators in Gang gesetzt. Als Initiatoren können die üblichen Polymerisationskatalysatoren, vorzugsweise in Form ihrer organischen oder wässrigen Lösung, eingesetzt werden, z.B. Azoverbindungen wie Azobis(iso-butyronitril) oder Azo-bis(dimethylvaleronitril), oxydierende Mittel, vorzugsweise Peroxyde wie Wasserstoffperoxyd oder Benzoylperoxyd oder vorzugsweise Persulfate wie Ammoniumpersulfat, ferner auch Chlorate oder Chromate, reduzierende Mittel, wie Sulfite, Bisulfite, Oxalsäure und Ascorbinsäure, sowie die Kombination, als sogenannte Redoxkatalysatoren, der vorstehend aufgeführten oxydierenden und reduzierenden Mittel. Im vorliegenden Fall eignet sich Natriumsulfit vorzugsweise als wässrige Lösung besonders gut als Initiator.

Die Polymerisation erfolgt in der Regel bei 30 bis 90°C, vorzugsweise 40 bis 70°C, und verläuft exotherm, so dass die Polymerisationstemperatur gegebenenfalls durch Kühlen eingehalten werden muss.

Zur üblichen Aufarbeitung wird das erhaltene Polymerisat in der Regel durch ein vorzugsweise mit Oel und Wasser mischbares Lösungsmittel gefällt, z.B. mit Methanol, Isopropanol oder Aceton, wobei die Fällung im allgemeinen durch Zugabe der Polymerisationslösung bzw. der Wasser-in-Oel Emulsion ins vorgelegte

Lösungsmittel, vorzugsweise bei Raumtemperatur (15 bis 25° C), durchgeführt wird, worauf nach der Filtration das gefällte Polymerisat vorzugsweise bei Temperaturen von höchstens 60° C, insbesondere bei Temperaturen von etwa 30 bis 50° C, zweckmässig unter vermindertem Druck getrocknet wird.

Um nötigenfalls eine Anreicherung an hochmolekulare Anteile (Molekulargewicht von $10^7$ bis $10^9$ innerhalb der breiten Molekulargewichtsverteilung von $10^4$ bis $10^9$) zu bewirken, kann das Polymer einer zusätzlichen Behandlung in den genannten Lösungsmitteln unterworfen werden. Eine solche Behandlung kommt vor allem für Polymere in Frage, die durch Lösungspolymerisation hergestellt werden.

Die an sich bekannten, so erhaltenen Homo- oder vorzugsweise Copolymerisate der Komponente (A) werden nun nach an sich bekannten Methoden mit den mono- bis oligomeren, quaternären Ammoniumsalzen als Komponente (B) und mit mindestens einem nicht-ionischen Tensid einer der Formeln (49) bis (81) und/oder einem anionischen Tensid einer der Formeln (82) bis (136) als Komponente (C), vorzugsweise bei Raumtemperatur (10 bis 25° C) miteinander vermischt, oder, sofern ein Copolymerisat als Komponente (A) und ein anionisches, gegebenenfalls zwitterionisches Tensid als Komponente (C) eingesetzt wird, bei bevorzugten, leicht erhöhten Temperaturen von 15 bis 90°,vorzugs-weise 15 bis 40° C, während 30 bis 100, insbesondere 60 bis 90 Minuten, mindestens teilweise miteinander umgesetzt, wobei in der Regel ein Ueberschuss mindestens eines anionischen, gegebenenfalls zwitterionischen Tensids der Formel (82), bezogen auf das monomere bis oligomere, quaternäre Ammoni-umsalz, z.B. der Formel (5) und auf das bei der Herstellung des Copolymerisats, angewandte, comonomere, quaternäre Ammoniumsalz der Formel (137) eingesetzt wird. Dieser Ueberschuss beträgt etwa 4 bis 500, vorzugsweise 5 bis 120 und insbesondere 7 bis 70 Mol Tensid pro Mol des angewandten monomeren Ammoniumsalzes.

Bei ihrer Verwendung in der Kosmetikindustrie werden die erfindungsgemässen Gemische der Komponenten (A), (B) und (C) vorzugsweise als Haarkosmetika eingesetzt.

Die erfindungsgemässen kosmetischen Mittel, vorzugsweise haarkosmetische Mittel, liegen in ihrer bevorzugten Ausführungsform als wässrige Lösungen vor, die z.B. 0,05 bis 1,5, vorzugsweise 0,2 bis 1,0 Gewichtsteile, berechnet als Wirksubstanz, mindestens eines polymeren, quaternären Ammoniumsalzes als Komponente (A), 0,1 bis 10, vorzugsweise 0,2 bis 6 Gewichtsteile, berechnet als Wirksubstanz, mindestens einem monomeren bis oligomeren, quaternären Ammoniumsalzes als Komponente (B) und 5 bis 20, vorzugsweise 8 bis 15, insbesondere 9 bis 12 Gewichtsteile, berechnet als Wirksubstanz, mindestens eines nicht-ionischen Tensides, oder eines Tensides mit intramolekular je einer positiven und negativen Ladung oder eines anionischen, gegebenenfalls zwitterionischen Tensides als Komponente (C) und gegebenenfalls kosmetische Hilfsmittel als Komponente (D) enthalten und mit entionisiertem Wasser auf insgesamt 100 Gewichtsteile verdünnt sind.

Die fakultativen, kosmetischen Hilfsmittel als Komponente (D) sind handelsübliche Mittel, wie sie in der Haarkosmetik eingesetzt werden. In Betracht kommen z.B. Tenside, die von den als Komponente (C) eingesetzten Tensiden der angegebenen Art verschieden sind, wie Polyglycerolester und Polyglykolester von Fettsäuren, insbesondere Polyglycerololeate, im weiteren auch Polyglykole und Schaumstabilisatoren, z.B. Fettsäurepolyalkanolamide, Verdickungsmittel natürlicher oder synthetischer Herkunft wie Hydroxypropylmethylcellulose und Polyacrylsäure, Opalisierungsmittel, wie Fettsäuremonoalkanol-amide oder vorzugsweise Glycerinmonostearat, Hautschutzmittel wie Eiweisshydrolysate und Allantoine, ferner auch u.a. Konservierungsmittel, Parfüms und Perlglanzmittel.

Erforderlichenfalls werden die haarkosmetischen Mittel auf einen pH-Wert von 3 bis 9, vorzugsweise 5 bis 9, vor allem 5 bis 8 und insbesondere 5 bis 6 eingestellt, was zweckmässig durch einen Zusatz von wässrigen Lösungen aus z.B. Natriumhydroxid oder vorzugsweise Zitronensäure erreicht werden kann.

Bei der Applikation der haarkosmetischen Mittel, vorzugsweise auf menschlichem Haar, wird im Haarbehandlungsverfahren das vorstehend beschriebene, wässrige, haarkosmetische Mittel auf mit Leitungswasser angefeuchtetem Haar, in der Regel bei Raumtemperatur bis leicht erhöhter Temperatur, z.B. 20 bis 40° C, aufgebracht und das Haar anschliessend schampooniert und gleichzeitig konditioniert. Das so behandelte Haar kann ebenfalls in Form von Perücken oder Toupets vorliegen.

Der wesentliche Vorteil der vorliegenden Erfindung liegt darin, dass bei der Applikation der haarkosmetischen Mittel, welche in neuartiger Kombination sowohl die polymeren als auch die monomeren bis oligomeren, quaternären Ammoniumsalze als Komponenten (A) und (B) neben den Tensiden der angegebenen Art als Komponente (C) enthalten, nicht nur eine gute Reinigung, sondern auch gleichzeitig ausgezeichnete Konditioniereffekte auf dem behandelten Haar erzielt werden. So weisen die mit der erfindungsgemässen Zusammensetzung behandelten Haare antistatische Eigenschaften und eine sehr gute Kämmbarkeit (nass und trocken) auf. Zudem ist bei wiederholter Verwendung der erfindungsgemässen Zusammensetzung keinerlei unerwünschter Akkumuliereffekt unter Beeinträchtigung des Griffs, der Fülle und des Glanzes der behandelten Haare zu beobachten. Im Vergleich zu den Konditioniereffekten auf Haaren, die mit herkömmlichen kosmetischen Mitteln behandelt werden, weisen die mit den erfindungsgemässen haarkosmetischen Mitteln behandelten Haare somit in unerwarteter Weise ausserordentlich gute Konditioniereffekte auf. Die üblichen kosmetischen Mittel enthalten zwar auch Mischungen aus Tensiden und quaternären Ammoniumsalzen, jedoch nicht die neuartige und erfinderische Kombination von polymeren und monomeren bis oligomeren, quaternären Ammoniumsalzen gemäss der vorliegenden Erfindung.

In den nachfolgenden Herstellungsvorschriften und Beispielen angegebene Teile und Prozente sind Gewichtseinheiten.

**Herstellungsvorschriften für die Komponente (A)**

Vorschrift A: (Wasser-in-Oel Emulsionscopolymerisat) Die folgende drei Lösungen werden in sauerstofffreier, inerter Stickstoffatmosphäre vorbereitet:

**Lösung I**

(Oelphase)
In einem Doppelmantelreaktionsgefäss werden
500 Teile eines verzweigten Paraffinöls (technisches Gemisch, Molekulargewicht 171, Siedebereich 188-206° C) vorgelegt.
140 Teile einer 2,5%igen Lösung eines synthetischen Kautschuks aus Polyisopren (Emulsionsstabilisator) in Paraffinenöl der angegebenen Art und anschliessend
78 Teile Sorbitanmonooleat (Wasser-in-Oel Emulgator) werden dem vorgelegten Paraffinöl unter Rühren bei 20° C zugegeben.
Man erhält eine klare, gelbe Lösung.

**Lösung II**

(wässrige Phase)
568,6 Teile Acrylamid (8 Mol) werden bei 20° C in
700 Teilen entionisiertem, sauerstofffreiem Wasser gelöst. In diese Lösung werden
220 Teile Natriumchlorid unter Rühren eingetragen und anschliessend
1133,2 Teile einer 50%igen, wässrigen Lösung aus Methacryloyl-oxyätyl-trimethylammonium-methylsulfat (2 Mol) zugegeben.
Man erhält eine klare, farblose Lösung.

**Lösung III**

(Initiatorlösung)
0,66 Teile Natriumsulfit werden in
40 Teile entionisiertem, sauerstofffreiem Wasser gelöst.

**Copolymerisationsreaktion**

Unter intensivem Rühren (3000 U/min) wird innerhalb von 10 Minuten in inerter Stickstoffatmosphäre die Lösung II in die vorgelegte Lösung 1 bei 20° C zugegeben. Man erhält eine homogene, weisse Emulsion, die bei 20° C weitergerührt wird, bis die Viskosität einer Emulsionsprobe 14000 mPa.s (Brookfield Viskosimeter LV, Spindel 3, 6 U/min, 25° C) beträgt, was im allgemeinen 10 Minuten in Anspruch nimmt. Hierauf wird das Reaktionsgemisch unter Rühren bei 300 U/min innerhalb von 30 Minuten auf 40° C geheizt. Mittels einer Dosierpumpe wird nun die Lösung III innerhalb von 150 Minuten in das Reaktionsgemisch zugegeben, wobei die Temperatur durch Kühlen auf 40 bis 41° C gehalten wird. Nach Beendigung der Initiatorlösung-Zugabe wird das Reaktionsgemisch bei 40° C und 300 U/min weitergerührt, bis die Viskosität einer Emulsionsprobe auf 7600 mPa.s. (Brookfield Viskosimeter LV, Spindel I, 60 U/min, 25° C) absinkt, was im allgemeinen eine Stunde in Anspruch nimmt.

**Aufarbeitung**

Die erhaltene Emulsion des Copolymerisates wird unter Rühren in 24000 Teilen Aceton bei 20° C gegeben und das Copolymerisat ausgefällt. Das ausgefällte Copolymerisat wird abfiltriert und während 2 Tagen unter vermindertem Druck bei 40° C getrocknet. Man erhält 1100 Teile eines Copolymerisates, das in beliebiger Reihenfolge 80 Mol% Strukturelemente der Formel

(141) $-CH_2-CH(CONH_2)-$ und

20 Mol% Strukturelemente der Formel (142)

(142) 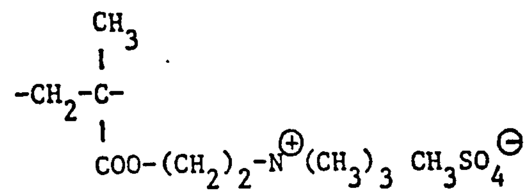

enthält, wobei das Molekulargewicht von 37% des Copolymerisates zwischen $10^7$ und $10^9$ liegt.

Die molekulare Gewichtsverteilung wird mittels der Gelpermeationschromatographie bestimmt, wobei als Trägermaterial FRACTOGEL® (Handelsmarke von MERCK und EM LABORATORIES) Typ OR-PVA eingesetzt wird, welches zweckmässig in Formamid als Lösungsmittel gequollen wird. Einzelheiten über das Trägermaterial sind aus dem Lehrbuch "Modern Size-Exclusion Chromatography" von W.W. Yau, J.J. Kirkland and D.D. Bly, Seiten 166 bis 173, Verlag John Wiley & Sons (1979) zu entnehmen. Als Detektor dient ein Differenzialrefraktometer.

Die Auswertung der GPC-Chromatogramme erfolgt anhand einer Eichkurve, die mit Polystyrol, Polymethylmethacrylat und den Standard-Polymeren SEPHADEX DEXTRAN® (Handelsmarke der Firmen PHARMACIA UPPSALA und PRESSURE CHEMICAL CORP., Pittsburg) erstellt wird. Die Konzentrationen werden über die unkorrigierten Flächenprozente ermittelt, wobei die Summe aller eluierten Komponenten 100 % beträgt.

**Vorschrift B:**

(Lösungscopolymerisat)

Die folgenden zwei Lösungen werden in sauerstofffreier inerter Stickstoffatmosphäre vorbereitet:

**Lösung 1**

(Monomerlösung)

In einem Doppelmantelgefäss werden

71,1 Teile Acrylamid (1 Mol) und

141,7 Teile einer 50%igen, wässrigen Lösung aus Methacryloyl-oxyäthyltrimethylammonium-methylsulfat (0,25 Mol) bei 20° C in

228 Teilen entionisiertem, sauerstofffreiem Wasser gelöst.

Man erhält eine klare farblose Lösung.

**Lösung II**

(Initiatorlösung)

0,2 Teile Ammoniumperoxodisulfat werden in 150 Teilen deionisiertem sauerstofffreiem Wasser gelöst.

**Copolymerisitionsreaktion**

Unter Rühren wird innerhalb 1 Minute in inerter Stickstoffatmosphäre die Hälfte der Lösung II in die vorgelegte Lösung 1 bei 35° C zugegeben. Nach 6 Stunden wird die Reaktionslösung auf 50° C erwärmt und die zweite Hälfte von Lösung II zugegeben. Das Reaktionsgemisch wird solange unter Rühren gehalten, bis nach 2 bis 3 Stunden eine hochviskose Lösung entstanden ist. Ohne Rühren wird das Reaktionsgemisch stehen gelassen. Nach 24 Stunden lässt man das entstandene farblose Gel abkühlen.

**Aufarbeitung**

Das Gel wird zerkleinert und in 1350 Teilen deionisiertem Wasser gelöst. Die hochviskose Lösung wird dann in 18000 Teilen Aceton in dünnem Strang bei 20°C eingepresst und das Copolymerisat ausgefällt. Das Copolymerisat wird dann abfiltriert und nochmals in 1800 Teilen Aceton geknetet, bis es hart und spröde wird. Das Copolymerisat wird wieder abfiltriert und während 2 Tagen unter vermindertem Druck bei 40°C getrocknet. Man erhält 110 Teile eines Copolymerisats, das in beliebiger Folge 80 Mol% Strukturelemente der Formel (141) und 20 Mol% Strukturelemente der Formel (142) enthält, wobei das Molekulargewicht von 12% des Copolymerisats zwischen $10^7$ und $10^9$ liegt. Hierbei wird diese Molekulargewichtsverteilung wie in Vorschrift A angegeben bestimmt.

**Vorschrift**

C: (Behandlung eines Handelsproduktes)
100 Teile eines im Handel erhältlichen Copolymerisates, das zu 75 Mol-% aus Strukturelementen der Formel (141) und zu 25 Mol-% aus Elementen der Formel

(142) 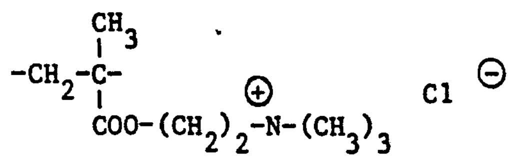

besteht, wobei das Molekulargewicht von 20 % des Copolymerisates zwischen $10^7$ und $10^9$ liegt, werden pulverisiert und mit einem Gemisch von 400 Teilen Methanol und 100 Teilen Wasser bei Raumtemperatur 30 Minuten lang gerührt. Weitere 500 Teile Methanol werden zugesetzt und das Gemisch noch 5 Minuten gerührt und dann eine Stunde stehen gelassen. Der gelige Brei wird dann in einer Drucknutsche unter einem Druck von 3 bar filtriert. Die gelige Masse wird anschliessend mit 100 Teilen Methanol aufgeschlämmt und wieder unter Druck abfiltriert. Nach dreimaliger Behandlung mit Methanol wird das Produkt einen Tag im Vakuum bei 40°C getrocknet. Das Molekulargewicht von 45 % des erhaltenen, umgefällten Copolymerisats liegt zwischen $10^7$ und $10^9$, wobei die Molekulargewichtsverteilung des behandelten Copolymerisats und des Ausgangspolymers (Handelsprodukt) wie in Vorschrift A angegeben bestimmt wird.

**Beispiel 1:**

2 Teile des polymeren Ammoniumsalzes gemäss Vorschrift A (1,41 m-Aequivalente, bezogen auf den Quatgehalt des Copolymerisats) und 10 Teile des monomeren Ammoniumsalzes der Formel (37) (27,74 mMol) werden bei 25 bis 35°C in kleinen Portionen in 100 Teilen entionisiertem Wasser eingetragen. Es entsteht eine viskose Lösung. 100 Teile des anionischen Tensides der Formel (94) (241 mMol oder 8,27 Mol pro Mol angewandtes Methacryloyl-oxyäthyl-trimethylammonium-methylsulfat und pro Mol monomeres, quaternäres Ammoniumsalz der Formel (94)) werden in 700 Teilen entionisiertem Wasser bei 25°C gelöst. Die Lösung der quaternären Ammoniumsalze wird nun in die Tensidlösung innerhalb von 80 Minuten zugegeben, wobei gleichzeitig die Umsetzung der quaternären Strukturelemente des Copolymerisats und die Umsetzung des monomeren, quaternären Ammoniumsalzes mit dem eingesetzten Tensid unter Ionenaustausch erfolgt. Man erhält 912 Teile einer wässrigen, schwach opalisierenden und viskosen Lösung, die durch Zugabe einer 10%-iden, wässrigen Zitronensäurelösung auf den pH-Wert von 7,1 eingestellt und mit entionisiertem Wasser auf 1000 Teile verdünnt wird. Die erhaltene, verdünnte, gebrauchsfertige Lösung ist vollständig klar und lagerstabil. Nun wird diese Lösung auf eine mit Leitungswasser angefeuchtete Perücke aus ungebleichtem und ungefärbtem, braunem Menschenhaar europäischer Herkunft bei 40°C in dreimaliger Applikation à 2 Schimponierungen im sogenannten Halbseitentest aufgebricht, worauf das Perückenhaar bei dieser Temperatur schampooniert und gleichzeitig konditioniert wird. Im Halbseitentest wird nur die eine Hälfte der Perücke mit der vorstehend genannten Lösung schampooniert und konditioniert, während die andere Hälfte der Perücke mit einer Lösung unter gleichen Bedingungen schampooniert wird, die kein erfindungsgemässes Gemisch aus den Ammoniumsalzen und dem Tensid, sondern nur das Tensid enthält. In dieser sogenannten Leerformulierung wird die Menge an Ammoniumsalzen durch die entsprechende Menge Tensid ersetzt, so dass z.B. die Leerformulierung als Vergleich 11,2% des Tensides der Formel (94) enthält, wobei der pH-Wert der Leerformulierung ebenfalls mit der wässrigen, 10%-igen Zitronensäurelösung auf den pH-Wert von 7,1 eingestellt wird. Nach jeder Applikation wird die Nass- und Trockenkämmbarkeit der erfindungsgemäss behandelten Perückenhälfte im Vergleich mit der mit der Leerformulierung behandelten Perückenhälfte mit Hilfe der nachfolgenden Notenskala beurteilt:

+3 viel besser als die Leerformulierung
+2 besser als die Leerformulierung
+1 etwas besser als die Leerformulierung
0 kein Unterschied zur Leerformulierung
-1 etwas schlechter als die Leerformulierung
-2 schlechter als die Leerformulierung
-3 viel schlechter als die Leerformulierung.
Die erzielten Kämmbarkeitsnoten sind in der nachfolgenden Tabelle I zusammengefasst.

**Tabelle I**

| | nach der 1. Applikation | nach der 2. Applikation | nach der 3. Applikation |
|---|---|---|---|
| Nasskämmbarkeit | +3 | +3 | +3 |
| Trockenkämmbarkeit | +2 | +2 | +2 |

Zudem weist die erfindungsgemäss behandelte Perückenhälfte bessere antistatische Eigenschaften auf als die mit der Leerformulierung behandelte Perückenhälfte. Die antistatischen Effekte werden qualitativ erfasst, indem die Neigung der Haare zum "Wegfliegen" (fly away) subjektiv beurteilt wird. Auf beiden Perückenhälften können keinerlei Akkumuliereffekte nach 3 Applikationen beobachtet werden.

**Beispiel 2:**

Man verfährt wie im Beispiel 1 angegeben, setzt jedoch 2 Teile des polymeren Ammoniumsalzes gemäss Vorschrift B (1,41 m-Aequivalente bezogen auf den Quatgehalt des Copolymerisates), 5 Teile des monomeren Ammoniumsalzes der Formel (48) (6,72 mMol) und 100 Teile des Tensides der Formel (93) (255 mMol oder 31,4 Mol pro Mol angewandte Methacryloyl-oxyäthyl-trimethylammonium-methylsulfat und pro Mol monomeres, quaternäres Ammoniumsalz der Formel (48)) ein.

Man erhält 907 Teile einer schwach opalisierenden und viskosen Lösung, die durch Zugabe einer 5%-igen, wässrigen Natriumhydroxydlösung auf den pH-Wert von 7,1 eingestellt und mit entionisiertem Wasser auf 1000 Teile verdünnt wird.

Das Schimpoonieren und Konditionieren der Perücke im Halbseitentest mit der verdünnten, klaren, lagerstabilen Lösung wird ebenfalls wie in Beispiel 1 durchgeführt, wobei die folgenden, in der nachstehenden Tabelle II zusammengefassten Kämmbarkeitsnoten erzielt werden:

**Tabelle II**

| | nach der 1. Applikation | nach der 2. Applikation | nach der 3. Applikation |
|---|---|---|---|
| Nasskämmbarkeit | +3 | +3 | +3 |
| Trockenkämmbarkeit | +2 | +2 | +2 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Aehnliche Ergebnisse werden beim Ersatz des monomeren, quaternären Ammoniumsalzes der Formel (48) durch diejenigen einer der Formeln (23) bis (32), (35), (36), (37), (38) oder (47) und beim Ersatz des anionischen Tensids der Formel (93) durch diejenigen einer der Formeln (85), (102), (105), (106), (118), (135) oder (136) erzielt. Dies trifft auch zu für den Ersatz des anionischen Tensids der Formel (93) durch nicht-ionische Tenside einer der Formeln (54) oder (56) oder Tenside mit intramolekular je einer positiven und negativen Ladung einer der Formeln (62), (63) oder (72) bis (75). In diesem letzten Fall braucht jedoch die Lösung der quaternären Ammoniumsalze nicht langsam in die vorgelegte Lösung des Tensides bei 35°C zugegeben zu werden, da keine

Umsetzung unter Ionenaustausch stattfindet. Die Lösungen der quaternären Ammoniumsalze und die Lösungen der nicht-ionischen Tenside oder der Tenside mit intramolekular je einer positiven und negativen Ladung können somit in beliebiger Reihenfolge bei Raumtemperatur (10 bis 25° C) durch Zugiessen vermischt werden.

**Beispiel 3**

Man verfährt wie im Beispiel 1 angegeben, setzt jedoch 2 Teile des polymeren Ammoniumsalzes gemäss Vorschrift C, 10 Teile des monomeren Ammoniumsalzes der Formel (37) in 100 Teilen entionisiertem Wasser und 150 Teile des Tensides der Formel (94) in 650 Teilen entionisiertem Wasser ein.

Man erhält 912 Teile einer schwach opalisierenden und viskosen Lösung, die durch Zugabe einer 10-%igen, wässrigen Zitronensäurelösung auf den pH-Wert von 5,5 eingestellt und mit entionisiertem Wasser auf 1000 Teile verdünnt wird.

Das Schampoonieren und Konditionieren der Perücke im Halbseitentest mit der verdünnten klaren lagerstabilen Lösung wird ebenfalls wie in Beispiel 1 durchgeführt, wobei die folgenden, in der nachstehenden Tabelle III zusammengefassten Kämmbarkeitsnoten erzielt werden.

**Tabelle III**

| | nach der 1. Applikation | nach der 2. Applikation |
|---|---|---|
| Nasskämmbarkeit | +2 | +2 |
| Trockenkämmbarkeit | +1 | +1 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 2 Applikationen beobachtet werden können.

**Beispiel 4:**

Man verfährt wie im Beispiel 1 angegeben, setzt jedoch 2 Teile des Polymeren Ammoniumsalzes gemäss Vorschrift A, 5 Teile des monomeren Ammoniumsalzes der Formel (25) in 100 Teilen entionisiertem Wasser und 150 Teile des Tensides der Formel (94) in 650 Teilen entionisiertem Wasser ein.

Man erhält 912 Teile einer schwach opalisierenden und viskosen Lösung, die durch Zugabe einer 10-%igen, wässrigen Zitronensäurelösung auf den pH-Wert von 6 Oeingestellt und mit entionisiertem Wasser auf 1000 Teile verdünnt wird.

Das Schampoonieren und Konditionieren der Perücke im Halbseitentest mit der verdünnten, klaren, lagerstabilen Lösung wird ebenfalls wie in Beispiel 1 durchgeführt, wobei die folgenden, in der nachstehenden Tabelle IV zusammengefassten Kämmbarkeitsnoten erzielt werden:

**Tabelle IV**

| | nach der 1. Applikation | nach der 2. Applikation | nach der 3. Applikation |
|---|---|---|---|
| Nasskämmbarkeit | +(2−3) | +(2−3) | +(2−3) |
| Trockenkämmbarkeit | +2 | +2 | +2 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Beispiel 5:**

Man verfährt wie im Beispiel 1 angegeben, setzt jedoch 2 Teile des polymeren Ammoniumsalzes gemäss Vorschrift A, 5 Teile des monomeren Ammoniumsalzes der Formel(39) in 100 Teilen entionisiertem Wasser und 150 Teile des Tensides der Formel (94) in 650 Teilen entionisiertem Wasser ein.

Man erhält 912 Teile einer schwach opalisierenden und viskosen Lösung, die durch Zugabe einer 10-%igen, wässrigen Zitronensäurelösung auf den pH-Wert von 6,0 eingestellt und mit entionisiertem Wasser auf 1000 Teile verdünnt wird.

Das Schampoonieren und Konditionieren der Perücke im Halbseitentest mit der verdünnten, klaren, lagerstabilen Lösung wird ebenfalls wie in Beispiel 1 durchgeführt, wobei die folgenden, in der nachstehenden Tabelle V zusammengefassten Kämmbarkeitsnoten erzielt werden:

**Tabelle V**

| | nach der 1. Applikation | nach der 2. Applikation | nach der 3. Applikation |
|---|---|---|---|
| Nasskämmbarkeit | +3 | +3 | +3 |
| Trockenkämmbarkeit | +(2–3) | +(2–3) | +(2–3) |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Beispiel 6:**

Man verfährt wie im Beispiel 1 angegeben, setzt jedoch 2 Teile des Polymeren Ammoniumsalzes gemäss Vorschrift C, 5 Teile des monomeren Ammoniumsalzes der Formel (37) und 100 Teile eines Tensidgemisches der Formeln (129) und (130) ein.

Man erhält 907 Teile einer schwach opalisierenden und viskosen Lösung, die durch Zugabe einer 10-%igen, wässrigen Zitronensäurelösung auf den pH-Wert von 5,1 eingestellt und mit entionisiertem Wasser auf 1000 Teile verdünnt wird.

Das Schampoonieren und Konditionieren der Perücke im Halbseitentest mit der verdünnten, klaren, lagerstabilen Lösung wird ebenfalls wie in Beispiel 1 durchgeführt, wobei die folgenden, in der nachstehenden Tabelle VI zusammengefassten Kämmbarkeitsnoten erzielt werden:

**Tabelle VI**

| | nach der 1. Applikation | nach der 2. Applikation | nach der 3. Applikation |
|---|---|---|---|
| Nasskämmbarkeit | +3 | +3 | +3 |
| Trockenkämmbarkeit | +2 | +2 | +2 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Gleiche Resultate, insbesondere gleiche Kämmbarkeitsnoten werden erzielt, wenn man anstelle von 100 Teilen eines Tensidgemisches der Formeln (129) und (130) 100 Teile eines anionischen Tensides der Formel (57), worin q, q' und q'' die Zahl 8 und $T_3'$, $T_3''$ und $T_3'''$ Oleyl bedeuten, einsetzt.

**Beispiele 7 bis 11**

Man verfährt wie im Beispiel 1 angegeben, setzt jedoch jeweils 2 Teile des polymeren Ammoniumsalzes gemäss Vorschrift C, 5 Teile eines monomeren Ammoniumsalzes und 100 Teile eines Tensides oder Tensidgemisches ein, wobei die Formeln des eingesetzten monomeren Ammoniumsalzes und der eingesetzten Tenside in der nachfolgenden Tabelle VII angegeben sind.

Man erhält 907 Teile einer schwach opalisierenden und viskosen Lösung, die durch Zugabe einer 10-%igen, wässrigen Zitronensäurelösung auf den ebenfalls in Tabelle VII angegebenen pH-Wert eingestellt und mit entionisiertem Wasser auf 1000 Teile verdünnt wird.

Das Schampoonieren und Konditionieren der Perücke im Halbseitentest mit der verdünnten, klaren, lagerstabilen Lösung wird ebenfalls wie in Beispiel 1 durchgeführt, wobei die folgenden, ebenfalls in der nachstehenden Tabelle VII zusammengefassten Kämmbarkeitsnoten erzielt werden.

Zudem weist die erfindungsgemäss behandelte Perückenhälfte im Vergleich zur Perückenhälfte, die mit der Leerformulierung behandelt wird, ebenfalls gute antistatische Eigenschaften auf.

**Tabelle VII**

| Beispiel | monomeres Ammoniumsalz | Tensid | pH-Wert der verdünnten Lösung | Nasskämmbarkeit | Trockenkämmbarkeit |
|---|---|---|---|---|---|
| 7 | (37) | Gemisch aus (131c) und (131d) | 5,1 | +3 | +2 |
| 8 | (39) | (63) | 5,5 | +3 | +1 |
| 9 | (39) | (133) | 5,1 | +(1−2) | +1 |
| 10 | (39) | Gemisch aus (133) und (133a) | 5,1 | +2 | +(1−2) |
| 11 | (39) | (104) | 5,5 | +3 | 0 |

**Beispiel 12:**

Man verfährt wie im Beispiel 1 angegeben, setzt jedoch 2 Teile des polymeren Ammoniumsalzes gemäss Vorschrift C, 5 Teile des monomeren Ammoniumsalzes der Formel (39) und 100 Teile des technischen Tensidgemisches der Formel (81a) ein.

Man erhält 907 Teile einer schwach opalisierenden und viskosen Lösung, die durch Zugabe einer 10-%igen, wässrigen Zitronensäurelösung auf den pH-Wert von 5,0 eingestellt und mit entionisiertem Wasser auf 1000 Teile verdünnt wird.

Das Schampoonieren und Konditionieren der Perücke im Halbseitentest mit der verdünnten, klaren, lagerstabilen Lösung wird ebenfalls wie in Beispiel 1 durchgeführt, wobei die folgenden, in der nachstehenden Tabelle VIII zusammengefassten Kämmbarkeitsnoten erzielt werden:

**Tabelle VIII**

| | nach der 1. Applikation | nach der 2. Applikation | nach der 3. Applikation |
|---|---|---|---|
| Nasskämmbarkeit | +1 | +1 | +1 |
| Trockenkämmbarkeit | 0 | +(0−1) | +(0−1) |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Beispiel 13:**

Man verfährt wie im Beispiel 1 angegeben, setzt jedoch 2 Teile des polymeren Ammoniumsalzes gemäss Vorschrift C, 10 Teile des monomeren Ammoniumsalzes der Formel (39), 150 Teile des Tensides der Formel (94) und 30 Teile des Tensides der Formel (58), worin $T_2$ Octadecyl bedeutet, ein, wobei das Gemisch der Tenside der Formeln (94) und (58) mit 100 Teilen Wasser gelöst werden.

Man erhält 992 Teile einer schwach opalisierenden und viskosen Lösung, die durch Zugabe einer 10-%igen, wässrigen Zitronensäurelösung auf den pH-Wert von 5,0 eingestellt und mit entionisiertem Wasser auf 1000 Teile verdünnt wird.

Das Schampoonieren und Konditionieren der Perücke im Halbseitentest mit der verdünnten, klaren, lagerstabilen Lösung wird ebenfalls wie in Beispiel 1 durchgeführt, wobei die folgenden, in der nachstehenden Tabelle IX zusammengefassten Kämmbarkeitsnoten erzielt werden:

**Tabelle IX**

| | nach der 1. Applikation | nach der 2. Applikation |
|---|---|---|
| Nasskämmbarkeit | +2 | +(1−2) |
| Trockenkämmbarkeit | +(2−3) | +2 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 2 Applikation beobachtet werden können.

**Patentansprüche**

1. Wässrige Zusammensetzung aus polymeren, quaternären Ammoniumsalzen, aus monomeren bis oligomeren, quaternären Ammoniumsalzen und aus nicht-ionischen oder anionischen Tensiden, dadurch gekennzeichnet, dass sie einen pH-Wert von 3 bis 9 aufweist und mindestens (A) ein in wässrigen Tensidsystemen lösliches oder mikroemulgierbares Ammoniumsalz, das eine Molekulargewichtsverteilung von $10^4$ bis $10^9$ aufweist, wobei das Molekulargewicht von mindestens 5 Gewichtsprozent insbesondere 5 bis 60 Gewichtsprozent des polymeren Salzes $10^7$ bis $10^9$ beträgt und das Salz wiederkehrende Strukturelemente der Formel

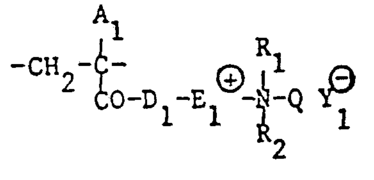

und gegebenenfalls in beliebiger Reihenfolge mindestens eines der wiederkehrenden Strukturelemente der Formeln

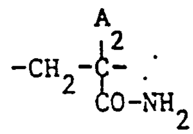,

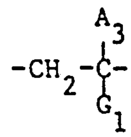 und

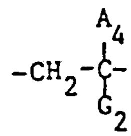

aufweist, worin $A_1$, $A_2$, $A_3$ und $A_4$ je Wasserstoff oder Methyl, $G_1$ und $G_2$ voneinander verschieden sind und je -CN, -COOH oder -CO-$D_2$-$E_2$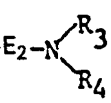,

$D_1$ und $D_2$ je Sauerstoff oder -NH-, $E_1$ und $E_2$ je Alkylen mit 1 bis 4 Kohlenstoffatomen, das gegebenenfalls durch Hydroxyl substituiert ist,

$R_1$, $R_2$ $R_3$ und $R_4$ je Methyl oder Aethyl, Q Alkyl, Hydroxyalkyl mit 1 bis 4 Kohlenstoffatomen oder Benzyl und $Y^1\ominus$ ein Halogenid-, Alkylsulfat- oder Alkylphosphonatani-on mit 1 bis 4 Kohlenstoffatomen im Alkylrest bedeuten,

(B) ein Ammoniumsalz, das 1 bis 3 quaternäre Stickstoffatome und ein Molekulargewicht von höchstens 9'000 aufweist und

(C) ein nicht-ionisches Tensid, ein Tensid mit intramolekular je einer positiven und negativen Ladung oder ein anionisches, gegebenenfalls zwitterionisches Tensid oder deren Gemische

enthält,

wobei bei Einsatz eines anionischen Tensides als Komponente (C) das polymere Ammoniumsalz als Komponente (A) neben Strukturelementen der Formel

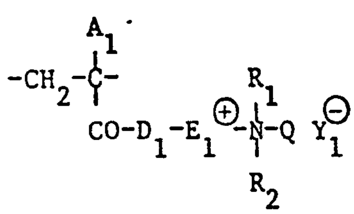

auch Strukturelemente der Formel

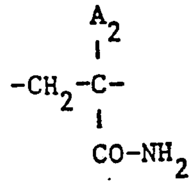

aufweist, und das anionische Tensid mindestens teilweise mit den Komponenten (A) und (B) unter Ionenaustausch umgesetzt ist.

2. Zusammensetzung nach Anspruch 1, dadurch gekennzeichnet, dass sie als Komponente (A) ein Ammoniumsalz enthält, das durchschnittlich 5 bis 100 Mol-% oder, sofern ein anionisches Tensid als Komponente (C) eingesetzt wird, durchschnittlich 5 bis 80 Mol-% Strukturelemente der Formel

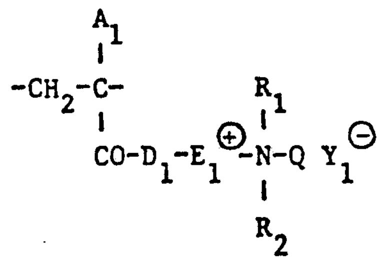
,

0 bis durchschnittlich 95 Mol-% oder, sofern ein anionisches Tensid als Komponente (C) eingesetzt wird, durchschnittlich 10 bis 75 Mol-% Strukturelemente der Formel

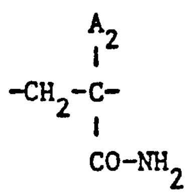
und

0 bis durchschnittlich 10 Mol-% Strukturelemente der Formel

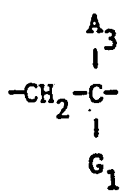
und gegebenenfalls
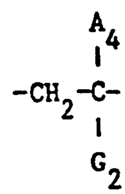
,

aufweist, worin $A_1$, $A_2$, $A_3$, $A_4$, $D_1$, $E_1$, $G_1$, $G_2$, $R_1$, $R_2$, Q und $Y_1^{\ominus}$ die in Anspruch 1 angegebenen Bedeutungen haben.

3. Zusammensetzung nach einem der Ansprüche 1 oder 2, dadurch gekennzeichnet, dass sie als Komponente (B) ein Ammoniumsalz enthält, welches der Formel

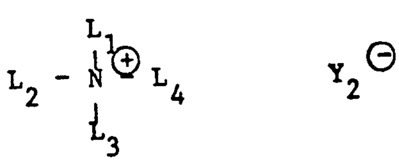

oder vorzugsweise der Formel

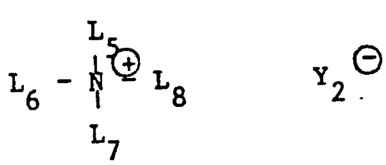
entspricht,

worin $L_1$ Wasserstoff, Alkyl oder Alkenyl mit höchstens 4 Kohlenstoffatomen, das gegebenenfalls durch Hydroxyl substituiert ist, $L_2$, $L_3$ und $L_4$ je gegebenenfalls verzweigtes Alkyl oder Alkenyl mit höchstens 22 Kohlenstoffatomen, das gegebenenfalls durch Sauerstoffatome oder Säureamidreste unterbrochen ist und gegebenenfalls endständig durch Hydroxyl, Carbamoyl, Dialkylamino, unsubstituiertes Phenyl oder Phenoxy, halogensubstituiertes oder halogenmethylsubstituiertes Phenyl oder Phenoxy substituiert ist, wobei durch -O- oder -CONH- unterbrochene $L_2$-, $L_3$- und $L_4$-Reste insgesamt je höchstens 120 Kohlenstoffatome aufweisen, oder bei Ammoniumsalzen mit zwei quaternären Stickstoffatomen, einer der $L_2$-, $L_3$- oder $L_4$-Reste der angegebenen Art endständig mit einem Di- oder Trialkylammonium-Kation substituiert ist, wobei die Alkylreste in Alkylamino- bzw. Alkylammonium-Substituenten 1 bis 4 Kohlenstoffatome aufweisen und gegebenenfalls durch Hydroxyl substituiert sind, oder $L_3$ und $L_4$ zusammen mit dem quaternären Stickstoffatom einen 4,5-Dihydroimidazolring oder einen 3,4,5,6-Tetrahydropyrimidinring bilden, wobei diese Ringe in 2-Stellung mit Alkyl mit 1 bis 22 Kohlenstoffatomen und am quaternären Stickstoffatom mit $L_1$ und $L_2$ mit je den angegebenen Bedeutungen substituiert sind, oder bei Ammoniumsalzen mit 3 quaternären Stickstoffatomen $L_1$ und $L_2$ die für $L_1$ angegebenen Bedeutungen haben, $L_3$ Cirbalkoxyalkyl oder Carbalkenyloxyalkyl mit 8 bis 22 Kohlenstoffatomen im Carbalkoxy- oder Carbalkenyloxyteil und 1 oder 2 Kohlenstoffatome im Alkylteil, und

$L_4$ einen geradkettigen Alkylrest mit 5 bis 8 Kohlenstoffatomen, der durch 2 quaternäre Stickstoffatome unterbrochen ist, wobei die Stickstoffatome durch Aethylen oder Propylen verbunden sind und jeweils mit $L_1$- und $L_3$-Reste der zuletzt angegebenen Bedeutungen substituiert sind, $L_5$ Wasserstoff Alkyl mit 1 bis 4 Kohlenstoffatomen oder Hydroxyalkyl mit 2 bis 4 Kohlenstoffatomen oder Alkenyl mit 3 oder 4 Kohlenstoffatomen, $L_6$, $L_7$ und $L_8$ je Alkyl mit 1 bis 22 Kohlenstoffatomen, Hydroxyalkyl mit 2 bis 4 Kohlenstoffatomen, Alkenyl mit 3 bis 22 Kohlenstoffatomen oder Reste der Formeln

$$-(CH_2)_{a-1}-(CH_2-\underset{(CH_3)_{2-n}}{\overset{(H)_{n-1}}{C}}HO)_b-H ,$$

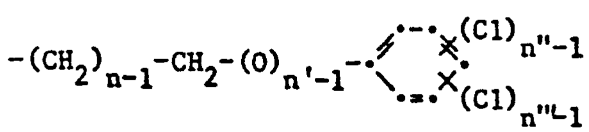
,

$$-(CH_2)_a-CONH_2 , \qquad -(CH_2)_a-CONH-L_9 \text{ oder } \qquad -(CH_2)_a-NHCO-L_9,$$

worin $L_9$ Hydroxyäthyl oder Alkyl mit 1 bis 22 Kohlenstoffatomen oder $L_9$ einen Rest der Formel

$$-(CH_2)_a-O-(CH_2-\underset{(CH_3)_{2-n}}{\overset{(H)_{n-1}}{C}}HO)_b-H \qquad \text{oder}$$

$$-(CH_2)_c-\underset{(L_{12})_{n-1}}{\overset{L_{10}}{N}}{}^{\oplus}_{n-1}-L_{11} \qquad Y^{\ominus}_{2\,n-1}$$

worin $L_{10}$ und $L_{11}$ je Methyl, Aethyl oder Hydroxyäthyl und L12 Wasserstoff, Methyl, Aethyl oder Hydroxyäthyl,

a 1 bis 22 b 1 bis 40 und c 1 bis 6 oder $L_7$ und $L_8$ zusammen mit dem quaternären Stickstoffatom einen Ring der Formel

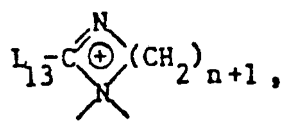

worin $L_{13}$ Alkyl oder Alkenyl mit 8 bis 22 Kohlenstoffatomem und n, n', n'' und n'' je 1 oder 2 bedeuten und $Y_2^{\ominus}$ ein Halogenid-, Alkylsulfat- oder Alkylphosphonatanion mit 1 bis 4 Kohlenstoffatomen im Alkylrest oder das Anion einer Alkylcarbonsäure oder einer Oxycarbonsäure mit höchstens 6 Kohlenstoffatomen oder einer Phosphorsäure bedeuten.

4. Zusammensetzung nach Anspruch 3, dadurch gekennzeichnet, dass sie als Komponente (B) ein Ammoniumsalz enthält, welches einer der Formeln

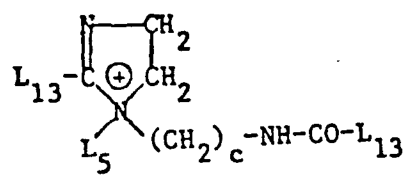 $Y_3^{\ominus}$ ,

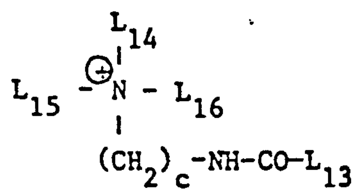 $Y_4^{\ominus}$ oder insbesondere

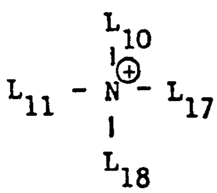 $Y_3^{\ominus}$

entspricht, worin $L_{14}$ und $L_{15}$ je Alkyl mit 1 bis 4 Kohlenstoffatomen,$L_{16}$ Wasserstoff, Alkkl mit 1 bis 4 Kohlenstoffatomen oder Hydroxyalkyl mit 2 bis 4 Kohlenstoffatomen oder $-CH_2-CO-NH_2$, $L_{17}$ Alkyl mit 10 bis 22 Kohlenstoffatomen und $L_{18}$ Alkyl mit 10 bis 22 Kohlenstoffatomen, Benzyl, Phenoxymethylen oder Phenoxyäthylen, $Y_3^{\ominus}$ ein Chlorid-, Methylsulfat- oder Acetatanion, $Y_4^{\ominus}$ ein Chlorid-, Methylsulfat- oder Acetatanion oder sofern $L_{16}$ Wasserstoff ist, ein Citrat-, Lactat- oder Phosphatanion bedeuten und $L_5$, $L_{10}$, $L_{11}$, $L_{13}$ und c die in Anspruch 3 angegebenen Bedeutungen haben.

5. Zusammensetzung nach einem der Ansprüche 1 bis 4, dadurch gekennzeichnet, dass sie als nichtionisches Tensid für die Komponente (C) äthoxylierte Fettsäure, Fettalkohole, Fetttsäureamide, Alkylphenole oder Kohlenhydrate, Addukte aus Aethylen- und Propylenoxyd, Phosphorsäurepolyglykolester oder Aminoxyde enthält, welche vorzugsweise einer der Formeln

$H-(O-CH_2-CH_2)_p-OOC-T_1$,

$H-(O-CH_2-CH_2)_p-NH-CO-T_1$,

$H-(O-CH_2-CH_2)_p-O-T_2$,

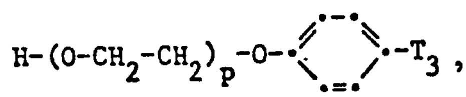 ,

$H-(O-CH_2-CH_2)_p-O-CH_2-(CHOH)_s-CHO$ ,

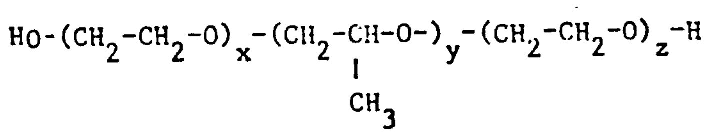 ,

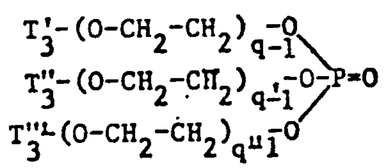 ,

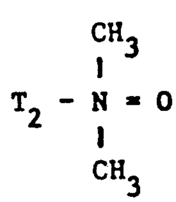 oder 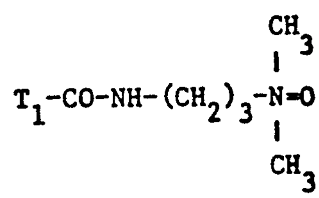

entsprechen, worin $T_1$ Alkyl oder Alkenyl mit 7 bis 21 Kohlenstoffatomen, $T_2$ Alkyl oder Alkenyl mit 8 bis 22 Kohlenstoffatomen, $T_3$,

$T'_3$, $T''_3$ und $T''_3{}'$ je Alkyl oder Alkenyl mit 6 bis 14 Kohlenstoffatomen,

p eine ganze Zahl von 1 bis 50, q, q' und q'' eine ganze Zahl von je 1 bis 13, s 3 oder 4 und x, y und z gleiche oder voneinander verschiedene ganze Zahlen bedeuten.

6. Zusammensetzung nach einem der Ansprüche 1 bis 4, dadurch gekennzeichnet, dass sie als Tensid mit intramolekular je einer positiven und negativen Ladung für die Komponente (C) Betaine oder Sulfobetaine enthält, die sich von einem Imidazolinderivat oder einem offenkettigen, aliphatischen Amin ableiten, wobei diese Betaine vorzugsweise einer der Formeln

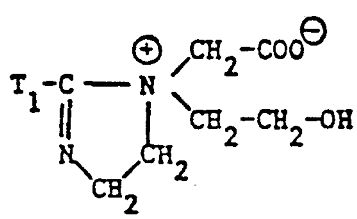 oder 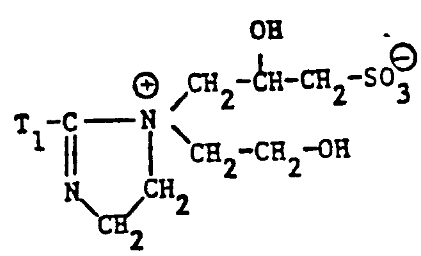 ,

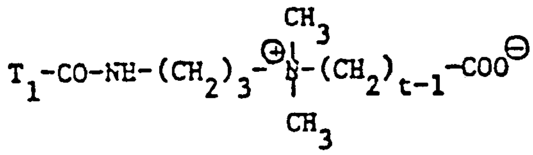 ,

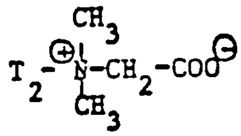 ,

$$T_2-\overset{\oplus}{N}H_2-CH_2-(CH_2)_{t-1}-COO^{\ominus}$$ ,

$$T_2-(NH-CH_2-CH_2)_{t-1}-NH-CH_2-CH_2-\overset{\oplus}{N}H_2-CH_2-COO^{\ominus}$$ ,

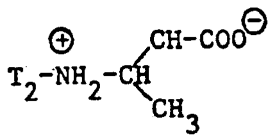 ,

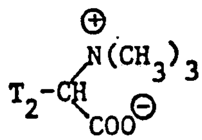 ,

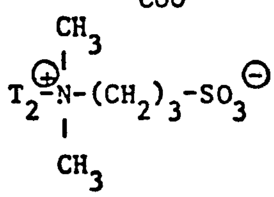 ,

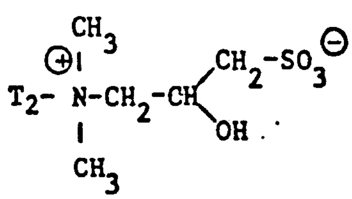 ;

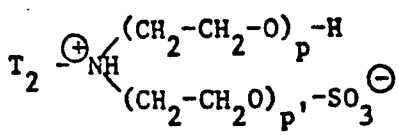 oder

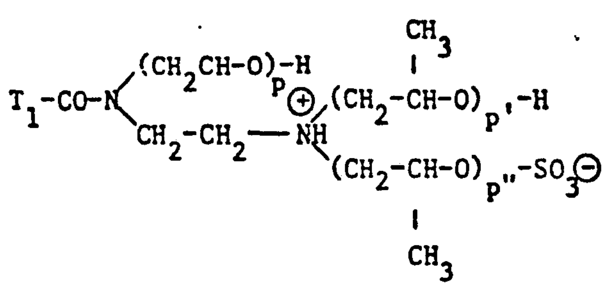 ,

entsprechen, worin $T_1$ Alkyl oder Alkenyl mit 7 bis 21 Kohlenstoffatomen, $T_2$ Alkyl oder Alkenyl mit 8 bis 22 Kohlenstoffatomen, p, p' und p'' eine ganze Zahl von je 1 bis 50 und t 1 oder 2 bedeuten.

7. Zusammensetzung nach einem der Ansprüche 1 bis 4, dadurch gekennzeichnet, dass sie als anionisches Tensid für die Komponente (C) ein Tensid der Formel $X^{\ominus}$ $Z^{\oplus}$ enthält, worin $X^{\ominus}$ den Rest eines oberflächenaktiven Sarkosinats, Sulfats (Sulfatäthers), Sulfonats oder Sulfobernsteinsäurederivates, eines fluorierten Tensids oder eines Phosphattensids und $Z^{\oplus}$ ein Alkalimetall- oder ein gegebenenfalls durch 1 bis 3 $(C_1\text{-}C_4)$-Alkyl- oder -Alkanolreste substituiertes Ammoniumkation bedeuten, wobei das Tensid vorzugsweise einer der Formeln

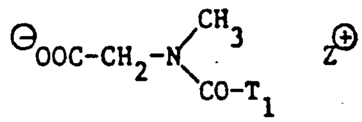 ,

$$T_1-O-(CH_2-CH_2-O)_{p-1}-SO_3^{\ominus}Z^{\oplus} ,$$

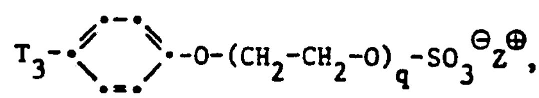

$$T_1-CO-NH-(CH_2)_r-O-SO_3^{\ominus}Z^{\oplus} ,$$

$$T_1-CO-NH-(CH_2-CH_2-O)_p-SO_3^{\ominus}Z^{\oplus} ,$$

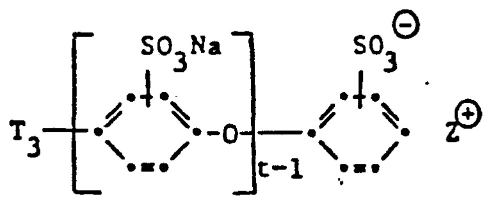 ,

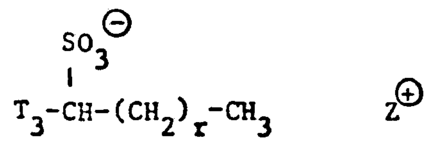 ,

$$T_3-COO-CH_2-CH_2-SO_3^{\ominus}\ Z^{\oplus} ,$$

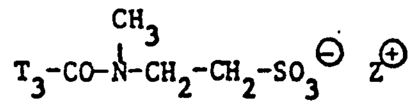 ,

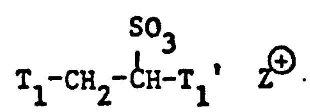 ,

$T_1-CH=CH-SO_3^{\ominus}\ \ Z^{\oplus}$ ,

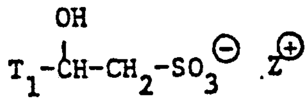 ,

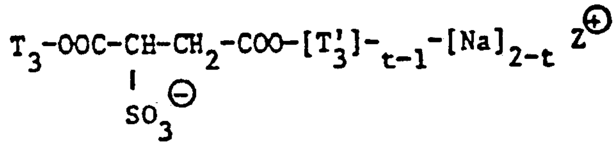 ,

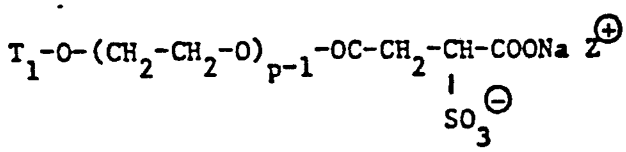 ,

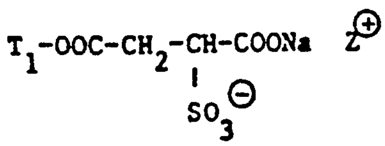 ,

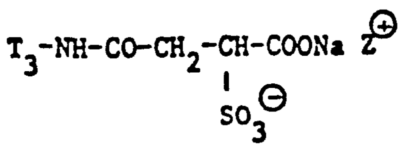 ,

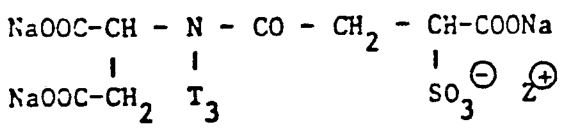 ,

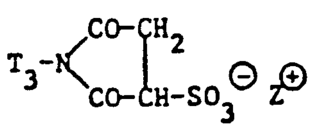 ,

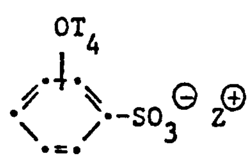 ,

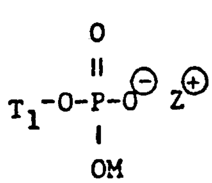 ,

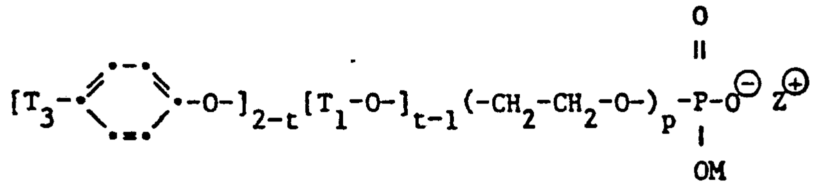

oder

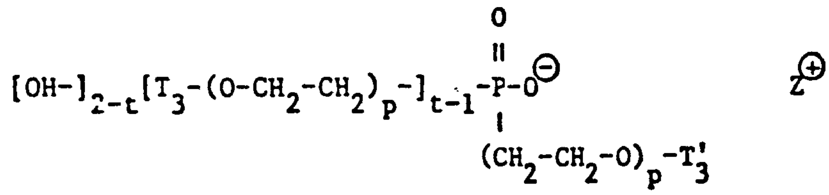

entspricht, worin $T_1$ und $T'_1$ je Alkyl oder Alkenyl mit 7 bis 21 Kohlenstoffatomen, $T_2$ Alkyl oder Alkenyl mit 8 bis 22 Kohlenstoffatomen, $T_3$ und $T'_3$ je Alkyl oder Alkenyl mit 6 bis 14 Kohlenstoffatomen, $T_4$ perfluoriertes Alkyl oder Alkenyl mit 6 bis 14 Kohlenstoffatomen, M Wasserstoff, Ammonium, ein Alkalimetall oder Alkyl mit 1 bis 3 Kohlenstoffatomen, p und p' je eine ganze Zahl von 2 bis 6, q eine ganze Zahl von 6 bis 12, r eine ganze Zahl von 2 bis 6 und t 1 oder 2 bedeuten und $Z^{\oplus}$ die angegebenen Bedeutungen hat.

8. Zusammensetzung nach einem der Ansprüche 1 bis 4, dadurch gekennzeichnet, dass sie als anionisches, zwitterionisches Tensid für die Komponente (C) ein Tensid der Formel $X^{\ominus}$ $Z^{\oplus}$ enthält, worin $X^{\ominus}$ den Rest eines oberflächenaktiven N-alkyl-α-iminodi-propionats oder eines in 2-Stellung alkylsubstituierten Imidazoliniumdicarbonsäurederivats bedeutet, wobei das Tensid vorzugsweise einer der Formeln

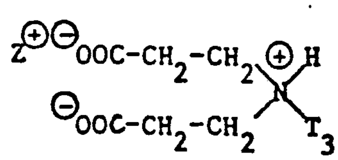

oder

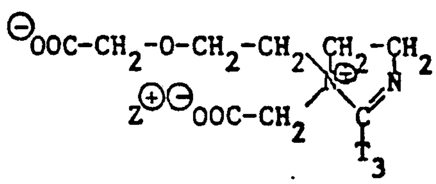

entspricht, worin $T_3$ Alkyl oder Alkenyl mit 6 bis 14 Kohlenstoffatomen bedeutet und $Z^{\oplus}$ die in Anspruch 7 angegebenen Bedeutungen hat.

9. Zusammensetzung nach einem der Ansprüche 1 bis 8 dadurch gekennzeichnet, dass sie die Komponenten (A), (3) und (C) in einem Gewichtsverhältnis (A):(B):(C) von 1:(1 bis 10): (2 bis 400) enthält.

10. Verfahren zur Herstellung der Zusammensetzung gemäss einem der Ansprüche 1 bis 9, wobei man ein Monomer der Formel

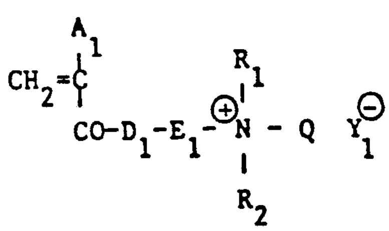

und gegebenenfalls mindestens ein Comonomer einer der Formeln

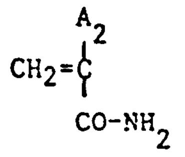 , 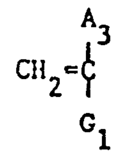 und 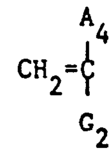 ,

worin $A_1$, $A_2$, $A_3$, $A_4$. $D_1$, $E_1$, $G_1$, $R_1$, $R_2$, Q und $Y_1^{\ominus}$ die in Anspruch 1 angegebenen Bedeutungen haben, durch Wasser-in-Oel Emulsionspolymerisation in Gegenwart eines Wasser-in-Oel Emulgators und gegebenenfalls eines Emulsionsstabilisators oder durch Lösungspolymerisation, jeweils in Gegenwart eines Polymerisationsinitiators zur Komponente (A) polymerisiert, das Polymerisat mit einem sowohl in Wasser wie in Oel löslichen Lösugsmittel ausfällt und anschliessend trocknet, dadurch gekennzeichnet, dass man das so erhaltene Polymerisat als Komponente (A) einsetzt und in wässrigem Medium mit mindestens einem 1 bis 3 quaternäre Stickstoffatome und ein Molekulargewicht von höchstens 9000 aufweisenden Ammoniumsalz als Komponente (B) sowie mit mindestens einem nicht-ionischen Tensid, einem Tensid mit intramolekular je einer positiven und negativen Ladung oder einem anionischen, gegebenenfalls zwitterionischen Tensid als Komponente (C) bei 10 bis 90 °C und einem pH-Wert von 3 bis 9 vermischt, wobei man gleichzeitig, sofern ein anionisches, gegebenenfalls zwitterionisches Tensid der Formel $X^{\ominus}Z^{\oplus}$ gemäss einem der Ansprüche 7 oder 8 als Komponente (C) eingesetzt wird, das anionische Tensid mit dem unter Einsatz des Comonomeren

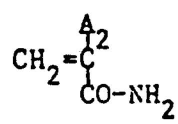

hergestelltes Copolymerisat als Komponente (A) und mit dem 1 bis 3 quaternäre Stickstoffatome aufweisenden Ammoniumsalz als Komponente (B) unter Ionenaustausch vorzugsweise bei 10 bis 90°C während 30 bis 100 Minuten mindestens teilweise umsetzt.

11. Verfahren nach Anspruch 10, dadurch gekennzeichnet, dass man einen Ueberschuss von vorzugsweise 4 bis 500 Mol mindestens eines anionischen, gegebenenfalls zwitterionischen Tensids gemäss einem der Ansprüche 7 oder 8 pro Mol des bei der Herstellung der Komponente (A) verwendeten, monomeren, quaternären Ammoniumsalzes und pro Mol Komponente (B), einsetzt.

12. Verwendung der Zusammensetzung gemäss einem der Ansprüche 1 bis 9 in kosmetischen, vorzugsweise haarkosmetischen Mitteln.

13. Kosmetische, vorzugsweise haarkosmetische Mittel, dadurch gekennzeichnet, dass sie mindestens eine der Zusammensetzungen gemäss einem der Ansprüche 1 bis 9, insbesondere 0,05 bis 1,5 Gewichtsteile, berechnet als Wirksubstanz, mindestens eines polymeren Ammoniumsalzes als Komponente (A) 0,1 bis 10 Gewichtsteile, berechnet als Wirksubstanz, mindestens eines monomeren bis oligomeren Ammoniumsalzes als Komponente (B), 5 bis 20 Gewichtsteile, berechnet als Wirksubstanz, mindestens eines nicht-ionischen Tensides, eines Tensides mit intramolekular je einer positiven und negativen Ladung oder eines anionischen, gegebenenfalls zwitterionischen Tensides als Komponente (C) und gegebenenfalls kosmetische Hilfsmittel enthalten, wobei die Mittel vorzugsweise mit entionisiertem Wasser auf insgesamt 100 Gewichtsteile verdünnt und mit einer wässrigen Lösung aus Natriumhydroxyd oder Zitronensäure auf einen pH-Wert von 5 bis 8, vorzugsweise 5 bis 6, eingestellt sind.

14. Haarbehandlungsverfahren, dadurch gekennzeichnet, dass man ein kosmetisches Mittel gemäss Anspruch 13 auf mit Leitungswasser angefeuchtetem Haar bei 20 bis 40°C aufbringt, das Haar schampooniert und konditioniert.

**Claims**

1. Am aqueous composition of polymeric, quaternary ammomium salts, of monomeric to oligomeric, quaternary ammonium salts and of monionic or anionic surfactants, which composition has a pH value of 3 to 9 and contains at least

(A) one ammomium salt which is soluble or micro-emulsifiable im aqueous surfactant systems and has a molecular weight distribution of $10^4$ to $10^9$, the molecular weight of at least 5 per cent by weight of the polymeric salt being $10^7$ to $10^9$, said salt containing recurring structural units of the formula

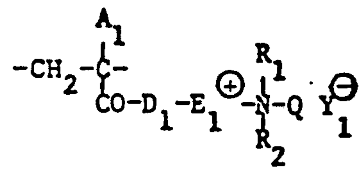

and, optionally, in any order, at least one of the recurring structural units of the formulae

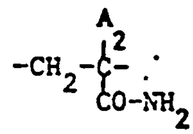 ,

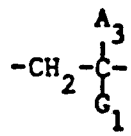 and

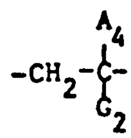

wherein $A_1$, $A_2$, $A_3$ amd $A_4$ are each hydrogen or methyl, $G_1$ and $G_2$ are different from each other and are each -CN, -COOH or -CO-$D_2$-$E_2$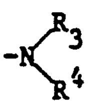 ,

$D_1$ and $D_2$ are each oxygen or -NH-, $E_1$ and $E_2$ are each $C_1$-$C_4$alkylene which is unsubstituted or substituted by hydroxyl, $R_1$, $R_2$, $R_3$ and $R_4$ are each methyl or ethyl, Q is alkyl, $C_1$-$C_4$hydroxyalkyl or benzyl and $Y_1^{\ominus}$ is a halide, alkylsulfate or alkylphosphonate anion containing 1 to 4 carbon atoms in the alkyl moiety,

(B) an ammonium salt which contains 1 to 3 quaternary nitrogen atoms and has a molecular weight of not more than 9 000, and

(C) a nonionic surfactant, a surfactant having a positive and a negative charge im each molecule or an anionic surfactant which may be im zwitterion form, or mixtures thereof,

with the proviso that if component (C) is an aniomic surfactant, the polymeric ammonium salt used as componemt (A), im addition to containing structural units of the formula

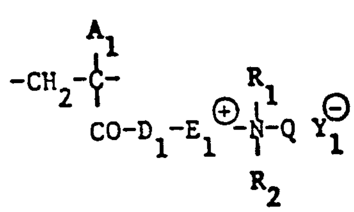

also contains structural units of the formula

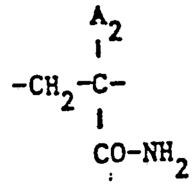

and the anionic surfactant has been at least partially reacted by ion exchange with components (A) and (B).

2. A composition according to claim 1, wherein component (A) is an ammonium salt which contains om average 5 to 100 mol %, or, if component (C) is an anionic surfactant, on average 5 to 80 mol %, of structural units of the formula

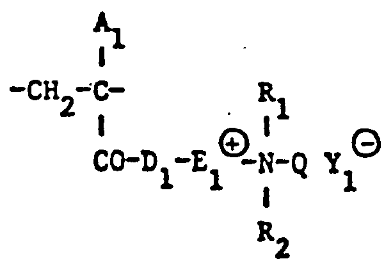

0 to an average of 95 mol %, or, if component (C) is an anionic surfactamt as, on average 10 to 75 mol %, of structural units of the formula

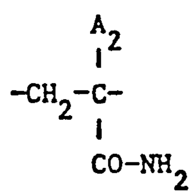

and

0 to an average of 10 mol % of structural units of the formula

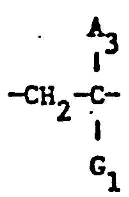

and optionally

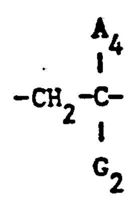

wherein $A_1$, $A_2$, $A_3$, $A_4$, $D_1$, $E_1$, $G_1$, $G_2$, $R_1$, $R_2$, Q and $Y_1^{\ominus}$ are as defined in claim 1.

3. A composition according to either of claims 1 or 2, wherein component (B) is an ammonium salt of the formula

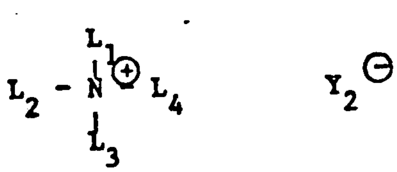

or preferably of the formula

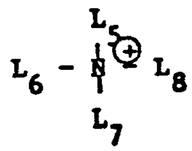 $Y_2^{\ominus}$

wherein $L_1$ is hydrogen or alkyl or alkenyl which contains not more than 4 carbon atoms and is unsubstituted, or substituted by hydroxyl, $L_2$, $L_3$ and $L_4$ are each unbranched or branched alkyl or alkenyl which contains not more than 22 carbon atoms and can be interrupted by oxygen atoms or acid amide radicals and can be terminally substituted by hydroxyl, carbamoyl, dialkylamino, unsubstituted phenyl or phenoxy or halogen-substituted or halomethyl-substituted phenyl or phenoxy, said $L_2$, $L_3$ and $L_4$ radicals which are interrupted by -O- or -CONH- each containing a total of not more than 120 carbon atoms or, where the ammonium salt contains two quaternary nitrogen atoms, one of the $L_2$, $L_3$ or $L_4$ radicals of the indicated kind is terminally substituted by a dialkylammonium or trialkylammonium cation, the alkyl moieties of alkylamino or alkylammonium substituents containing 1 to 4 carbon atoms and being unsubstituted or substituted by hydroxyl, or $L_3$ amd $L_4$, together with the quaternary nitrogen atom, form a 4,5-di-hydroimidazole ring or a 3,4,5,6-tetrahydropyrimidine ring, said rings being substituted in the 2-position by alkyl of 1 to 22 carbon atoms and being substituted at the quaternary nitrogen atom by $L_1$ and $L_2$, each of which is as defined, or, where the ammonium salt contains 3 quaternary nitrogen atoms, $L_1$ and $L_2$ are as defined for $L_1$; $L_3$ is carboalkoxyalkyl or carboalkenyloxyalkyl containing 8 to 22 carbon atoms in the carboalkoxy or carboalkenyloxy moiety and 1 or 2 carbon atoms in the alkyl moiety; and $L_4$ is a straight-chain alkyl radical which contains 5 to 8 carbon atoms and is interrupted by 2 quaternary nitrogen atoms which are linked through ethylene or propylene and are each substituted by $L_1$ and $L_3$ radicals as defined above, $L_5$ is hydrogen, $C_1$-$C_4$alkyl or $C_2$-$C_4$hydroxyalkyl or $C_3$-$C_4$alkenyl, $L_6$, $L_7$ and $L_8$ are each $C_1$-$C_{22}$alkyl, $C_2$-$C_4$hydroxyalkyl, $C_3$-$C_{22}$alkenyl or radicals of the formulae

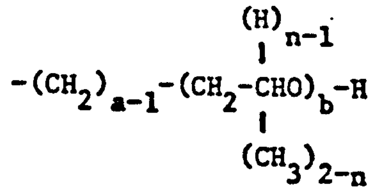 ,

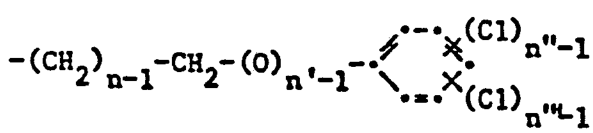 ,

$-(CH_2)_a-CONH_2$ , $-(CH_2)_a-CONH-L_9$ or $-(CH_2)_a-NHCO-L_9$,

in which $L_9$ is hydroxyethyl or $C_1$-$C_{22}$alkyl or $L_9$ is a radical of the formula

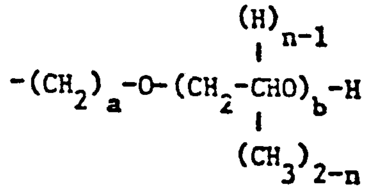 or

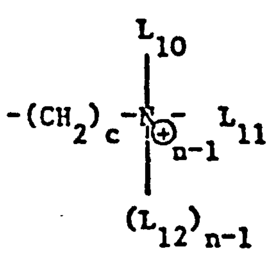 $Y_{2\ n-1}^{\ominus}$

in which $L_{10}$ and $L_{11}$ are each methyl, ethyl or hydroxyethyl and $L_{12}$ is hydrogen, methyl, ethyl or hydroxyethyl, a is 1 to 22, b is 1 to 40 and c is 1 to 6, or $L_7$ and $L_8$, together with the quaternary nitrogen atom, are a ring of the formula

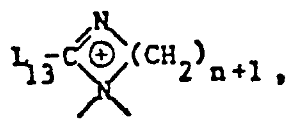

in which $L_{13}$ is alkyl or alkenyl, each of 8 to 22 carbon atoms, and n, n', n'' and n'' are each 1 or 2, and $Y_2^{\ominus}$ is a halide, alkylsulfate or alkylphosphonate anion having 1 to 4 carbon atoms in the alkyl moiety or the anion of an alkylcarboxylic acid or a hydroxycarboxylic acid having not more than 6 carbon atons or a phosphoric acid.

4. A composition according to claim 3, wherein component (B) is an ammoniun salt of the formula

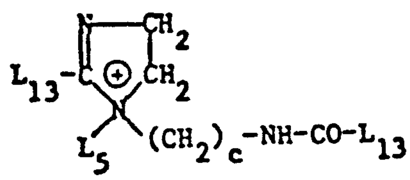

$Y_3^{\ominus}$ ,

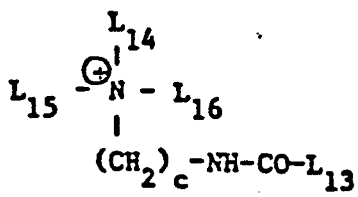

$Y_4^{\ominus}$ or preferably

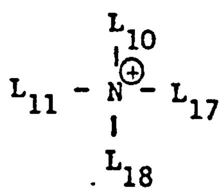

$Y_3^{\ominus}$

in which L 4 and L are each $C_1$-$C_4$alkyl $L_{16}$ is hydrogen, $C_1$-$C_4$alkyl or $C_2$-$C_4$hydroxyalkyl or -CH -CO-NH L 7 is $C_1$ -$C_{22}$alkyl and $L_{18}$ is $C_{10}$-$C_{22}$alkyl, benzyl, phenoxymethylene or phenoxyethylene, $Y^{\ominus}_3$ is a chloride, methylsulfate or acetate ion, and $Y_4^{\ominus}$ is a chloride, methylsulfate or acetate anion or, if $L_{16}$ is hydrogen, is a citrate, lactate or phosphate anion, and $L_5$, $L_{10}$, $L_{11}$, $L_{13}$ and c are as defined in claim 3.

5. A composition according to any one of claims 1 to 4, wherein the nonionic surfactant of component (C) is selected from ethoxylated fatty acids, fatty alcohols, fatty acid amides, alkylphenols or carbohydrates, adducts formed from ethylene oxide and propylene oxide, phosphoric acid polyglycol esters or amine oxides, preferably of one of the formulae

$H-(O-CH_2-CH_2)_p-OOC-T_1$,

$H-(O-CH_2-CH_2)_p-NH-CO-T_1$,

$H-(O-CH_2-CH_2)_p-O-T_2$,

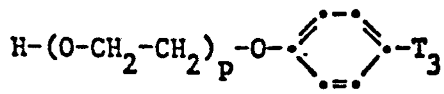
,

$H-(O-CH_2-CH_2)_p-O-CH_2-(CHOH)_s-CHO$ ,

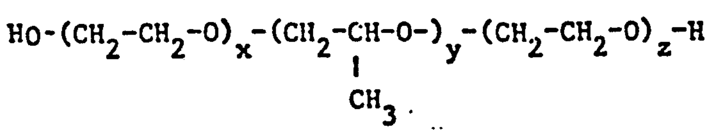
,

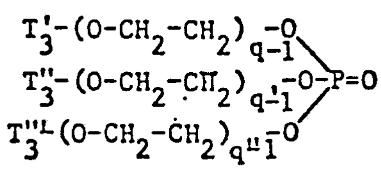
,

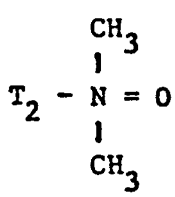
or
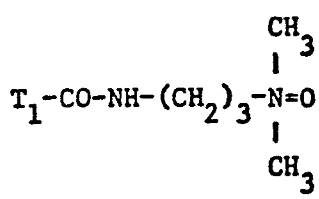

in which $T_1$ is alkyl or alkenyl, each of 7 to 21 carbon atoms, $T_2$ is alkyl or alkenyl each of 8 to 22 carbon atoms, $T_3$, $T'_3$, $T_3''$ and $T_3'''$ are each alkyl or alkenyl of 6 to 14 carbon atoms, p is an integer from 1 to 50, each of q, q' and q'' is an integer from 1 to 13, s is 3 or 4, and x, y and z are identical or different integers.

6. A composition according to any one of claims 1 to 4, wherein the surfactant having a positive and a negative charge in each molecule, as component (C), is selected from betaines or sulfobetaines derived from an imidazoline derivative or from an open-chain, aliphatic amine, said betaines having preferably one of the formulae

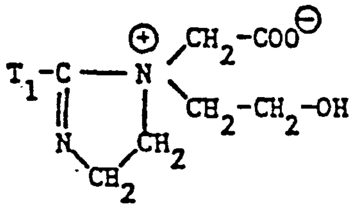
or
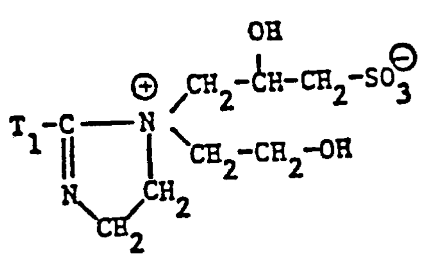
,

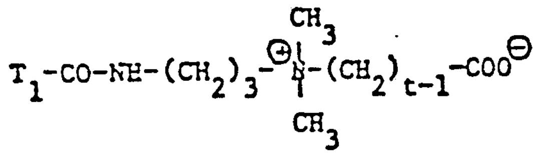,

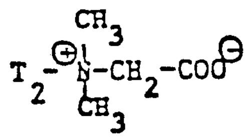,

$T_2-\overset{\oplus}{NH_2}-CH_2-(CH_2)_{t-1}-COO^{\ominus}$ ,

$T_2-(NH-CH_2-CH_2)_{t-1}-NH-CH_2-CH_2-\overset{\oplus}{NH_2}-CH_2-COO^{\ominus}$ ,

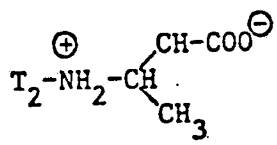,

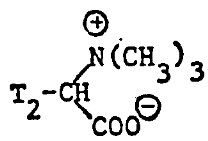,

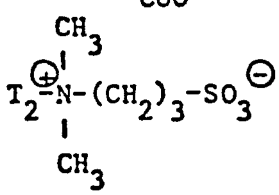,

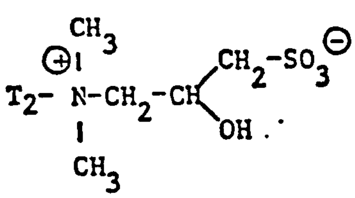;

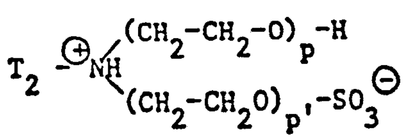

or

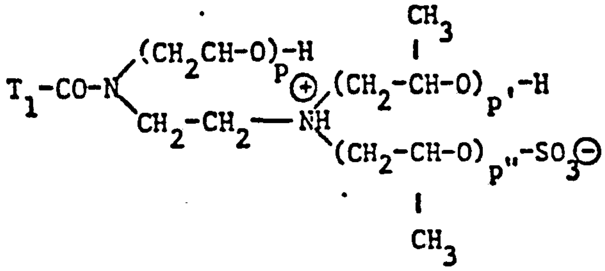,

in which $T_1$ is alkyl or alkenyl having 7 to 21 carbon atoms, $T_2$ is alkylor alkenyl having 8 to 22 atoms and p, p' and p'' are each an integer from 1 to 50, and t is 1 or 2.

7. A composition according to any one of claims 1 to 4, wherein the anionic surfactant of component (C) is a surfactant of the formula $X^{\ominus}\ Z^{\oplus}$ in which $X^{\ominus}$ is the radical of a surface-active sarcosinate, a fluorinated surfactant or of a phosphate surfactant and $Z^{\oplus}$ is an alkali metal cation or an ammonium cation which is unsubstituted or substituted by 1 to 3 ($C_1$-$C_4$)alkyl or ($C_1$-$C_4$)alkanol radicals, said surfactant preferably corresponding to one of the formulae

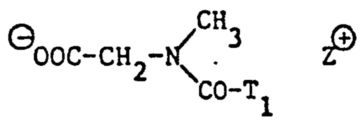 ,

$$T_1-O-(CH_2-CH_2-O)_{p-1}-SO_3^{\ominus}Z^{\oplus}$$ ,

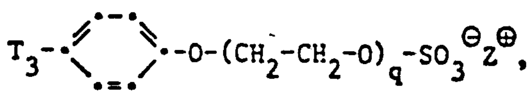 ,

$$T_1-CO-NH-(CH_2)_r-O-SO_3^{\ominus}Z^{\oplus}$$ ,

$$T_1-CO-NH-(CH_2-CH_2-O)_p-SO_3^{\ominus}Z^{\oplus}$$ ,

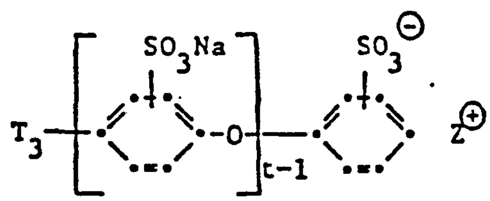 ,

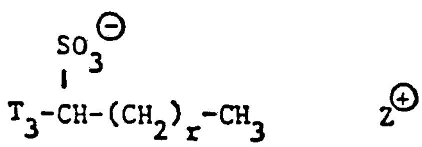 ,

$$T_3-COO-CH_2-CH_2-SO_3^{\ominus}\ Z^{\oplus}$$ ,

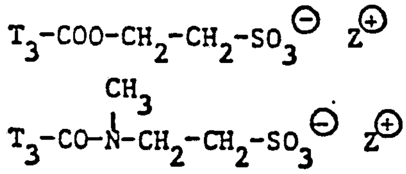 ,

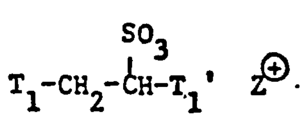 ,

$$T_1-CH=CH-SO_3^{\ominus}\ Z^{\oplus}$$ ,

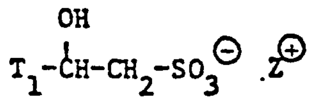
,

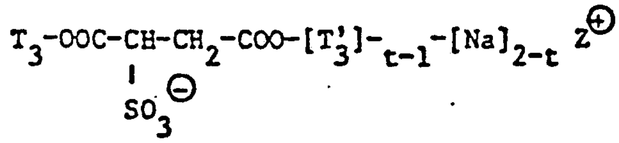
,

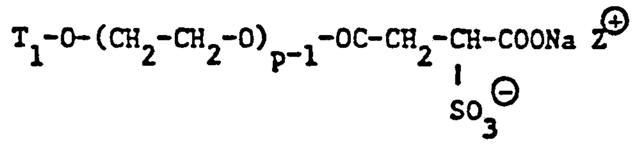
,

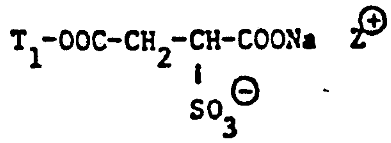
,

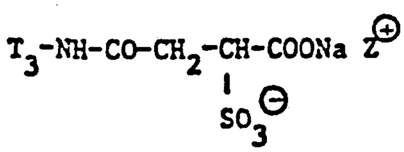
,

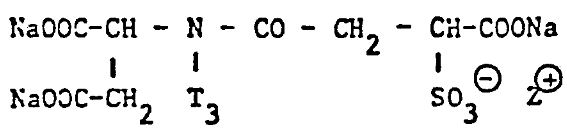
,

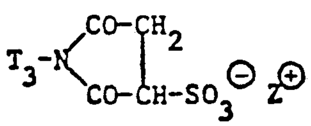
,

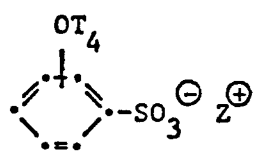
,

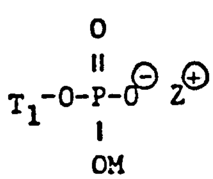
,

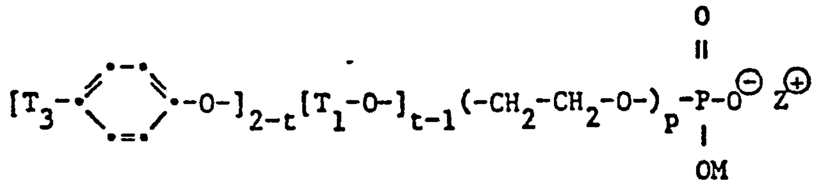

or

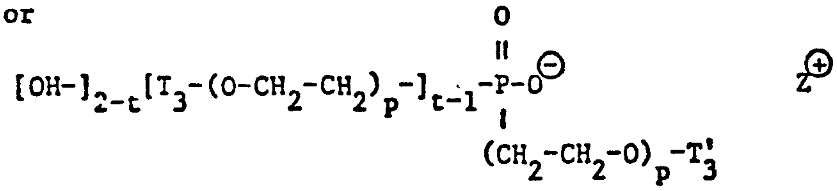

wherein $T_1$ and $T'_1$ are each alkyl or alkenyl of 7 to 21 carbon atoms, $T_2$ is alkyl or alkenyl, each of 8 to 22 carbon atoms, $T_3$ and $T'_3$ are each alkyl or alkenyl of 6 to 14 carbon atoms, $T_4$ is perfluorinated alkyl or alkenyl, each of 6 to 14 carbon atoms, M is hydrogen, ammoniun, an alkali metal or alkyl having 1 to 3 carbon atoms, p and p' are each an integer from 1 to 50, r is an integer from 2 to 6, t is 1 or 2 and $Z^{\oplus}$ is as defined above.

8. A composition according to any one of claims 1 to 4, wherein the anionic surfactant in zwitterion form of couponent (C) is a surfactant of the formula $X^{\ominus}$ $Z^{\oplus}$ in which $X^{\ominus}$ is the radical of a surfaceactive N-alkyl-α-iminodipropionate or of an imidazolinium dicarboxylic acid derivative which is substituted by alkyl in the 2-position, said surfactant corresponding preferably to one of the formulae

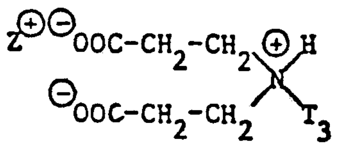

or

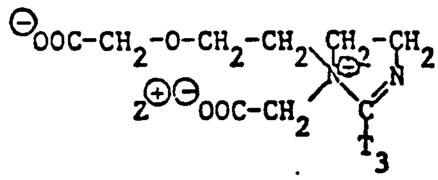

in which $T_3$ is alkyl or alkenyl each of 6 to 14 carbon atoms, and $Z^{\oplus}$ is as defined in claim 7.

9. A composition according to any one of claims 1 to 8, which contains components (A), (B) and (C) in a weight ratio of (A):(B):(C) of 1:(1 to 10):(2 to 400).

10. A process for the preparation of the composition as claimed in any one of claims 1 to 9, by polymerising a monomer of the formula

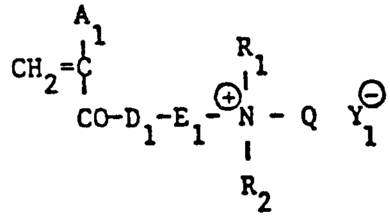

and, optionally at least one comonomer of one of the formulae

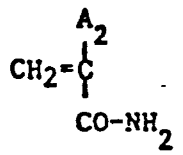 , 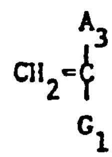 and 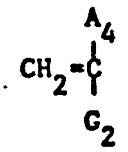 ,

in which $A_1$, $A_2$, $A_3$, $A_4$, $A_1$, $E_1$, $G_1$, $R_1$, $R_2$, Q and $Y_1^{\ominus}$ are as defined in claim 1, by water-in-oil emulsion polymerisation in the presence of a water-in-oil emulsifier and optionally an emulsion stabiliser, or by solution polymerisation, in each case in the presence of a polymerisation initiator, to give component (A), precipitating the polymer with a solvent which is soluble in water and in oil and then drying the precipitate which process comprises using the resultant polymer as component (A) and mixing it in an aqueous medium with at least one ammonium salt containing 1 to 3 quaternary nitrogen atoms and having a molecular weight of not more than 9000, as component (B), and also with at least one nonionic surfactant, a surfactant having a positive and a negative charge in each molecule or an anionic surfactant which may be in zwitterion from, as component (C), in the temperature range from 10 to 90° C and at a pH value of 5 to 9, and, if component (C) is an anionic surfactant which may be in zwitterion form, of the formula $X^{\ominus} Z^{\oplus}$ according to any one of claims 21 to 30, simultaneously reacting the anionic surfactant at least partially by ion exchange with the copolymer which has been prepared using the comonomer

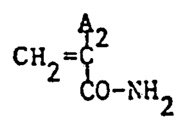

as component (A), and with the ammonium salt containing 1 to 3 quaternary nitrogen atoms as component (B).

11. A process according to claim 10, which comprises using an excess of preferably 4 to 500 moles of at least one anionic surfactant which nay be in zwitterion form according to either of claims 7 or 8 per mole of monomeric quaternary ammonium salt employed in the preparation of component (A), and per mole of component (B).

12. Use of a composition as claimed in any one of claims 1 to 9 in cosmetic, preferably hair cosmetic, compositions.

13. A cosmetic, preferably hair care cosmetic, composition, which contains at least one composition as claimed in any one of claims 1 to 9, preferably 0.05 to 1.5 parts by weight, calculated as active substance, of at least one polymeric ammonium salt as component (A), 0.1 to 10 parts by weight, calculated as active substance, of at least one monomeric to oligomeric ammonium salt as component (B), 5 to 20 parts by weight, calculated as active substance, of at least one nonionic surfactant, of a surfactant that carries one positive and one negative charge in each molecule or of an anionic surfactant which may be in zwitterion form as component (C), and optional cosmetic assistants, which composition is preferably diluted with deionised water to a total of 100 parts by weight and adjusted to a pH value of 5 to 8, preferably 5 to 6, with an aqueous solution of sodium hydroxide or citric acid.

14. A method of treating hair, which comprises applying a cosmetic composition as claimed in claim 13 at 20° to 40° C to hair which has been moistened with mains water, and shampooing and conditioning said hair.

**Revendications**

1. Composition aqueuse à base de sels d'ammonium quaternaire polymères, de sels d'ammonium quaternaire monomères à oligomères et d'agents tensio-actifs non ioniques ou anioniques, caractérisée par le fait qu'elle présente un pH de 3 à 9 et contient au moins

(A) un sel d'ammonium soluble ou micro-émulsifiable dans des systèmes tensio-actifs aqueux, qui présente une distribution de poids moléculaire allant de $10^4$ à $10^9$, le poids moléculaire d'au moins 5% en poids, en particulier de 5 à 60% en poids, du sel polymère allant de $10^7$ à $10^9$, et le sel comportant des motifs structuraux répétitifs de formule

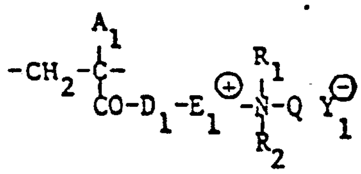

et éventuellement, dans un ordre quelconque, au moins un des motifs structuraux répétitifs de formules

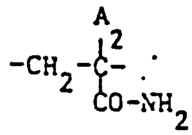

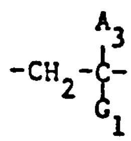

et

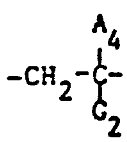

dans lesquelles $A_1$, $A_2$, $A_3$ et $A_4$ représentent chacun un atome d'hydrogène ou un groupe methyle; $G_1$ et $G_2$ sont différents l'un de l'autre et représentent chacun -CN, -CCOH ou $-CO-D_2-E_2-N\begin{smallmatrix}R\\R_4\end{smallmatrix}$

3;$D_1$ et $D_2$ sont chacun un atome d'oxygène ou -NH; $E_1$ et $E_2$ représentent chacun un radical alkylène ayant de 1 à 4 atomes de carbone, qui est eventuellement substitué par un groupe hydroxyle; $R_1$, $R_2$, $R_3$ et $R_4$ représentent chacun un groupe méthyle ou éthyle; Q représente un groupe alkyle, hydroxyalkyle, contenant de 1 à 4 atomes de carbone, ou un groupe benzyle; et

$Y_1\ominus$ représente un anion halogénure, un anion alkylsulfate ou alkylphosphonate ayant de 1 à 4 atomes de carbone dans le radical alkyle;

(B) un sel d'ammonium qui possède de 1 à 3 atomes d'azote quaternaire et qui a un poids moléculaire de 9000 au plus; et

(C) un agent tensio-actif non ionique, un agent tensio-actif ayant dans sa molécule à la fois une charge positive et une charge négative, ou un agent tensio-actif anionique, éventuellement amphotère, ou des mélanges de ceux-ci,

le sel d'ammonium polymère en tant que composant (A) comportant également, dans le cas d'utilisation d'un agent tensio-actif anionique en tant que composant (C), en plus de motifs structuraux de formule

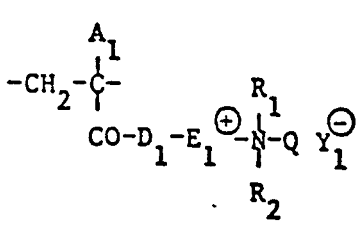

des motifs structuraux de formule

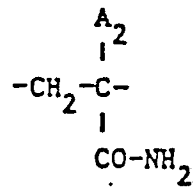

et l'agent tensio-actif anionique étant mis à réagir au moins en partie avec les composants (A) et (B) par échange d'ions.

2. Composition selon la revendication 1, caractérisée par le fait qu'elle contient en tant que composant (A) un sel d'ammonium qui comporte en moyenne de 5 à 100% en mole ou, dans la mesure où on utilise en tant que composant (C) un agent tensio-actif anionique, en moyenne de 5 à 80% en mole de motifs structuraux de formule

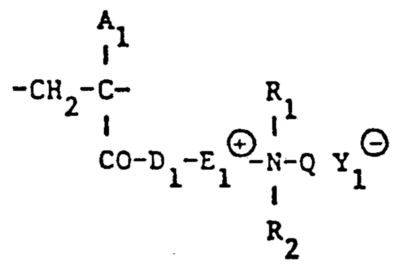 ,

de 0 à en moyenne 95% en mole ou, dans la mesure où on utilise en tant que composant (C) un agent tensio-actif anionique, en moyenne 10 à 75% en mole de motifs structuraux de formule

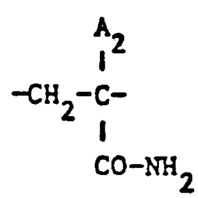 et

de 0 à en moyenne 10% en mole de motifs structuraux de formule

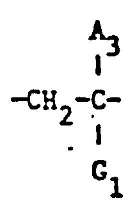 et éventuellement de formule 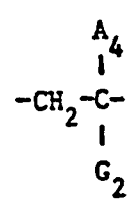 ,

$A_1$, $A_2$, $A_3$, $A_4$, $D_1$, $E_1$, $G_1$, $G_2$, $R_1$, $R_2$, Q et $Y_1 \ominus$ ayant les significations données dans la revendication 1.

3. Composition selon l'une des revendications 1 ou 2, caracterisée par le fait qu'elle contient en tant que composant (B) un sel d'ammonium qui correspond à la formule

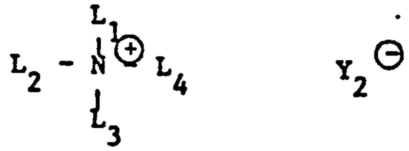

ou de préférence à la formule

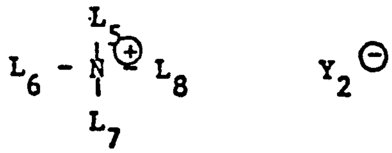

dans lesquelles $L_1$ est un atome d'hydrogène, un radical alkyle ou alcényle, ayant au plus 4 atomes de carbone, qui est éventuellement substitué par un groupe hydroxyle; $L_2$, $L_3$ et $L_4$ représentent chacun un radical alkyle ou alcényle, éventuellement ramifié, contenant au plus 22 atomes de carbone, qui est éventuellement interrompu par des atomes d'oxygène ou par des restes amido et qui est éventuellement substitué en bout de chaîne par un groupe hydroxyle, carbamyle, dialkylamino, phényle ou phénoxy non substitué, phényle ou phénoxy substitué par un atome d'halogène ou substitué par un reste halogénométhyle, les radicaux $L_2$, $L_3$ et $L_4$ interrompus par -O- ou par -CONH- comportant chacun au total 120 atomes de carbone au maximum ou, dans le cas de sels d'ammonium ayant deux atomes d'azote quaternaire, un des radicaux $L_2$, $L_3$ ou $L_4$ de type indiqué est substitué enbout de chaîne par un cation di- ou tri-alkylammonium, les restes alkyle dans les substituants alkylamino ou alkylammonium comportant de 1 à 4 atomes de carbone et étant éventuellement substitués par un groupe hydroxyle, ou bien $L_3$ et $L_4$ forment ensemble, avec l'atome d'azote quaternaire, un cycle 4,5-dihydro-imidazole ou un cycle 3,4,5,6-tétrahydropyrimidine, ces cycles étant substitués en position 2 par un groupe alkyle ayant de 1 à 22 atomes de carbone et, sur l'atome d'azote quaternaire, par $L_1$ et $L_2$ qui ont chacun les significations indiquées ou, dans le cas de sels d'ammonium ayant 3 atomes d'azote quaternaire, $L_1$ et $L_2$ ont les significations données pour $L_1$;

$L_3$ représente un radical carbalcoxyalkyle ou carbalcényloxyalkyle, ayant de 8 à 22 atomes de carbone dans la portion carbalcoxy ou carbalcényloxy et 1 ou 2 atomes de carbone dans la portion alkyle; et

$L_4$ représente un radical alkyle à chaîne droite ayant de 5 à 8 atomes de carbone, qui est interrompu par 2 atomes d'azote quaternaire, les atomes d'azote étant reliés par un groupe éthylène ou propylène et étant

chacun substitués par les radicaux $L_1$ et $L_3$ qui ont les significations données en dernier lieu; $L_5$ est un atome d'hydrogène, un groupe alkyle ayant de 1 à 4 atomes de carbone, ou un groupe hydroxyalkyle ayant de 2 à 4 atomes de carbone, ou un groupe alcényle ayant 3 ou 4 atomes de carbone; $L_6$, $L_7$ et $L_8$ représentent chacun un groupe alkyle ayant de 1 à 22 atomes de carbone, un groupe hydroxyalkyle ayant de 2 à 4 atomes de carbone, un groupe alcényle ayant de 3 à 22 atomes de carbone, ou l'un des restes de formules

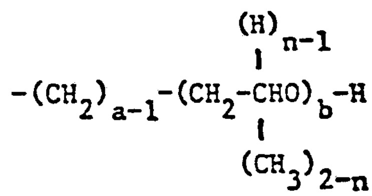
,

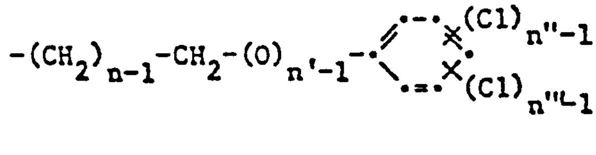
,

$-(CH_2)_a-CONH_2$ , $-(CH_2)_a-CONH-L_9$ ou $-(CH_2)_a-NHCO-L_9$,

dans lesquelles $L_9$ est un groupement hydroxyéthyle ou alkyle ayant de 1 à 22 atomes de carbone, ou bien $L_9$ est un radical de formule

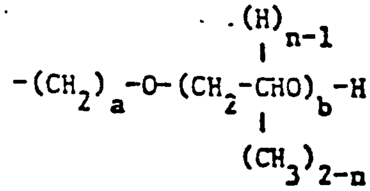

ou de formule

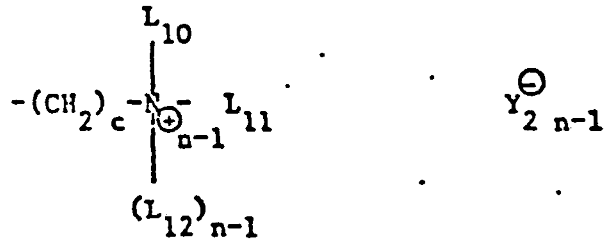

dans lesquelles $L_{10}$ et $L_{11}$ sont chacun un groupe méthyle, éthyle ou hydroxyéthyle;
a va de 1 à 22, b va de 1 à 40 et c va de 1 à 6; ou $L_7$ et $L_8$ forment ensemble, avec l'atome d'azote quaternaire, un cycle de formule

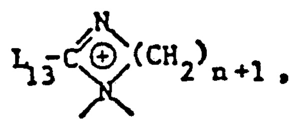

dans laquelle $L_{13}$ est un groupe alkyle ou alcényle ayant de 8 à 22 atomes de carbone, et n, n', n'' et n''' représentent chacun 1 ou 2; et $Y_2^{\ominus}$ représente un anion halogénure, un anion alkylsulfate ou alkylphosphonate ayant de 1 à 4 atomes de carbone dans le radical alkyle, ou l'anion d'un acide alkylcarboxylique ou d'un acide oxycarboxylique, contenant au plus 6 atomes de carbone, ou d'un acide phosphorique.

4. Composition selon la revendication 3, caractérisé par le fait qu'elle contient en tant que composant (B) un sel d'ammonium qui correspond à l'une des formules

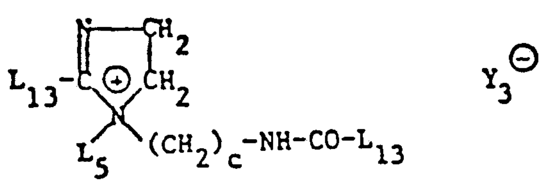

,

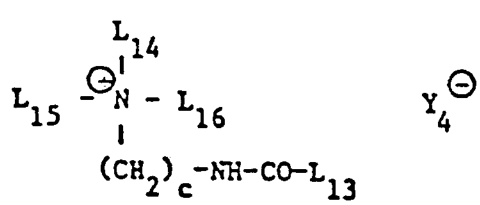

ou en particulier

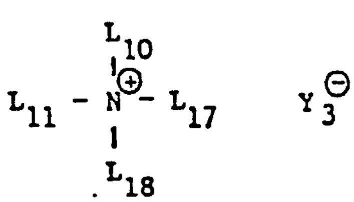

dans lesquelles $L_{14}$ et $L_{15}$ représentent chacun un groupe alkyle ayant de 1 à 4 atomes de carbone; $L_{16}$ représente un atome d'hydrogène, un groupe alkyle ayant de 1 à 4 atomes de carbone ou un groupe hydroxyalkyle ayant de 2 à 4 atomes de carbone, ou $-CH_2-CO-NH_2$; $L_{17}$ représente un radical alkyle ayant de 10 à 22 atomes de carbone; et $L_{18}$ représente un radical alkyle ayant de 10 à 22 atomes de carbone, un radical benzyle, phénoxyméthylène ou phénoxyéthylène; $Y_3^{\ominus}$ représente un anion chlorure, méthylsulfate ou acétate; $Y_4^{\ominus}$ représente un anion chlorure, méthylsulfate ou acétate ou, dans la mesure ou $L_{16}$ est un atome d'hydrogène, un anion citrate, lactate ou phosphate; et $L_5$, $L_{10}$, $L_{11}$ et c ont les significations données dans la revendication 3.

5. Composition selon l'une des revendications 1 à 4, caractérisée par le fait qu'elle contient en tant qu'agent tensio-actif non ionique pour le composant (C) des acides gras, alcools gras, amides gras, alkylphénols ou hydrates de carbone, oxy-éthylés, des produits d'addition d'oxyde d'éthylène et d'oxyde de propylène, des phosphates de polyglycols ou des oxydes d'amines, qui correspondent de préférence à l'une des formules

$$H-(O-CH_2-CH_2)_p-OOC-T_1,$$

$$H-(O-CH_2-CH_2)_p-NH-CO-T_1,$$

$$H-(O-CH_2-CH_2)_p-O-T_2,$$

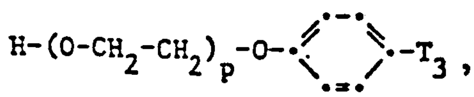

$$H-(O-CH_2-CH_2)_p-O-CH_2-(CHOH)_s-CHO\ ,$$

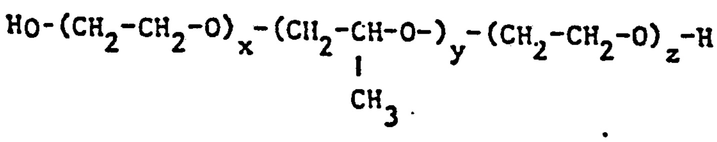

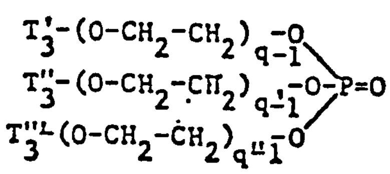

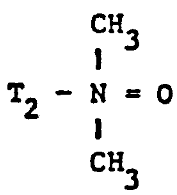

ou

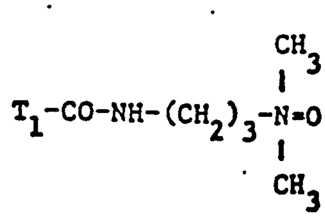

dans lesquelles $T_1$ représente un groupe alkyle ou alcényle ayant de 7à 21 atomes de carbone; $T_2$ représente un groupe alkyle ou alcényle ayant de 8 à 22 atomes de carbone; $T_3$, $T_3'$, $T_3''$ et $T_3'''$ représentent chacun un groupe alkyle ou alcényle ayant de 6 à 14 atomes de carbone; p est un nombre entier allant de 1 à 50; q, q' et q'' sont chacun un nombre entier allant de 1 à 13; s est 3 ou 4; et x, y et z représentent des nombres entiers égaux ou différents les uns des autres.

6. Composition selon l'une des revendications 1 à 4, caractérisée par le fait qu'elle contient, pour le composant (C), en tant qu'agent tensio-actif ayant dans sa molécule à la fois une charge positive et une charge négative, des bétaines ou des sulfobétaines qui dérivent d'un dérivé d'imidazoline ou d'une amine aliphatique à chaîne ouverte, ces bétaines correspondant de préférence à l'une des formules

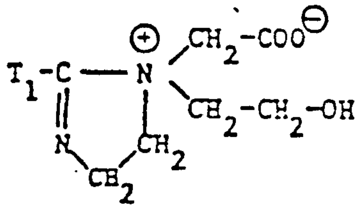

ou

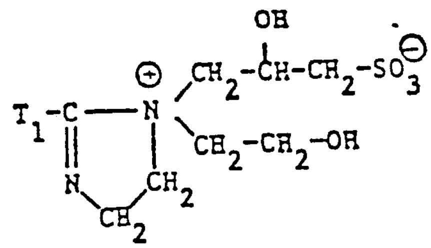

,

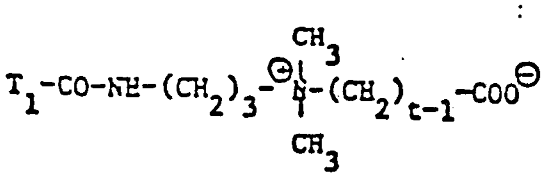

,

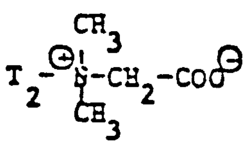

,

$$T_2-\overset{\oplus}{N}H_2-CH_2-(CH_2)_{t-1}-COO^{\ominus}$$ ,

$$T_2-(NH-CH_2-CH_2)_{t-1}-NH-CH_2-CH_2-\overset{\oplus}{N}H_2-CH_2-COO^{\ominus}$$ ,

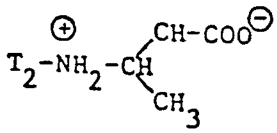

,

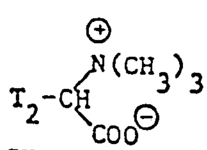

,

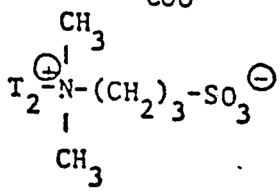

,

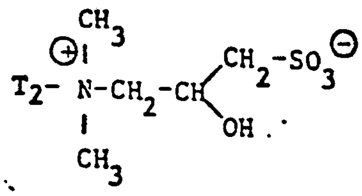

;

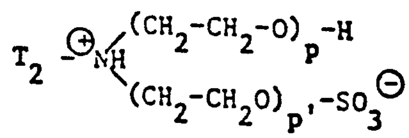

ou

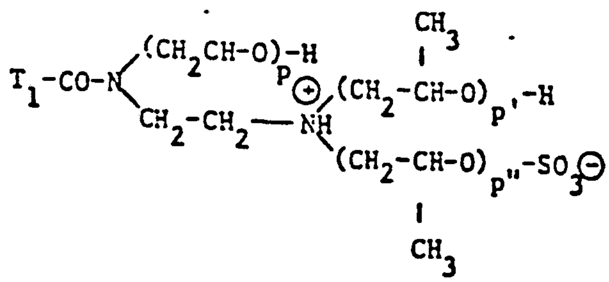

,

dans lesquelles $T_1$ représente un groupe alkyle ou alcényle ayant de 7 à 21 atomes de carbone; $T_2$ représente un groupe alkyle ou alcényle ayant de 8 à 22 atomes de carbone; p, p' et p'' sont chacun un nombre entier allant de 1 à 50; et t est 1 ou 2.

7. Composition selon l'une des revendications 1 à 4, caractérisée par le fait qu'elle contient, en tant qu'agent tensio-actif anionique pour le composant (C), un agent tensio-actif de formule $X^{\ominus}$ $Z^{\oplus}$, dans laquelle $X^{\ominus}$ représente le reste d'un sarcosinate tensio-actif, d'un sulfats (éther-sulfate) tensio-actif, d'un sulfonate tensio-actif ou d'un dérivé d'acide sulfosuccinique tensio-actif, d'un agent tensio-actif fluoré ou d'un agent tensio-actif phosphaté, et $Z^{\oplus}$ représente un cation de métal alcalin ou un cation ammonium éventuellement substitué par 1 à 3 restes alkyle en $C_{1-4}$ ou alcanol en $C_{1-4}$, l'agent tensio-actif correspondant de préférence à l'une des formules

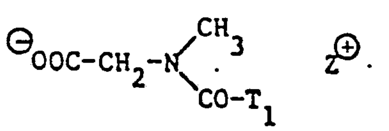

,

$$T_1-O-(CH_2-CH_2-O)_{p-1}-SO_3^{\ominus}Z^{\oplus} ,$$

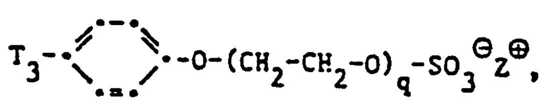

,

$$T_1-CO-NH-(CH_2)_r-O-SO_3^{\ominus}Z^{\oplus} ,$$

$$T_1-CO-NH-(CH_2-CH_2-O)_p-SO_3^{\ominus}Z^{\oplus} ,$$

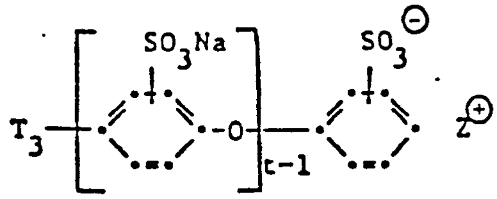,

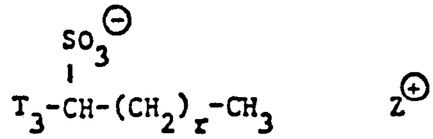,

$T_3-COO-CH_2-CH_2-SO_3^{\ominus}\ Z^{\oplus}$ ,

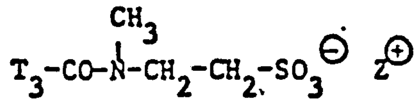,

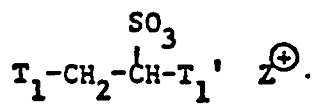,

$T_1-CH=CH-SO_3^{\ominus}\ Z^{\oplus}$ ,

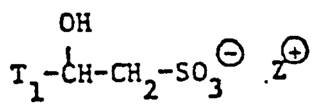,

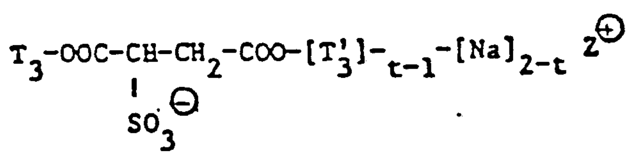,

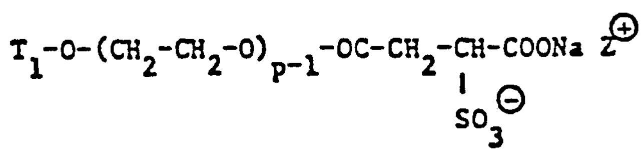,

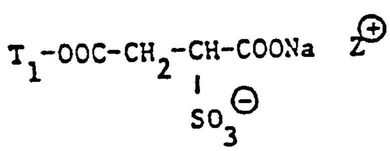,

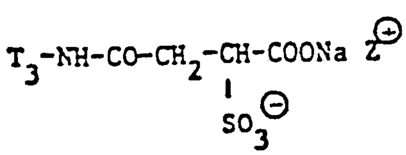,

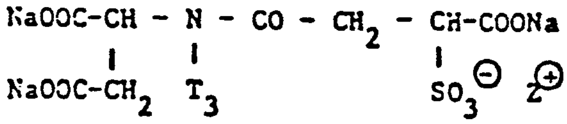,

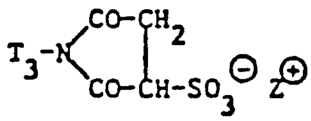,

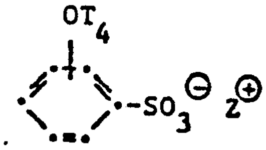,

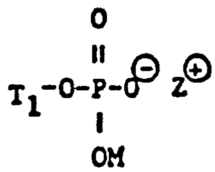,

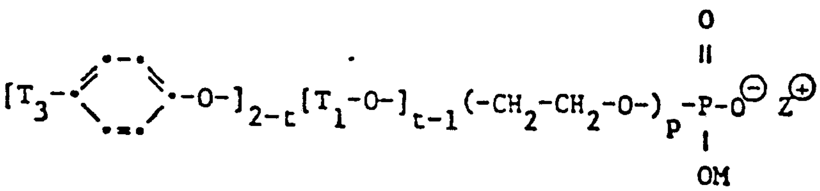

ou

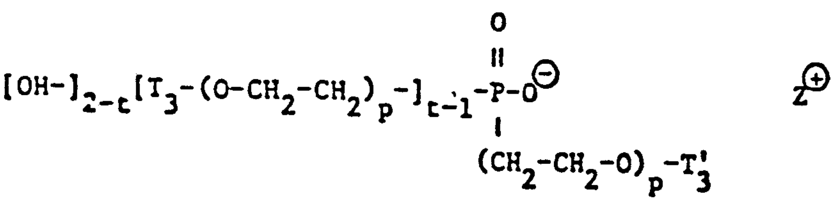

dans lesquelles $T_1$ et $T_1'$ représentent chacun un radical alkyle ou alcényls ayant de 7 à 21 atomes de carbone; $T_2$ représente un radical alkyle ou alcényle ayant de g à 22 atomes de carbone; $T_3$ et $T_3'$ représentent chacun un radical alkyle ou alcényle ayant de 6 à 14 atomes de carbone; $T_4$ représente un radical alkyle ou alcényle perfluoré ayant de 6 à 14 atomes de carbone; M représente un atome d'hydrogène, le groupe ammonium, un métal alcalin ou un groupe alkyle ayant de 1 à 3 atomes de carbone; p et p' représentent chacun un nombre entier allant de 2 à 6; q représente un nombre entier allant de 6 à 12; r est un nombre entier allant de 2 à 6; t est 1 ou 2; et $Z^{\oplus}$ a les significations données.

8. Composition selon l'une des revendications 1 à 4, caractérisée par le fait qu'elle contient, pour le composant (C), en tant qu'agent tensio-actif anionique, amphotère, un agent tensio-actif de formule $X^{\ominus}$ $Z^{\ominus}$, dans laquelle $X^{\ominus}$ représente le reste d'un N-alkyl-α iminodipropionate tensio-actif ou d'un dérivé d'acide imidazoliniumdicarboxylique substitué en position 2 par un groups alkyle,

l'agent tensio-actif correspondant ds préference à l'une des formules

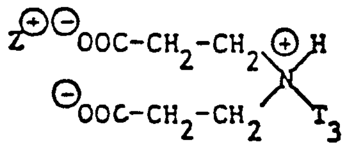

ou

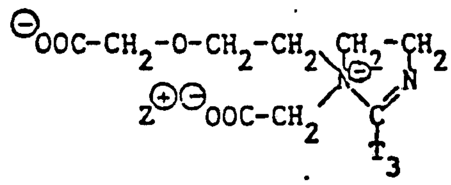

dans lesquelles $T_3$ représente un groupe alkyle ou alcényle ayant de 6 à 14 atomes de carbone et $Z^{\oplus}$ a les significations données dans la revendication 7.

9. Composition selon l'une des revendications 1 à 8, caractérisés par le fait qu'elle contient les composants (A), (B) et (C) en une proportion en poids (A):(B):(C) de 1:(1 à 10):(2 à 400).

10. Procédé pour la préparation de la composition selon l'une des revendications 1 à 9, dans lequel on polymérise un monomère de formule

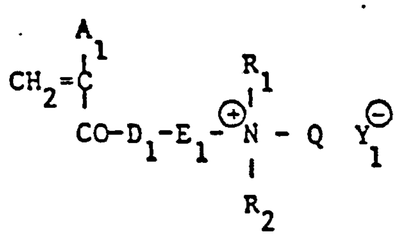

et, éventuellement, au moins un comonomère d'une des formules

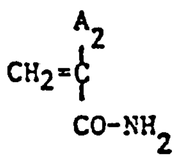 ,

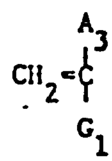 et

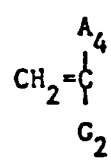 ,

dans lesquelles $A_1$, $A_2$, $A_3$, $A_4$, $D_1$, $E_1$, $C_1$, $R_1$, $R_2$, Q et $Y_1^{\ominus}$ ont les significations données dans la revendication 1, par polymérisation en émulsion eau-dans-huile en présence d'un émulsionnant eau-dans-huile et, éventuellement, d'un stabilisant d'émulsion, ou par polymérisation en solution, en présence chaque fois d'un amorceur de polymérisation, pour aboutir au composant (A), en précipite le polymère avec un solvant soluble aussi bien dans l'eau que dans l'huile, puis on le sèche, caractérisé par le fait que l'on utilise en tant que composant (A) le polymère ainsi obtenu et on le mélange en milieu aqueux avec au moins, en tant que composant (B), un sel d'ammonium comportant de 1 à 3 atomes d'azote quaternaire et ayant un poids moléculaire de 9000 au plus, ainsi qu'avec, en tant que composant (C), au moins un agent tensio-actif non ionique, un agent tensio-actif ayant dans sa molécule à la fois une charge positive et une charge négative, ou un agent tensio-actif anionique, éventuellement amphotère, à une température de 10 à 90°C et à un pH de 3 à 9, en faisant réagir simultanément, au moins en partie, dans la mesure cû on utilise, en tant que composant (C), un agent tensio-actif anionique, éventuellement amphotère, de formule $X^{\ominus} Z^{\oplus}$ selon l'une des revendications 7 et 8, l'agent tensio-actif anionique avec, en tant que composant (A), le copolymère préparé par utilisation du comonomère

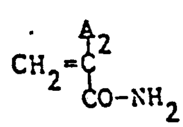

et avec, en tant que composant (B), le sel d'ammonium comportant de 1 à 3 atomes d'azote quaternaire, par échange d'ions, de préférence à une température de 10 à 90° C, pendant 30 à 100 minutes.

11. Procédé selon la revendication 10, caractérisé par le fait que l'on utilise un excès, de préférence de 4 à 500 moles, d'au moins un agent tensio-actif anionique éventuellement amphotère selon l'une des revendications 7 et 8, par mole du sel d'ammonium quaternaire, monomère, utilisé pour la préparation du composant (A), et par mole de composant (B).

12. Utilisation de la composition selon l'une des revendications 1 à 9 dans des produits cosmétiques, de préférence dans des produits cosmétiques capillaires.

13. Produits cosmétiques, de préférence produits cosmétiques capillaires, caractérisés par le fait qu'ils contiennent au moins une des compositions selon l'une des revendications 1 à 9, en particulier 0,05 à 1,5 partie en poids, calculée en tant que substance active, d'au moins un sel d'ammonium polymère, en tant que composant (A), 0,1 à 10 parties en poids, calculées en tant que substance active, d'au moins un sel d'ammonium monomère à oligomère, en tant que composant (B), 5 à 20 parties en poids, calculées en tant que substance active, d'au moins un agent tensio-actif non ionique, d'un agent tensio-actif ayant dans sa molécule à la fois une charge positive et une charge négative, ou d'un agent tensio-actif anionique, éventuellement amphotère, en tant que composant (C), et éventuellement des produits cosmétiques auxiliaires, les produits étant de préférence dilués, avec de l'eau désionisée, à 100 parties en poids au total, et ajustés à un pH de 5 à 8, de 5 à 6 de préférence, avec une solution aqueuse d'hydroxyde de sodium ou d'acide citrique.

14. Procédé de traitement capillaire, caractérisé par le fait que l'on applique, à 20-40° C, un produit cosmétique selon la revendication 13 sur des cheveux mouillés à l'eau du robinet, shampooigne les cheveux et les conditionne.